# EUROPEAN PATENT APPLICATION

(11) **EP 4 792 833 A1**
(43) Date of publication of application: **19.08.2026**
(21) Application number: 24876390.6
(22) Date of filing: 25.09.2024
(51) Int. Cl.: A61K 47/54, A61K 47/64, A61K 47/68, A61K 31/4745, A61P 35/00

(54) **CLEAVABLE LINKER COMPOUND FOR PRODRUG DELIVERY SYSTEM, AND PREPARATION AND USE THEREOF**

(30) Priority: 13.10.2023 CN 202311327506
(71) Applicant: East China Normal University, Shanghai 200062 (CN)
(72) Inventor: YU, Jiahui, Shanghai 200062 (CN); LU, Wei, Shanghai 200062 (CN); JIANG, Xing, Shanghai 200062 (CN); LI, Xiaomei, Shanghai 200062 (CN); ZHU, Shulei, Shanghai 200062 (CN)
(74) Representative: karo IP
(86) International application number: PCT/CN2024/120991
(87) International publication number: WO 2025/077572

(57) **Abstract**

Disclosed in present invention are a novel cleavable linker compound as shown in formula (1) for a prodrug delivery system, and a pharmaceutically acceptable salt, solvate, polymorph or isomer thereof. Further disclosed are a prodrug compound as shown in formula (J) and formula (L) formed by covalently bonding an albumin or antibody to a camptothecin derivative by means of a novel linker compound, and a pharmaceutically acceptable salt, solvate, polymorph or isomer thereof. Further disclosed are a synthesis method for the compound, and a preparation method for the compound in an enzyme release prodrug system. Further disclosed are a use of the compound in preparation of an anti-tumor drug, and a use of the compound in preparation of an albumin-bound anti-tumor drug or antibody-drug conjugate. The present invention innovatively proposes a structure in which a trigger fragment in the compound is linked to the novel linker by means of p-hydroxyl or para-amino, an active drug is linked to the novel linker by means of the hydroxyl part of the active drug, and a modified fragment is linked to the novel linker by means of an amino group. Disclosed in present invention are a novel cleavable linker compound as shown in formula (1) for a prodrug delivery system, and a pharmaceutically acceptable salt, solvate, polymorph or isomer thereof. Further disclosed are a prodrug compound as shown in formula (J) and formula (L) formed by covalently bonding an albumin or antibody to a camptothecin derivative by means of a novel linker compound, and a pharmaceutically acceptable salt, solvate, polymorph or isomer thereof. Further disclosed are a synthesis method for the compound, and a preparation method for the compound in an enzyme release prodrug system. Further disclosed are a use of the compound in preparation of an anti-tumor drug, and a use of the compound in preparation of an albumin-bound anti-tumor drug or antibody-drug conjugate. The present invention innovatively proposes a structure in which a trigger fragment in the compound is linked to the novel linker by means of p-hydroxyl or para-amino, an active drug is linked to the novel linker by means of the hydroxyl part of the active drug, and a modified fragment is linked to the novel linker by means of an amino group.

## Description

### Technical Field

The present invention belongs to the fields of biomedical technology and pharmaceutical formulation prodrug design, and relates to the design, synthesis, and synthetic products of a novel linker for a cleavable prodrug delivery system, as well as its application in prodrug delivery systems.

### Background of the invention

With the deepening understanding of tumors, various therapeutic modalities such as small molecule cytotoxic drugs, nucleic acids, proteins or peptides, and immunomodulators are continuously evolving. Among these, chemotherapy remains one of the important means for treating cancer. However, while chemotherapy uses cytotoxic drugs to kill tumor cells, it inevitably affects normal tissues, leading to various side effects.

A characteristic of tumor cells is abnormal growth and rapid proliferation; therefore, most chemotherapeutic drugs target this characteristic by selecting compounds with strong cytotoxicity. However, due to the lack of selectivity, these drugs damage normal tissues while killing tumor cells, adding an extra burden to already weakened cancer patients. Furthermore, some drugs, such as camptothecin derivatives, although occupying a place in anticancer drugs due to their considerable efficacy, present difficulties in administration and poor patient adaptability because of their physical properties, such as poor water solubility.

Therefore, how to modify these drugs to increase their targeting, solubility, stability, and improve their clinical adaptability has become a major challenge in the development process of chemotherapeutic drugs. The novel linker of the present invention, which can simultaneously provide a trigger, attachment sites for modifying side chains, and achieve self-immolation under specific conditions to release the active drug, has become one of the methods to solve this problem.

Glucuronide prodrugs are selectively activated by β-glucuronidase, an enzyme highly expressed in the microenvironment of many solid tumors such as lung cancer, breast cancer, and gastrointestinal cancer. Over the past two decades, this has been used to deliver drugs around tumors. The prodrug form can reduce the toxicity of the active drug, but because glucuronide prodrugs are cleared via the kidneys and metabolized quickly, the development of this type of prodrug has been limited.

Human serum albumin (HSA) is an endogenous protein, abundantly present in plasma. It is preferentially taken up by tumors as a nutrient. Moreover, when conjugated with albumin, its macromolecular nature can confer the EPR effect to the prodrug. The stable structure of albumin and its half-life of up to 19 days can stabilize the prodrug and enhance its half-life.

Antibody-Drug Conjugates (ADCs) are produced by conjugating a biologically active small molecule drug to a monoclonal antibody (mAb) via a linker. Currently, the vast majority of ADCs are formed by conjugating antibodies targeting tumor antigens with highly cytotoxic small molecule chemical drugs via linkers. They utilize the specific binding characteristic of antibodies to target antigens to deliver the small molecule drug to tumor cells, thereby exerting a tumor-killing effect. Additionally, specifically binding ligands, antigens, polypeptides, and other proteins and peptides with specific binding properties similar to antibodies can also be conjugated to linker compounds.

### Summary of the Invention

To address the shortcomings of the prior art, the purpose of the present invention is to propose a novel cleavable linker/branched linker compound of general formula for use in a prodrug delivery system, its further synthetic products, and its preparation methods and applications, including applications in prodrug delivery systems. The present invention innovatively proposes for the first time a new strategy for triggering drug release via hydrolysis of peptide bonds or glycosidic bonds by cathepsin or β-glucuronidase. Specifically, the linker compound useful in a prodrug delivery system of the present invention undergoes hydrolysis of a peptide bond by cathepsin to form an amino group, or hydrolysis of a glycosidic bond by β-glucuronidase or galactosidase to form a hydroxyl group, followed by 1,6-self-immolation and then a self-cyclization process to release the corresponding active drug.

The present invention proposes a novel cleavable linker compound for use in a prodrug delivery system, which has one or more independently modifiable sites, allowing different triggers, modifying side chains, and/or active drugs to be combined to form a new compound. The structure of the cleavable linker is as shown in the following formula (1): 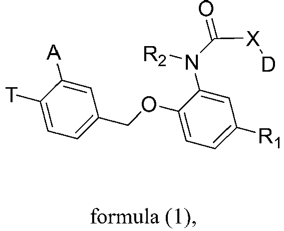
wherein, T represents an attachment site for linking R-TR;
in the R-TR, TR is selected from one or more of the following: a cathepsin substrate; an amino acid or short peptide chain of a cathepsin substrate; a β-glucuronidase substrate; a galactosidase substrate; a metalloproteinase substrate; an aromatic boronic acid or boronic ester oxidizable to a hydroxyl group; a nitro group, an azide group, or an azo group reducible to an amino group; a phosphate ester of a hydroxyl group, or a sulfate ester of a hydroxyl group; R is a substituent group selected from one or more of -H, PEG, maleimide-modified PEG, a sugar, folic acid, a polypeptide, an antibody, or a protecting group;
wherein, A represents an attachment site for linking LR;
LR is selected from one or more of H, CH₃(CH₂)n, a nitro group, a methoxy group, a PEG chain, maleimide-modified PEG, a maleimide-modified fatty chain, a maleimide-modified polyamino acid chain, a fatty chain, a polyamino acid chain, a sugar chain, a fatty acid, a sugar, folic acid, a polypeptide, or an antibody;
wherein, D represents an attachment site for linking DR;
the DR contains an exposed -OH, -NH2, -NHR, or -SH; the DR is a chemical drug, selected preferably from camptothecin compounds and their derivatives such as SN-38, DXd, irinotecan, beloliticon, as well as anti-tumor drugs such as paclitaxel, docetaxel, taxol, and CA4;
wherein, X is O, N, or S;
wherein, R₂ is H or CH₃(CH₂)n, n = 0-20;
wherein, R₁ is an electron-withdrawing group, including halogen (e.g., fluorine, chlorine, bromine), cyano, nitro, carboxylic acid, sulfonic acid, carboxylate ester group, sulfonate ester group, amide, and a group linked via an amide bond.

Specifically, the structure of the linker compound includes those shown in the following formula (1-1) and formula (1-2):
wherein, when the attachment site T of formula (1) is connected via an amide bond, its structure is as shown in the following formula (1-1): 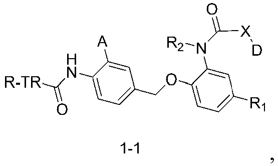
wherein, TR is selected from a single amino acid, a dipeptide, or a tetrapeptide among cathepsin substrates or metalloproteinase substrates; wherein the single amino acid is selected from unprotected or protected single amino acids, PEG chains, polyamino acid chains, and maleimide-modified single amino acids, including -Lys-, -Cit-, -Arg-, -Leu-, -His-, -Asp-, -Glu-, -Ala-, -Val-, -Ile-, -Pro-, -Phe-, -Trp-, -Met-, -Ser-, -Cys-, or -Asn-, etc.; the dipeptide is selected from unprotected or protected dipeptides, PEG chains, polyamino acid chains, and maleimide-modified dipeptides, including -Phe-Lys-, -Val-Lys-, -Ala-Lys-, -Val-Cit-, -Phe-Cit-, -Leu-Cit-, -Ile-Cit, -Trp-Cit-, -Phe-Gly-, -Ala-Ala-, or -Phe-Arg-, etc.; the tetrapeptide is selected from unprotected or protected tetrapeptides, PEG chains, polyamino acid chains, and maleimide-modified tetrapeptides, including -Gly-Gly-Phe-Gly-.

Wherein, when the attachment site T of formula (1) is connected via an ether bond or an ester bond, its structure is as shown in the following formula (1-2): 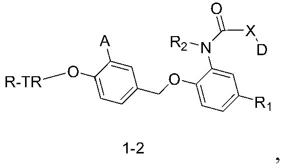
wherein, TR includes β-glucuronidase, β-galactosidase, phosphatase, sulfatase, and pyrophosphatase.
Wherein, A represents an attachment site for linking LR;
LR is selected from one or more of H, CH₃(CH₂)n, a nitro group, a methoxy group, a PEG chain, maleimide-modified PEG, a maleimide-modified fatty chain, a maleimide-modified polyamino acid chain, a fatty chain, a polyamino acid chain, a sugar chain, a fatty acid, a sugar, folic acid, a polypeptide, or an antibody;
wherein, when the attachment site A of formula (1) is preferably an amide bond, the structure of the linker compound is as shown in the following formula (1-5): 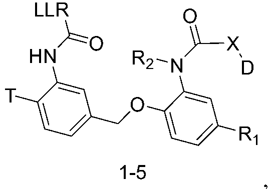
wherein, LLR is a modifying fragment, including one or more of PEG, a sugar, an alkyl chain, maleimide-modified PEG, a modified alkyl chain, or a fatty acid.

The group attached at the attachment site T is preferably β-glucuronidase, β-galactosidase, phosphatase, sulfatase, or pyrophosphatase;
wherein, when the group attached at the attachment site A of formula (1) is preferably H, the structure of the linker compound is as shown in the following formula (1-6): 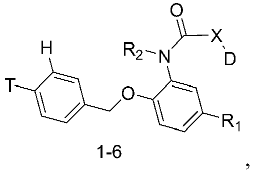
T is selected from a single amino acid, a dipeptide, or a tetrapeptide among cathepsin substrates or metalloproteinase substrates;
Wherein the single amino acid is selected from unprotected or protected single amino acids, PEG chains, polyamino acid chains, and maleimide-modified single amino acids, including -Lys-, -Cit-, -Arg-, -Leu-, -His-, -Asp-, -Glu-, -Ala-, -Val-, -Ile-, -Pro-, -Phe-, -Trp-, -Met-, -Ser-, -Cys-, or -Asn-, etc.; the dipeptide is selected from unprotected or protected dipeptides, PEG chains, polyamino acid chains, and maleimide-modified dipeptides, including -Phe-Lys-, -Val-Lys-, -Ala-Lys-, -Val-Cit-, -Phe-Cit-, -Leu-Cit-, -Ile-Cit, -Trp-Cit-, -Phe-Gly-, -Ala-Ala-, or -Phe-Arg-, etc.; the tetrapeptide is selected from unprotected or protected tetrapeptides, PEG chains, polyamino acid chains, and maleimide-modified tetrapeptides, including -Gly-Gly-Phe-Gly-.

Wherein, in the structural formula (1), when R₁ is a nitro group, the structure of the linker compound is as shown in formula (J) below:

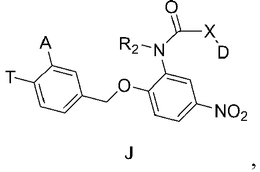

Wherein, in the structural formula (1), when R₁ is a group linked via an amide bond, the linker compound further comprises an attachment site L₁, and the structure of the linker compound is as shown in formula (L) below: 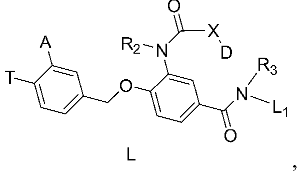 the group attached at the attachment site T is selected from: cathepsin substrate amino acids or short peptide chains, β-glucuronidase substrates, galactosidase substrates, metalloproteinase substrates, aromatic boronic acids or boronic esters oxidizable to hydroxyl groups; nitro, azide, azo and other groups reducible to amino groups; phosphate esters of hydroxyl groups, sulfate esters of hydroxyl groups, etc.

The group attached at the attachment site A is a linking group LR used to regulate the physicochemical properties of the compound or to connect antibodies, polypeptides, albumin, etc.; LR is selected from: H, a fatty chain, a PEG chain, a polyamino acid chain, a sugar chain, and a maleimide-modified PEG chain, a maleimide-modified fatty chain, a maleimide-modified polyamino acid chain, a maleimide-modified sugar chain, etc.;
D is a pharmaceutically active drug, and the active drug D has an exposed -OH, -NH2, -NHR, -SH; including but not limited to anti-tumor drugs, anti-inflammatory drugs, anti-aging drugs, and drugs for treating fibrosis, etc.;
X represents the attachment site in the active drug D, it is O, N, or S;
wherein, R₂ is H or CH₃(CH₂)n, n = 0-20;
R₃ is selected from H, C1-C6 alkyl or cycloalkyl;
L₁ is selected from H, an amino protecting group, C1-C6 alkyl or cycloalkyl, a fatty chain, a linear or branched PEG chain, a sugar chain, a polyamino acid chain, a maleimide-modified PEG chain, a maleimide-modified fatty chain, a maleimide-modified polyamino acid chain, or a maleimide-modified sugar chain;
R₃ and L₁ are capable of linking to form a 3-6 ring, including 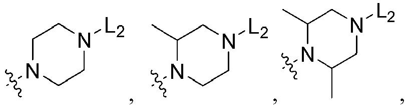 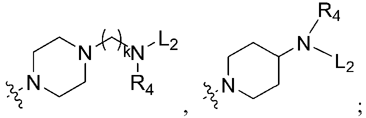
R₄ selected from one or more of H, C1-C6 alkyl, or cycloalkyl;
L₂ selected from one or more of H, an amino protecting group, a C1-C6 alkyl or cycloalkyl group, a fatty chain, a linear or branched PEG chain, a sugar chain, a polyamino acid chain, a maleimide-modified PEG chain, a maleimide-modified fatty chain, a maleimide-modified polyamino acid chain, or a maleimide-modified sugar chain;
the linker compound (L) includes (L-5) and (L-6), respectively as shown in the following formulas: 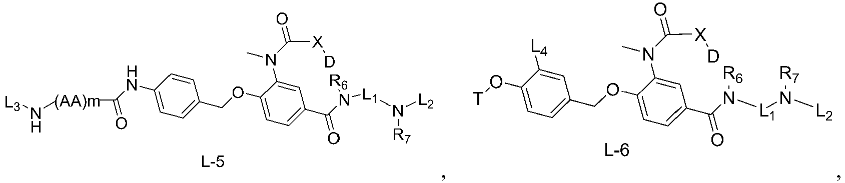 wherein,
   in formula (L-5), AA represents an amino acid, m represents the number of amino acids, and m is selected from 1-6; L₃ is selected from one or more of H, an amino protecting group, a fatty chain, a PEG chain, a polyamino acid chain, a sugar chain, and a maleimide-modified PEG chain, a maleimide-modified fatty chain, a maleimide-modified polyamino acid chain, or a maleimide-modified sugar chain; R₆ and R₇ are selected, either together or independently, from H or C1-C6 alkyl or cycloalkyl; L₁ is selected from C1-C6 alkyl or cycloalkyl, or R₆ and L₁ may form a cycloalkyl group; L₂ is selected from one or more of H, an amino protecting group, a fatty chain, a PEG chain, a polyamino acid chain, a sugar chain, and a maleimide-modified PEG chain, a maleimide-modified fatty chain, a maleimide-modified polyamino acid chain, or a maleimide-modified sugar chain; L₂ and L₃ are selected, either together or independently, from one or more of H, an amino protecting group, a fatty chain, a PEG chain, a polyamino acid chain, a sugar chain, and a maleimide-modified PEG chain, a maleimide-modified fatty chain, a maleimide-modified polyamino acid chain, or a maleimide-modified sugar chain; L₂ and L₃ are not simultaneously selected from a maleimide-modified PEG chain, a fatty chain, a polyamino acid chain, or a sugar chain;
   in formula (L-6), T is selected from one or more of β-glucuronide, β-galactoside, phosphate ester, pyrophosphate ester, or sulfate ester; L₄ is selected from hydrogen, C1-C6 alkyl or cycloalkyl, a nitro group, an amino group, a methoxy group, a carboxyl group, an ester group, a fatty chain, a linear or branched PEG chain, a sugar chain, or a polyamino acid chain 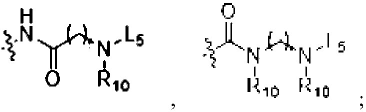 R₁₀ is selected from hydrogen, C1-C3 alkyl, or cycloalkyl; L₅ is selected from hydrogen, an amino protecting group, C1-C6 alkyl or cycloalkyl, a fatty chain, a linear or branched PEG chain, a sugar chain, a polyamino acid chain, a maleimide-modified PEG chain, a maleimide-modified fatty chain, a maleimide-modified polyamino acid chain, or a maleimide-modified sugar chain; R₆ and R₇ are selected, either together or independently, from H or C1-C6 alkyl or cycloalkyl; L₁ is selected from C1-C6 alkyl or cycloalkyl, or R₆ and L₁ may form a cycloalkyl group; L₂ is selected from one or more of H, an amino protecting group, a fatty chain, a PEG chain, a polyamino acid chain, a sugar chain, and a maleimide-modified PEG chain, a maleimide-modified fatty chain, a maleimide-modified polyamino acid chain, or a maleimide-modified sugar chain.

The present invention also provides a covalently conjugated prodrug or antibody-drug conjugate, which is formed by covalently linking the maleimide of the linker compound shown in formula (L) and formula (J) to albumin, an antibody, or an antigen-binding fragment thereof (formed by covalently linking an albumin or antibody fragment), its structure is as shown in the following formulas (L1)-(L8) and formulas (J1)-(J4): 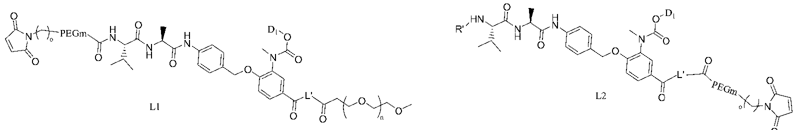 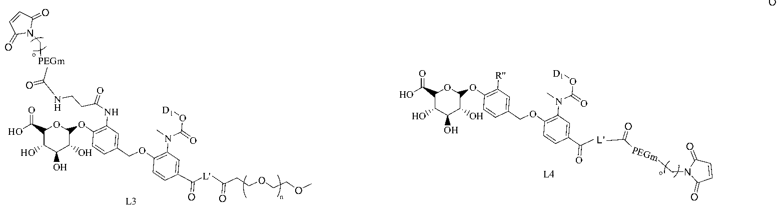 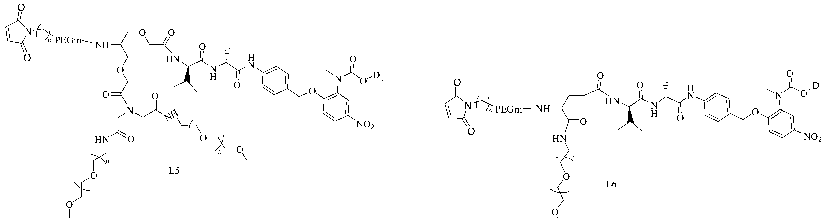 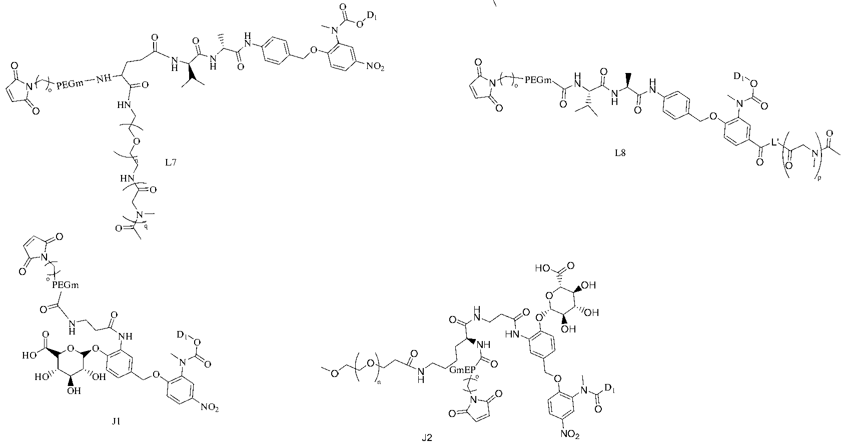 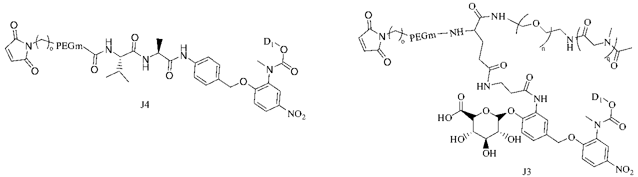
wherein, in the above formulas (L1)-(L8), formula (J1)-formula (J4), n, o, m, p, q represent the number of repeating units, n, p, q is selected from 1-24; o is selected from 2-6; m is selected from 0-12;
in formulas (L1) to (L4) and (L8), L' is selected from one or more of 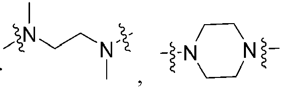 and 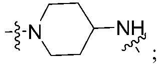
in formula (L2), R' is selected from hydrogen; acetyl; amino protecting groups including Boc, Cbz, Fmoc, Alloc; as well as PEG chains; polyamino acid chains; sugar chains, and fatty chains; in formula (L4), R" is selected from hydrogen, nitro, and methoxy;
D1 represents a hydroxyl-containing drug including camptothecins, and D1 includes:

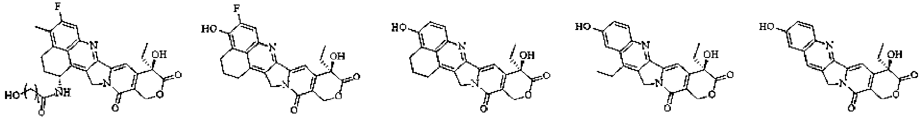

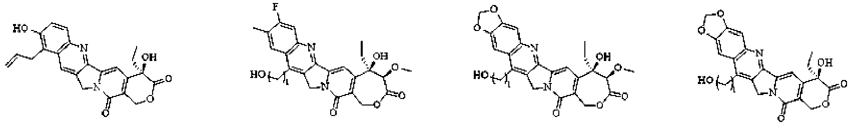

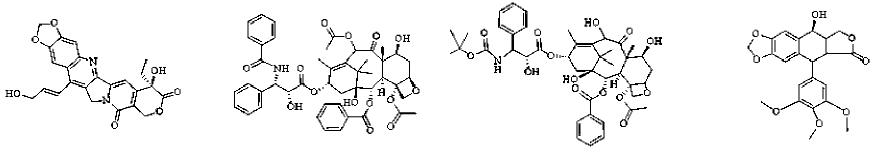

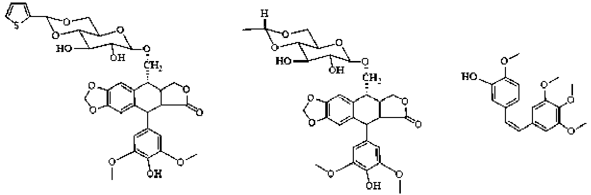

The present invention also provides the covalently conjugated prodrug or antibody-drug conjugate of formulas (L1)-(L8) and formulas (J1)-(J4), including the following formulas (J1-1)-(J1-3), (J2-1)-(J2-2), (J3-1), (J4-1)-(J4-2), (L1-1)-(L1-4), (L2-1), (L3-1), (L4-1), (L5-1)-(L5-3), (L6-1), (L7-1)-(L7-3), (L8-1)-(L8-3): 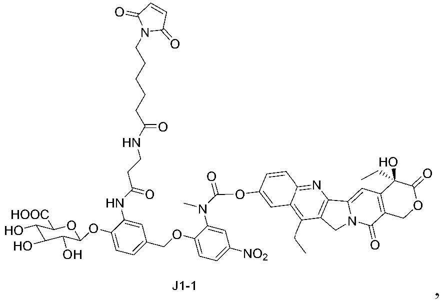 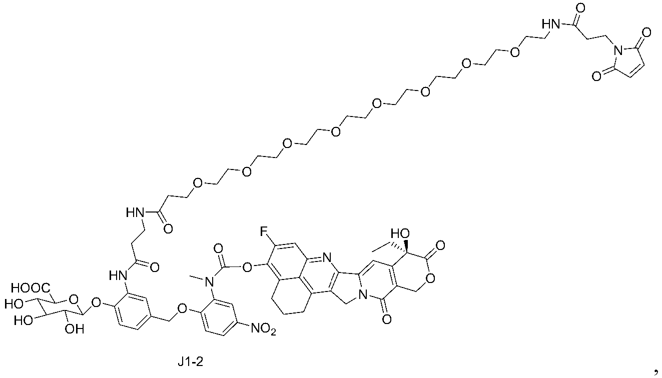 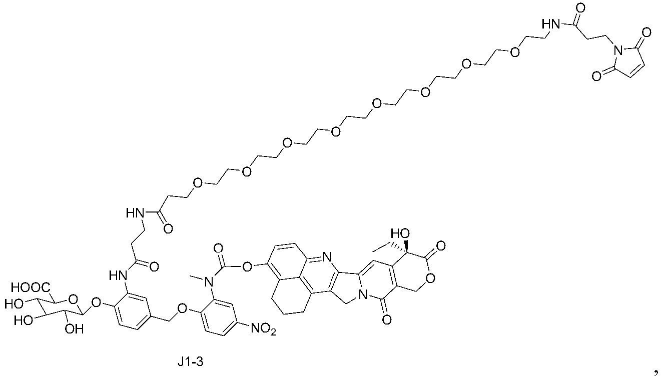 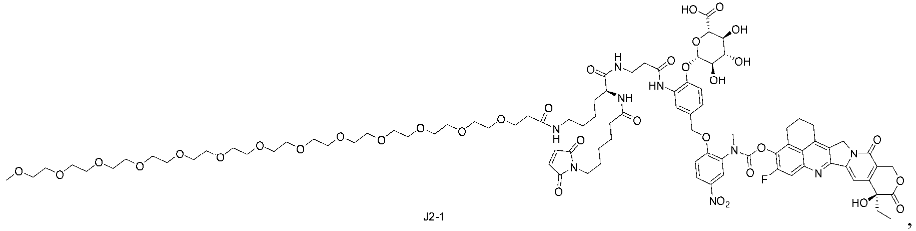 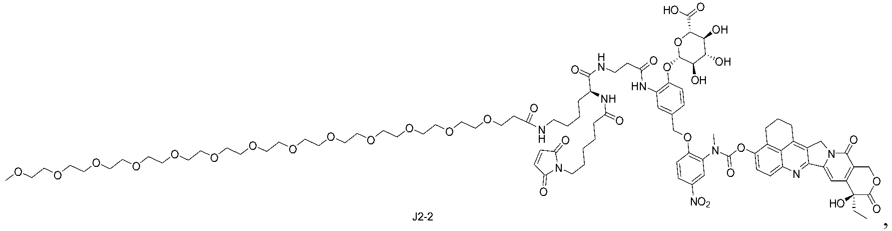 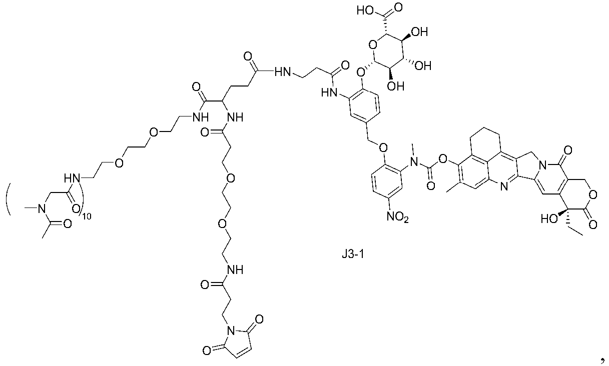 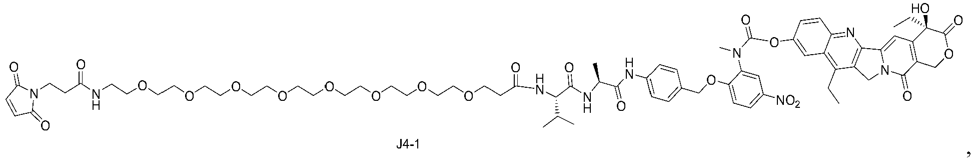 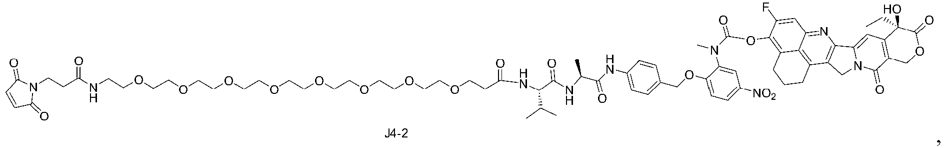 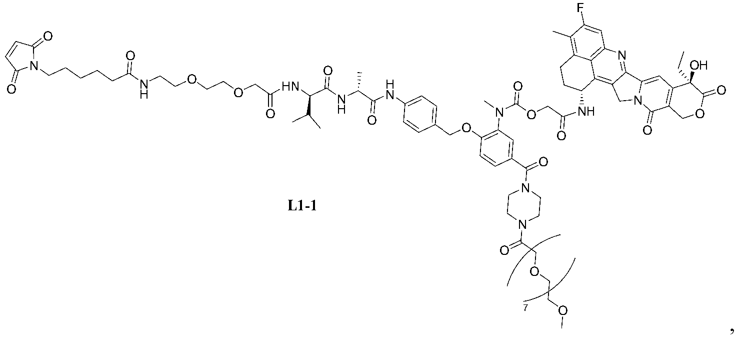 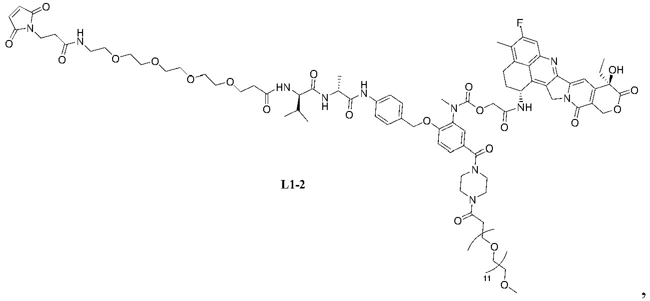 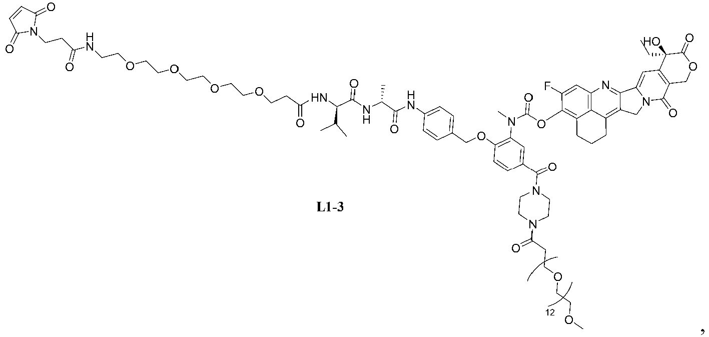 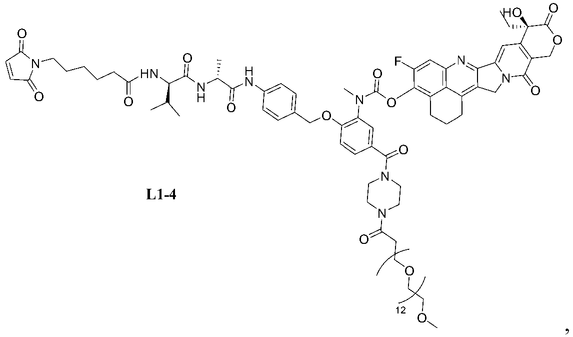 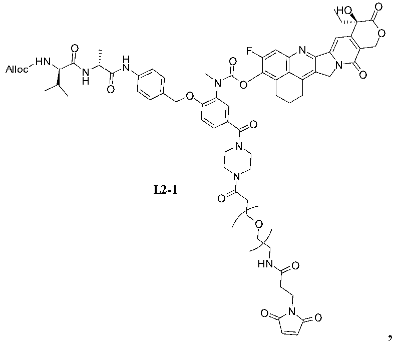 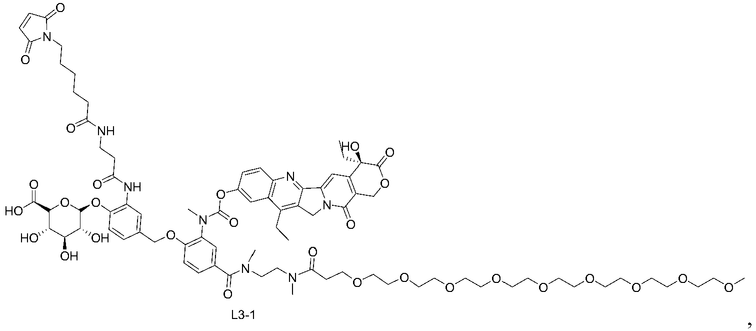 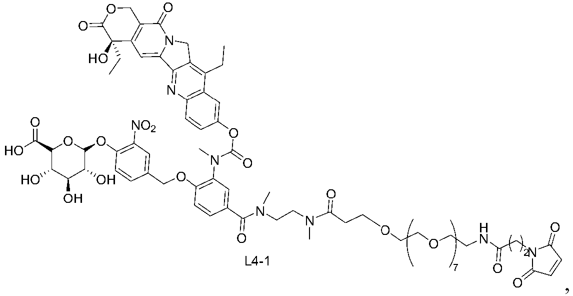 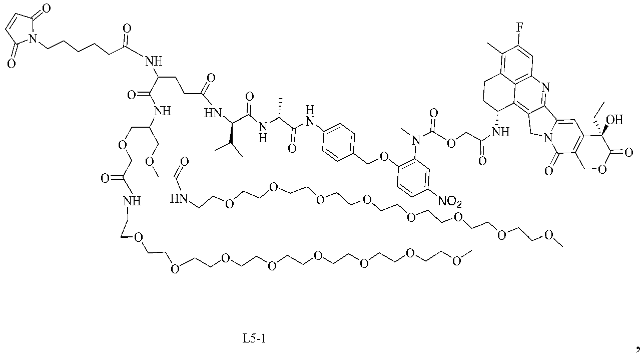 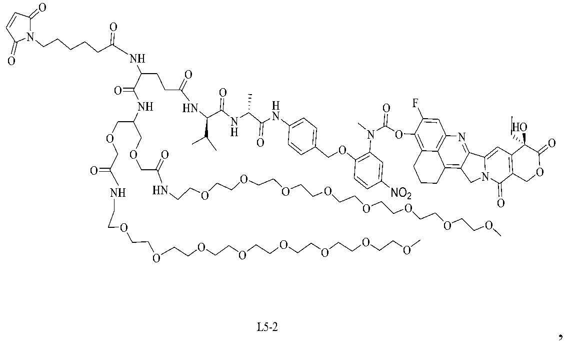 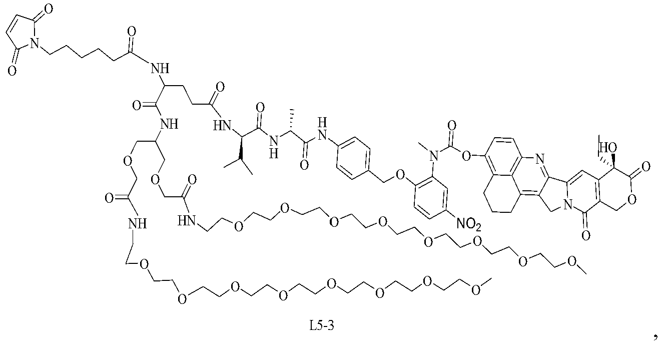 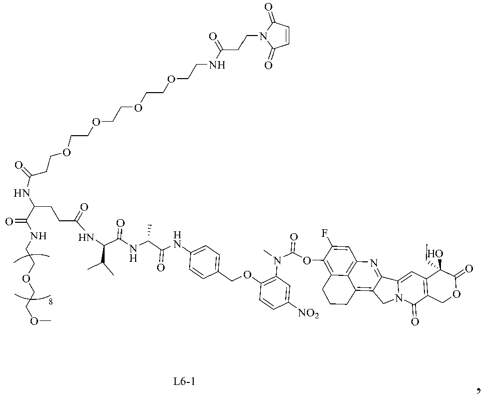 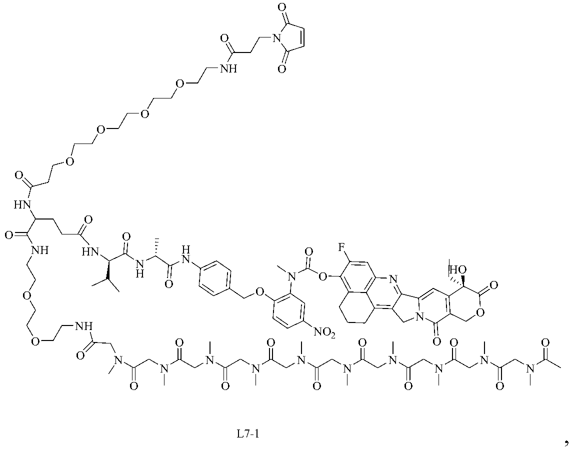 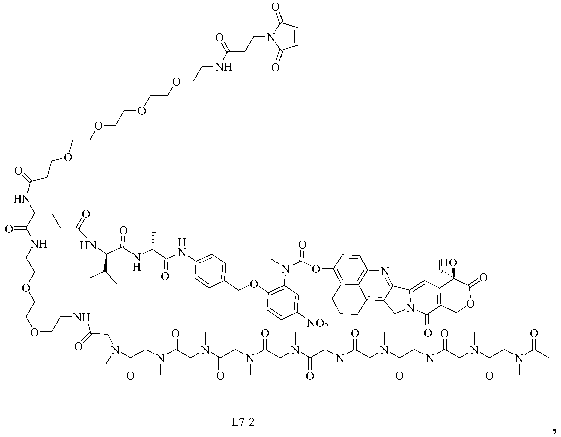 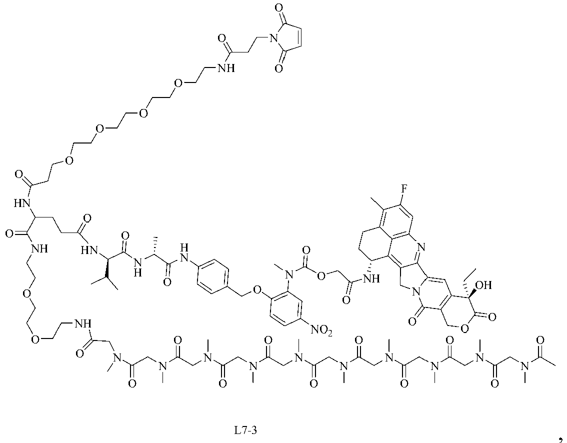 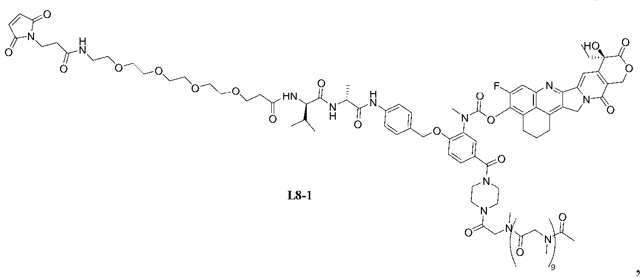 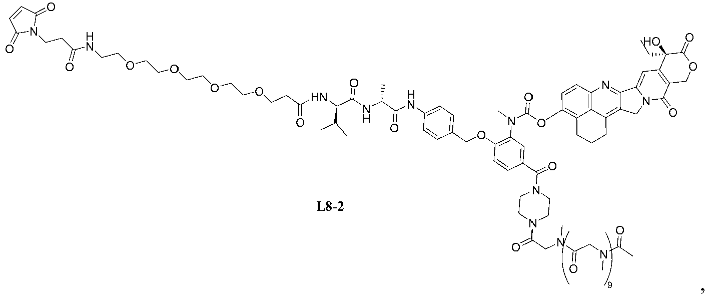 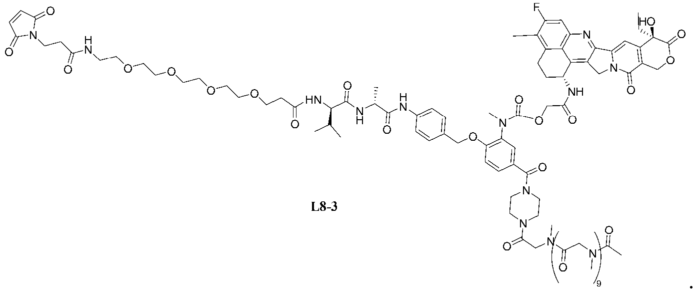 wherein, the antibody or antigen-binding fragment thereof is selected from a rabbit-derived antibody, a mouse-derived antibody, a chimeric antibody, a humanized antibody, a fully human antibody, or a protein fragment with functions similar to an antibody.

Furthermore, the antibody or antigen-binding fragment thereof is selected from: anti-CD3 antibody, anti-FOLR1 antibody, anti-ROR1 antibody, anti-TNFα antibody, anti-TF antibody, anti-EpCAM antibody, anti-EGFRvIII antibody, anti-DLL-3 antibody, anti-PSMA antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-Claudin 6 antibody, anti-Claudin 9 antibody, anti-Claudin 18.2 antibody, anti-Mesothelin antibody, anti-HER2 antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3 antibody, anti-MUC1/CD227 antibody, anti-AXL antibody, anti-CD166 antibody, anti-B7-H3(CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, anti-CD70 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD20 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody and anti-CD123 antibody;
and/or, the antibody or antigen-binding fragment thereof is selected from:
i) Trastuzumab antibody;
ii) ILB-3101 antibody;
iii) 59H4 antibody.

The conjugation ratio of the antibody or antigen-binding fragment thereof to the linker compound is 3-8.

The conjugation ratio is optimized to 6-8 or 4-6.

The present invention also provides a method for preparing the linker compound of formula (L), wherein the preparation reaction process of this method is as shown in the following scheme: 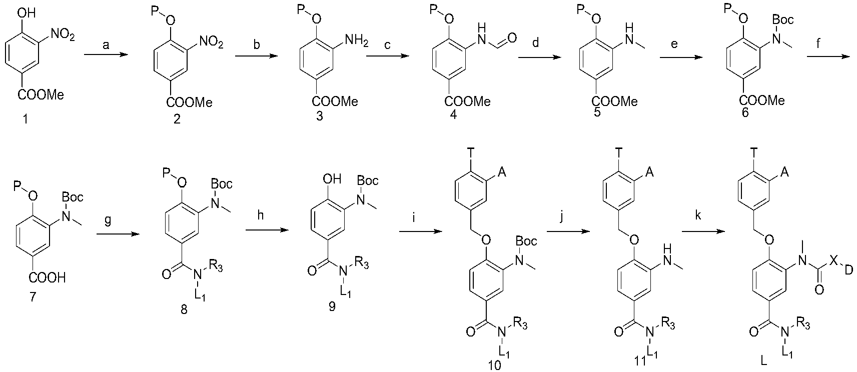 the reaction steps include:
a) substitution reaction: under alkaline conditions, compound 1 undergoes nucleophilic substitution reactions with conventional hydroxyl protecting groups (P), such as bromobenzene, p-methoxy-bromobenzene (MOMCl), etc.; the bases used are potassium carbonate, cesium carbonate, sodium carbonate, etc., and the solvents are acetone, acetonitrile, or N,N-dimethylformamide, etc.;
b) reduction reaction: compound 2 is reduced using a zinc powder/acid or iron powder/acid system to obtain compound 3, with the solvent being a conventional organic solvent/acid;
c) condensation reaction: under a nitrogen atmosphere, compound 3 undergoes a condensation reaction with formic acid and acetic anhydride to generate compound 4;
d) reduction reaction: under a nitrogen atmosphere, compound 4 is dissolved in a solvent and undergoes a reduction reaction with borane dimethyl sulfide complex to generate compound 5;
e) substitution reaction: under alkaline conditions, compound 5 undergoes a substitution reaction with BOC anhydride to obtain compound 6; the base used is DIPEA, DMAP, etc.;
f) hydrolysis reaction: under alkaline conditions, compound 6 undergoes a hydrolysis reaction to obtain compound 7; the base is LiOH, NaOH, and KOH, etc.;
g) condensation reaction: compound 7 undergoes a condensation reaction with an amine in the presence of a condensing agent to generate compound 8; the coupling agent can be DCC, EEDQ, HATU, EDC/HOBt, PyBOP, etc., which are common condensation agents;
h) decomposition reaction: compound 8 undergoes a deprotection reaction with Pd/C to generate compound 9, with the solvent being methanol or a mixed solvent of methanol and tetrahydrofuran and/or ethyl acetate;
i) etherification reaction: under nitrogen protection, different trigger fragment benzyl bromide substrates react in the presence of silver oxide or a base to generate compound 10, with the solvent being anhydrous tetrahydrofuran, anhydrous dichloromethane, or anhydrous acetonitrile;
j) deprotection reaction: compound 10 is dissolved in a solvent and subjected to acidic conditions to remove the N-Boc protection, yielding compound 11; the acid used was hydrochloric acid, trifluoroacetic acid, etc., and the solvent was tetrahydrofuran or dichloromethane, etc.;
k) condensation reaction: under nitrogen protection, compound 11 is dissolved in an organic solvent, bis(trichloromethyl) carbonate and an anhydrous organic base such as triethylamine, DIPEA, pyridine, etc. are added, after reacting for 1 h, the active drug and DMAP are added, and the reaction is carried out at 20-60 °C overnight to obtain compound 12; the drug (D) must have exposed -OH, -NH2, -NHR, -SH groups, and the solvent can be anhydrous tetrahydrofuran or dichloromethane, etc.

In a specific embodiment, it specifically comprises the following steps:
a) substitution reaction: under alkaline conditions, compound 1 undergoes a nucleophilic substitution reaction with a conventional hydroxyl protecting group (P) such as benzyl bromide, p-methoxybenzyl bromide (MOMCl), etc., to generate compound 2; the base is potassium carbonate, cesium carbonate, sodium carbonate, etc.; the solvent is acetone, acetonitrile, or N,N-dimethylformamide, etc.; the reaction temperature is 20-80 °C; the molar ratio of compound 1 to the base is 1:1.5-2, the molar ratio of compound 1 to the protecting group is 1:1.5-2.
b) reduction reaction: compound 2 is reduced using a zinc powder/acid or iron powder/acid system to obtain compound 3; the solvent is a conventional organic solvent/acid; the reaction temperature is 20-80 °C; the molar ratio of compound 2 to iron powder or zinc powder is 1:5.
c) condensation reaction: under a nitrogen atmosphere, compound 3 undergoes a condensation reaction with formic acid and acetic anhydride to generate compound 4; the molar ratio of compound 3 to formic acid is 1:5-10, and the molar ratio of compound 3 to acetic anhydride is 1:5-10; the reaction temperature is 0-80 °C, and the reaction time is 2-3 h; the solvent is anhydrous tetrahydrofuran.
d) reduction reaction: under a nitrogen atmosphere, compound 4 is dissolved in a solvent and undergoes a reduction reaction with borane dimethyl sulfide complex to generate compound 5; the molar ratio of compound 4 to borane dimethyl sulfide complex is 1:3; the reaction temperature is 0-5 °C, and the reaction time is 2-3 h; the solvent is anhydrous tetrahydrofuran.
e) substitution reaction: under alkaline conditions, compound 5 undergoes a substitution reaction with BOC anhydride, etc., to obtain compound 6; the base is DIPEA, DMAP, etc.; the reaction temperature is 20-80 °C; the solvent is BOC anhydride.
f) hydrolysis reaction: under alkaline conditions, compound 6 undergoes a hydrolysis reaction to obtain compound 7; the base is LiOH, NaOH, KOH, etc.; the molar ratio of compound 4 to borane dimethyl sulfide complex is 1:2-5; the reaction temperature is 0-30 °C; the solvent is tetrahydrofuran/methanol/acetonitrile/dioxane, etc., and water.
g) condensation reaction: Compound 7 undergoes a condensation reaction with an amine in the presence of a condensing agent to generate compound 8; the condensing agent can be common condensing agents such as DCC, EEDQ, HATU, EDC/HOBt, PyBOP, etc.; the molar ratio of compound 7 to the amine is 1:1-2; the molar ratio of compound 7 to the condensing agent is 1:2-5; the reaction temperature is 0-30 °C; the solvent is a conventional organic solvent such as dichloromethane, N,N-dimethylformamide, acetonitrile, etc.
h) decomposition reaction: compound 8 undergoes a deprotection reaction with Pd/C, etc., to generate compound 9; the molar ratio of compound 8 to Pd/C is 1:0.1-0.2; the reaction temperature is 0-60 °C; the solvent is methanol or a mixed solvent of methanol and other organic solvents including tetrahydrofuran and/or ethyl acetate.
i) etherification reaction: under nitrogen protection, different trigger fragment benzyl bromide substrates react in the presence of silver oxide or a base to generate compound 10, compound 9 can also react with trigger fragment benzyl alcohol substrates in the presence of a phosphorus reagent and an azo compound to generate compound 10. The molar ratio of compound 9 to different benzyl bromides or benzyl alcohols is 1:1-1.2; the molar ratio of compound 9 to silver oxide/phosphorus reagent/azo compound is 1:1.2-2. The reaction temperature is 0-60 °C; the solvent is anhydrous tetrahydrofuran, anhydrous dichloromethane, or anhydrous acetonitrile.
j) deprotection reaction: compound 10 undergoes N-Boc deprotection under acidic conditions to obtain compound 11; the acid is hydrochloric acid, trifluoroacetic acid, etc.; the reaction temperature is 0-60°C; the solvent is tetrahydrofuran/water or dichloromethane, etc.
l) condensation reaction: under nitrogen protection, compound 11 is dissolved in an organic solvent, bis(trichloromethyl) carbonate and an anhydrous organic base are added, the molar ratio of compound 11 to bis(trichloromethyl) carbonate is 1:0.5, and the molar ratio of compound 11 to the anhydrous organic base is 1:3-5, after reacting for 1 h, the drug (D) and DMAP are added, and the reaction is carried out at 20-60 °C overnight to obtain compound 12, the molar ratio of compound 11 to the drug (D) derivative is 1:1-1.2; the drug (D) must have exposed -OH, -NH₂, -NHR, or -SH; the solvent is anhydrous tetrahydrofuran or dichloromethane, etc.

The present invention also provides a method for preparing a covalently conjugated prodrug or antibody-drug conjugate (J1), wherein the reaction process of the preparation method is as shown in the following scheme: 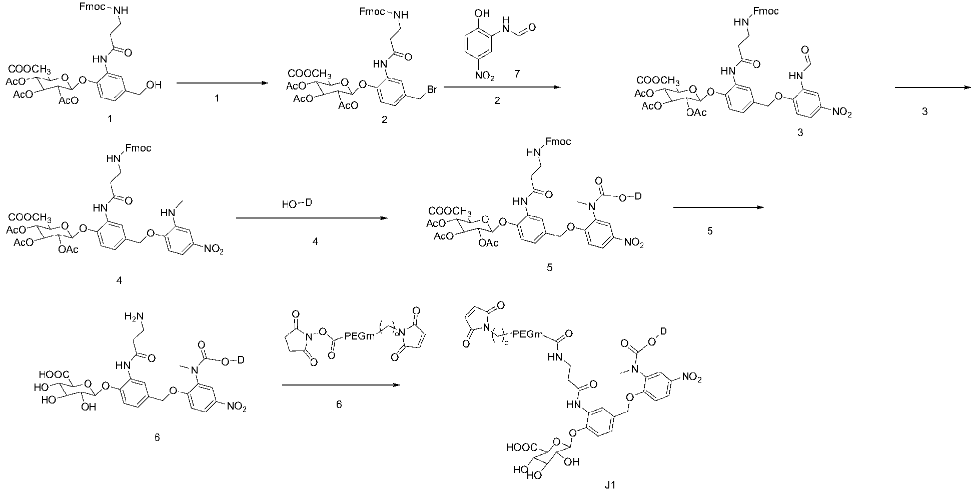 the reaction steps include:
1) bromination reaction: compound 1 undergoes a bromination reaction to obtain compound 2; the bromination reagent includes phosphorus tribromide, triphenylphosphine and carbon tetrabromide, and the solvent is anhydrous dichloromethane;
2) etherification reaction: compound 2 undergoes an etherification reaction to generate compound 3, and the organic solvent is anhydrous acetonitrile;
3) reduction reaction: under a nitrogen atmosphere, compound 3 and borane dimethyl sulfide complex are dissolved in a solvent to undergo a reduction reaction to generate compound 4, and the solvent is anhydrous tetrahydrofuran;
4) condensation reaction: under nitrogen protection, compound 4 is dissolved in an organic solvent, bis(trichloromethyl) carbonate and triethylamine are added. After reacting for 1 h, a hydroxyl-containing drug is added, and the reaction is carried out at room temperature overnight to obtain compound 5;
5) deprotection: under nitrogen protection, compound 5 is dissolved in an organic solvent, and a 0.2 M aqueous alkali solution is added under ice bath conditions for deprotection to obtain compound 6; the alkali can be lithium hydroxide, sodium hydroxide, or potassium hydroxide, or a mixture of these; the organic solvent can be methanol, acetonitrile, tetrahydrofuran, or a mixture of water and these;
6) condensation reaction: compound 6 and compound 9 are dissolved in an organic solvent and stirred at room temperature for 6-8 h to react to generate the prodrug having the structure of general formula (J1); the organic solvent was a mixture of methanol, acetonitrile, tetrahydrofuran, N,N-dimethylformamide, and water, or any one or more of these substances.

In a specific embodiment, it specifically comprises the following steps:
1) bromination reaction: compound 1, triphenylphosphine, and carbon tetrabromide are dissolved in a solvent and reacted at room temperature overnight to obtain compound 2; the molar ratio of compound 1 to triphenylphosphine is 1:3, and the molar ratio of compound 1 to carbon tetrabromide is 1:3; the solvent is anhydrous dichloromethane;
2) substitution reaction: compound 2, N-(2-hydroxy-5-nitrophenyl)formamide, and silver oxide are reacted in an organic solvent to generate compound 3; the molar ratio of compound 2 to N-(2-hydroxy-5-nitrophenyl)formamide is 1:1, and the molar ratio of compound 2 to silver oxide is 1:1.4; the reaction time is 12-14 h. The organic solvent is anhydrous acetonitrile;
3) reduction reaction: under a nitrogen atmosphere, compound 3 and borane dimethyl sulfide complex are dissolved in a solvent to undergo a reduction reaction to generate compound 4; the molar ratio of compound 3 to borane dimethyl sulfide complex is 1:3; the reaction temperature is 0-5 °C, and the reaction time is 2-3 h; the solvent is anhydrous tetrahydrofuran;
4) condensation reaction: under nitrogen protection, compound 4 is dissolved in an organic solvent, bis(trichloromethyl) carbonate and triethylamine are added; the molar ratio of compound 4 to bis(trichloromethyl) carbonate is 1:0.5, and the molar ratio of compound 4 to triethylamine is 1:4, after reacting for 1 h, a camptothecin derivative is added, and the reaction is carried out at room temperature overnight to obtain compound 5; the molar ratio of compound 4 to the camptothecin derivative is 1:1; the camptothecin derivative is SN38;
5) deprotection: under nitrogen protection, compound 5 is dissolved in an organic solvent, a 0.2M aqueous alkali solution is added under ice bath conditions for deprotection to obtain compound 6; the reaction time is 0.2-1 h; the molar ratio of compound 5 to the base is 1:8; the base is one or a mixture of lithium hydroxide, sodium hydroxide, and potassium hydroxide; the organic solvent is one or a mixture of methanol, acetonitrile, tetrahydrofuran, and water;
6) condensation reaction: compound 6 and compound 9 are dissolved in an organic solvent and stirred at room temperature for 6-8 h to react to generate the prodrug having the structure of general formula (J-1); the molar ratio of compound 6 to compound 9 is 1:1; the organic solvent is one or a mixture of methanol, acetonitrile, tetrahydrofuran, N,N-dimethylformamide, and water.

Another object of the present invention is to provide a possible drug release mechanism under different conditions when a ligand, a trigger, and a drug are connected to the linker to form a targeted drug conjugate.

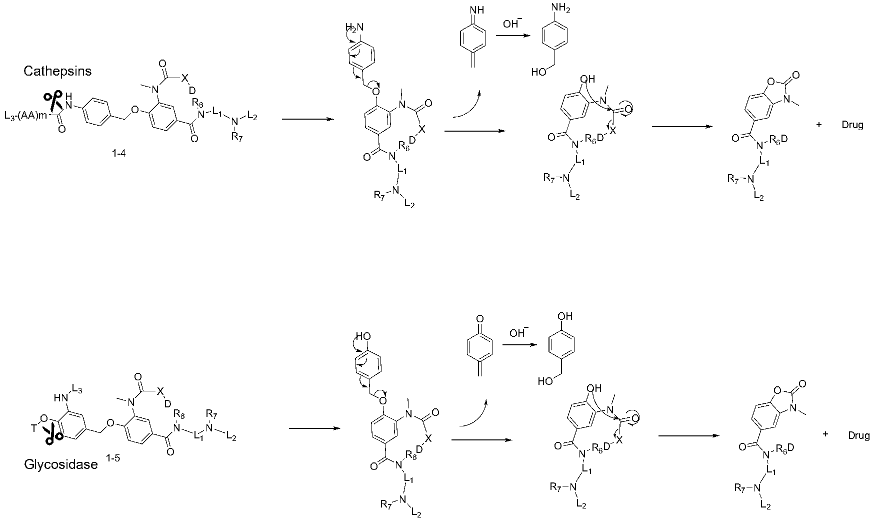

This linker is hydrolyzed by cathepsin to form an amino group via peptide bond cleavage, or by β-glucuronidase or galactosidase to form a hydroxyl group via glycosidic bond cleavage; subsequently, a 1,6-self-immolation occurs, followed by a self-cyclization process to release the corresponding active drug.

The present invention also proposes a drug and a pharmaceutical composition, comprising the linker compound, its pharmaceutically acceptable salts, solvates, polymorphs, isomers, derivatives, or the covalently conjugated prodrug or antibody conjugate, as well as pharmaceutically acceptable excipients, diluents, or carriers thereof.

The present invention also proposes an application, wherein the linker compound (1) includes but is not limited to compounds such as formula (J1-1), formula (J4-1), formula (L4-1), formula (L7-1), formula (L1-1), formula (L7-2), formula (L7-3), formula (L7-4), etc., and/or the covalently conjugated prodrug or antibody-drug conjugate, as well as drugs and/or pharmaceutical compositions containing the aforementioned compounds, for use in the preparation of anti-tumor drugs, and in the preparation of albumin-binding anti-tumor drugs; further includes applications in the preparation of drugs for tumor therapy using albumin-binding β-glucuronidase prodrugs, and for tumor therapy using antibody-drug conjugates.

The tumors are solid tumors or hematologic malignancies such as breast cancer, ovarian cancer, ovarian teratoma, cervical cancer, uterine cancer, prostate cancer, kidney cancer, urethral cancer, testicular cancer, bladder cancer, liver cancer, gastric cancer, endometrial cancer, salivary gland cancer, esophageal cancer, head and neck cancer, lung cancer, colon cancer, rectal cancer, colorectal cancer, bone cancer, skin cancer, thyroid cancer, pancreatic cancer, melanoma, neurological tumors, glioma, neuroblastoma, glioblastoma multiforme, squamous cell carcinoma, myeloma, sarcoma, lymphoma, and leukemia.

The present invention has advantages and beneficial effects including, but not limited to: the present invention proposes a novel cleavable linker of formula (I) that can be used for prodrugs. This linker has multiple independently modifiable sites, allowing different triggers, modifying side chains, and/or active drugs to be combined to form a new compound. This makes the active drug more stable, less toxic, and increases its targeting capability while still retaining its activity. The present invention provides an albumin-binding camptothecin derivative prodrug that is cleaved by β-glucuronidase, triggering the self-immolation of this linker, as well as its preparation method and application.

In specific embodiments, it has been experimentally demonstrated that compounds of formula (J) constructed with this linker, including but not limited to formula (J1-1) and formula (J4-1), possess excellent in vitro and in vivo stability and can rapidly release the active drug under conditions simulating the tumor microenvironment. Binding to albumin effectively improves the in vivo metabolic time. Among them, compound (J1-1) significantly prolonged the survival period and inhibited tumor growth in an anti-tumor experiment using an HCT116 xenograft mouse model (NCG or equivalent). This indicates that the novel linker and the compounds constructed based on it can achieve the goal of rapid drug release at the tumor site, improve the druggability of the drug, and enhance its anti-cancer efficacy.

In a specific embodiment, it has been experimentally demonstrated that compounds of formula (J) constructed with this linker, including but not limited to formula (J1-2), formula (J1-3), formula (J2-1), formula (L1-3), formula (L1-4), formula (L5-1), and formula (L5-3), when conjugated with multiple antibodies, yield ADC drugs with different DAR values. These exhibit anti-tumor activity in various cell lines in vitro and demonstrate strong anti-tumor effects in multiple mouse CDX models, with some being superior to the positive control ADC. This indicates that the novel linker and the compounds constructed based on it can exhibit excellent anti-tumor activity after antibody conjugation and can be used for the treatment of various cancers such as lung cancer, breast cancer, and colon cancer.

The prodrug compounds and linker compounds described in the present invention have the same meaning as linker-payload.

The antibody is selected from the invention patent CN 2023108539993, including:
3101 antibody, which is clone 7B7, an anti-human B7-H3 antibody, with the heavy chain and light chain sequences as follows:
Heavy chain sequence of 3101: 
EVQLVESGGGLVKPGGSLRLSCAASGFTFSSYAVSWVRQAPGKGLEWVASISGGGIYIYYPDSVKGR
FTISRDNAKNSLYLQMNSLRAEDTAVYYCARHGGAGYFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO.1)
Light chain sequence of 3101:

DSQMTQSPSSLSASVGDRVTITCRGSESVHSYLAWYQQKPGKAPKLLVYNAKTLQSGVPSRFSGSGS
GTDFTLTISSLQPEDFATYYCQQHYGSPPWTFGGGTKVEIKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPVTKS
FNRGEC (SEQ ID NO.2)

Trastuzumab, an anti-human HER2 antibody, with the antibody heavy chain and light chain sequences as follows:
Heavy chain sequence of Trastuzumab: 
EVQLVESGGGLVQPGGSLRLSCAASGFNIKDTYIHWVRQAPGKGLEWVARIYPTNGYTRYADSVKG
RFTISADTSKNTAYLQMNSLRAEDTAVYYCSRWGGDGFYAMDYWGQGTLVTVSSASTKGPSVFPLAPSS
KSTSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDGSFFLYSKLTVDK
SRWQQGNVFSCSVMHEALHNHYTQKSLSLSPGK (SEQ ID NO.3)
Light chain sequence of Trastuzumab:

DIQMTQSPSSLSASVGDRVTITCRASQDVNTAVAWYQQKPGKAPKLLIYSASFLYSGVPSRFSGSRSG
TDFTLTISSLQPEDFATYYCQQHYTTPPTFGQGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO.4)

59H4 clone, an anti-human DLL3 antibody, selected from invention patent CN 2023108539993, with the sequence as follows:
Heavy chain sequence of 59H4: 
EVQLVQSGSELKKPGASVKVSCKASGYTFTNYGMNWVRQAPGQGLEYMGWINTYTGEPTYGEDFK
GRFVFSLDTSVSTAYLQISSLKAEDTAVYYCAKKGDSTVVDFDVWGQGTTVTVSSASTKGPSVFPLAPSSK
STSGGTAALGCLVKDYFPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQP
REPQVYTLPPSRDELTKNQVSLTCLVKGFYPSDIAVEWESNGQPENNYKTTPPVLDSDGSFFLYSKLTVDK
SRWQQGNVFSCSVMHEALHNHYTQKSLSLSPGK (SEQ ID NO.5)
Light chain sequence of 59H4:

DIVLTQSPDSLAVSLGERATINCKASQSVDYDGDSYMNWYQQKPGQPPKLLIYAASILESGIPDRFSGS
GSGTDFTLTISSLQAEDVAVYYCQQSYEDPLTFGQGTKLEIKRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSPVT
KSFNRGEC (SEQ ID NO.6)

The antibody for DS-7300 is derived from Daiichi Sankyo's invention patent US9808537.

An antiviral antibody is used as a negative control antibody, which does not bind to the target antigen.

The Isotype antibody sequence is as follows:
Heavy chain sequence of Isotype: 
QVQLVESGGGLVKPGGSLRLSCAASGFTFSDYYMSWIRQAPGKGLEWVSYITYSGSTIYYADSVKGR
FTISRDNAKSSLYLQMNSLRAEDTAVYYCARDRGTTMVPFDYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSFFLYSKLTVDKSR
WQQGNVFSCSVMHEALHNHYTQKSLSLSPGK (SEQ ID NO.7)
Light chain sequence of Isotype:

DIQMTQSPSSLSASVGDRVTITCQASQDITNYLNWYQQKPGKAPKLLIYAASNLETGVPSRFSGSGSG
TDFTFTISGLQPEDIATYYCQQYDNLPLTFGGGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO.8)

All the aforementioned antibodies can be expressed and purified according to the method described in the invention patent document CN 2023108539993, and used for conjugate preparation.

The linker-payload for the positive control conjugate can be Deruxtecan (CAS 1599440-13-7), as described in US9808537, which is commercially available or can be synthesized according to the patent method.

The linker-payload for the positive control conjugate can be AZ0133, which is compound SG3932 described in patent WO2022/053685, i.e., the linker-payload of AZD8205, commercially available or synthesizable according to the patent method.

The antibodies were respectively conjugated with the linker compound of the present invention, Deruxtecan, and AZ0133 to prepare conjugates for in vitro and in vivo activity evaluation.

The present invention innovatively proposes a novel cleavable linker compound for use in a prodrug delivery system, wherein the trigger fragment is connected to the novel linker via a para-hydroxy or para-amino group, the active drug is connected to the novel linker via its hydroxyl moiety, and the modifying fragment is connected to the novel linker via an amino group. Based on its abundant modification sites, different modification methods can be selected to adapt to different administration requirements, achieving the goals of improving targeting, reducing the toxicity of the active drug, and enhancing druggability. Upon administration of the present invention, the trigger fragment is released at specific targets in the body, the modifying fragment ensures stable long circulation in the body, and the active drug moiety provides the therapeutic effect, ultimately achieving accumulation at the target site and targeted drug release, while reducing the toxic side effects of the active drug and improving the druggability of the drug.

### Description of the Drawings

To explain the technical solutions in the embodiments of the present invention or in the prior art more clearly, the following briefly introduces the drawings required for describing the embodiments or the prior art. Obviously, the drawings in the following description are only some embodiments of the present invention. For those skilled in the art, other drawings can be obtained based on these drawings without involving any inventive effort.
Figure 1 shows the stability of the prodrug compound (J1-1) according to an embodiment of the present invention in PBS and mouse plasma.
Figure 2 shows the drug release rate of the prodrug compound (J1-1) according to an embodiment of the present invention under the action of β-glucuronidase.
Figure 3 shows the inhibition of cell growth by the prodrug compound (J1-1) according to an embodiment of the present invention in the presence of β-glucuronidase.
Figure 4 is a graph showing the time versus tumor volume change in an in vivo anti-tumor experiment of the prodrug compound (J1-1) according to an embodiment of the present invention.
Figure 5 A, B, and C are graphs showing the time versus tumor volume change in in vivo anti-tumor experiments of B7-H3 ADC according to embodiments of the present invention.
Figure 6 A and B are graphs showing the time versus tumor volume change in in vivo anti-tumor experiments of B7-H3 ADC according to embodiments of the present invention.
Figure 7 is a graph showing the time versus tumor volume change in an in vivo anti-tumor experiment of HER2 ADC according to an embodiment of the present invention.
Figure 8 is a graph showing the time versus tumor volume change in an in vivo anti-tumor experiment of DLL3 ADC according to an embodiment of the present invention.
Figure 9 shows the inhibitory activity of the payload according to an embodiment of the present invention on tumor cell growth in vitro.
Figure 10 shows the inhibitory activity of the conjugate according to an embodiment of the present invention on tumor cell growth in vitro.

### PREFERRED EMBODIMENTS OF THE INVENTION

The present invention will be further described in detail in conjunction with the following specific examples and drawings. The processes, conditions, experimental methods, etc. for implementing the present invention, except for the content specifically mentioned below, are common knowledge and general knowledge in the art, and the present invention is not particularly limited thereto.

### Example 1 Synthesis of compound J1-1

### The synthesis of J1-1 was performed according to Route A.

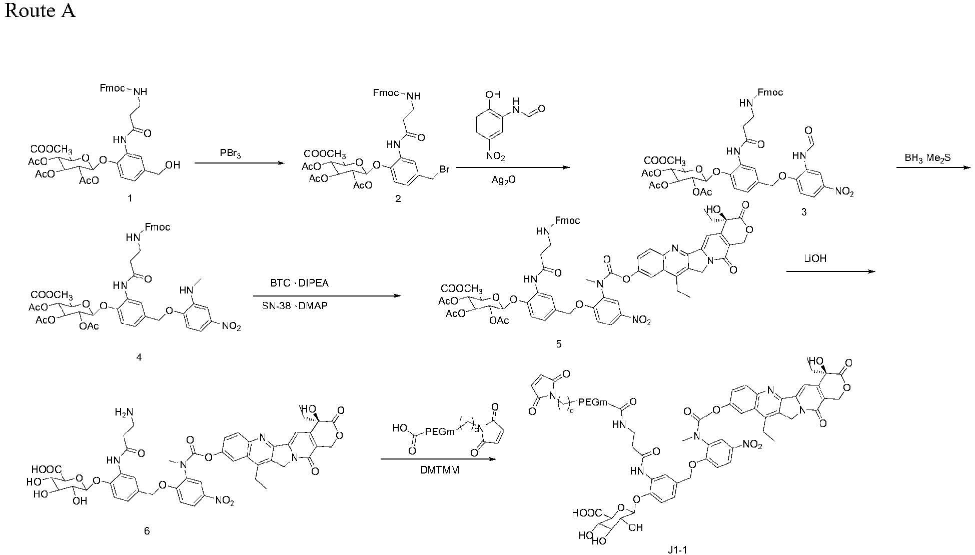

### Synthesis of Compound 2

Carbon tetrabromide (1.77 g, 5.36 mmol) was weighed into a three-necked flask, purged with nitrogen, and 20 mL of anhydrous dichloromethane was injected. A solution of triphenylphosphine (1.4 g, 5.36 mmol) in anhydrous dichloromethane was added dropwise under ice bath. The mixture was stirred for 30 min, then a solution of Compound 1 (1 g, 1.34 mmol) in anhydrous dichloromethane was added dropwise, and the reaction was carried out at room temperature overnight. The reaction was completed as monitored by TLC, and concentrated under reduced pressure to give a yellow oily liquid. Purification by column chromatography (dichloromethane:methanol = 100:1) afforded 750 mg of a pale yellow fluffy solid with a yield of 83.9%.1H NMR(400 MHz, CDCl3) δ 8.45 (d,J = 20.7 Hz, 1H), 8.03 (d,J = 21.8 Hz, 1H), 7.73 (dd,J = 21.2, 7.0 Hz, 2H), 7.57 (d,J = 13.9 Hz, 2H), 7.41 - 7.28 (m, 3H), 7.21 (s, 2H), 7.06 (dd,J =21.1, 8.2 Hz, 1H), 6.89 (dd,J = 21.4, 8.3 Hz, 1H), 5.60 (s, 1H), 5.41 - 5.21 (m, 3H), 5.03 (dd,J = 21.1, 6.9 Hz, 1H), 4.44 (d,J = 21.7 Hz, 2H), 4.39 - 4.16 (m, 3H), 3.71 (d,J = 21.5 Hz, 3H),3.55 (s, 2H), 2.68 (s, 2H), 2.02 (d,J = 18.8 Hz, 9H).

### Synthesis of Compound 3

Silver oxide (300 mg, 1.29 mmol), 2-hydroxy-5-nitrobenzamide (186 mg, 1.02 mmol), and Compound 2 (750 mg, 0.92 mmol) were weighed into a three-necked flask, purged with nitrogen, and 10 mL of anhydrous dichloromethane was injected. The reaction was carried out at room temperature overnight. The reaction was completed as monitored by TLC, filtered through Celite, and the filtrate was collected and concentrated under reduced pressure to afford the crude product. Purification by column chromatography (dichloromethane:methanol = 100:1 to 50:1) afforded 500 mg of a white fluffy solid with a yield of 52.2%.1H NMR(400 MHz, CDCl3) δ 9.27 (d,J = 17.6 Hz, 1H), 8.47 (t,J = 21.4 Hz, 2H), 8.12 (d,J = 19.8 Hz, 1H), 8.02 - 7.91 (m, 2H), 7.52 (s, 3H), 7.45 (s, 3H), 7.34 (d,J = 11.2 Hz, 1H), 7.22 (s, 2H),7.06 - 6.88 (m, 3H), 5.43 - 5.35 (m, 1H), 5.29 (d,J = 7.9 Hz, 2H), 5.12 (d,J = 20.3 Hz, 3H), 4.34 (s, 2H), 4.17 (s, 2H), 3.71 (d,J = 14.6 Hz, 3H), 3.56 (s, 2H), 2.70 (s, 2H), 2.03 (d,J = 17.1 Hz, 9H).

### Synthesis of Compound 4

Under strict nitrogen protection, Compound 3 (500 mg, 0.55 mmol) was weighed into a three-necked flask, purged with nitrogen, and 10 mL of anhydrous tetrahydrofuran was added. Borane dimethyl sulfide (0.875 mL, 1.64 mmol) was slowly added dropwise in an ice-salt bath, and the reaction was carried out at room temperature for 3 hours. The reaction was completed as monitored by TLC, and concentrated under reduced pressure to give 420 mg of a yellow solid with a yield of 85.4%.

### Synthesis of Compound 5

Compound 4 (100 mg, 0.11 mmol) and triphosgene (12.56 mg, 0.04 mmol) were weighed into a three-necked flask, purged with nitrogen, and 10 mL of anhydrous tetrahydrofuran was injected. DIEA (51.7 mg, 0.42 mmol) was slowly added dropwise under ice bath, and the reaction was carried out at room temperature for 1 hour. SN38 (48 mg, 0.12 mmol) was added to the reaction mixture, and the mixture was stirred at 40 °C overnight. The reaction was completed as monitored by TLC. 10 mL of dichloromethane was added, washed twice with 1 M dilute hydrochloric acid and once with saturated sodium chloride solution. The organic phase was dried over anhydrous sodium sulfate and concentrated under reduced pressure to give the crude product. Purification by column chromatography (dichloromethane:methanol = 100:1 to 50:1) afforded 118 mg of a yellow solid with a two-step yield of 81.0%.1H NMR(400 MHz, CDCl3) δ 8.56 (s, 1H), 8.33 (s, 1H), 8.28 - 8.22 (m, 1H), 8.19 - 8.06 (m, 2H), 7.84 (s, 1H), 7.64 (t,J = 22.0 Hz, 5H), 7.47 (d,J = 6.8 Hz, 2H), 7.30 (d,J = 8.0 Hz, 2H), 7.21 -7.06 (m, 4H), 7.01 (s, 1H), 5.97 (s, 1H), 5.73 (d,J = 16.2 Hz, 1H), 5.50 - 5.36 (m, 1H), 5.35 - 5.26 (m, 4H), 5.22 (d,J = 14.3 Hz, 3H), 5.14 (d,J = 7.0 Hz, 3H), 4.33 - 4.18 (m, 3H), 3.61 (s, 2H), 3.40 (s, 3H), 3.15 (dd,J = 15.2, 7.5 Hz, 1H), 2.97 (s, 2H), 2.75 (s, 2H), 2.06 (s, 9H), 1.86 (dd,J = 14.0, 7.0 Hz, 2H), 1.40 (t,J = 7.7 Hz, 3H), 1.01 (dd,J = 15.6, 7.5 Hz, 3H). 13C NMR (101 MHz, CDCl3) δ 173.89, 173.80, 170.47, 170.44, 169.82, 169.45, 166.70, 159.20, 157.63, 157.51, 156.50, 150.19, 146.93, 146.84, 146.56, 143.88, 141.45, 141.11, 131.86, 131.61, 129.51, 127.53, 127.37, 127.30, 127.19, 126.94, 125.06, 124.98, 119.74, 118.62, 114.36, 114.16, 112.63, 112.61, 98.16, 98.14, 72.87, 72.44, 71.25, 71.01, 70.84, 69.17, 66.64, 66.30, 66.25, 53.48, 53.25, 49.43, 49.33, 47.06, 37.90, 37.08, 36.96, 31.59, 31.50, 23.18, 23.03, 20.85, 20.58, 20.49, 14.00, 13.93, 7.86.

### Synthesis of Compound 6

Compound 5 (118 mg, 0.09 mmol) was weighed into a flask under ice bath, 1 mL of water and 1 mL of acetonitrile were added, and 0.5 mL of 1 M aqueous lithium hydroxide solution was slowly added dropwise. The reaction was carried out at room temperature for 1 h. The reaction was completed as monitored by LC-MS, and concentrated under reduced pressure to give 66 mg of a yellow solid with a yield of 92.1%.

### Synthesis of Compound (J1-1)

Compound 6 (66 mg, 0.07 mmol) and 6-maleimidohexanoic acid N-hydroxysuccinimide ester (53 mg, 0.17 mmol) were weighed into a flask, 2 mL of acetonitrile and 1 mL of methanol were added, and the pH of the system was adjusted to 8 with DIEA. The reaction was carried out at room temperature for 5 h. The reaction was completed as monitored by LC-MS, and concentrated under reduced pressure. Purification by reversed-phase column chromatography (0.1% aqueous trifluoroacetic acid:acetonitrile = 15:1 to 30:1), concentration under reduced pressure, and lyophilization afforded 45 mg of a yellowish-white solid with a yield of 59.2%.1H NMR(400 MHz, DMSO) δ 12.81 (s, 1H), 9.12 (s, 1H), 8.53 (s, 1H), 8.34 (s, 1H), 8.28 (s, 1H), 8.12 (d,J = 8.6 Hz, 1H), 7.87 (s, 1H), 7.80 (s, 1H), 7.49 (s, 2H), 7.32 (s, 1H), 7.22 (d,J = 7.6 Hz,1H), 7.15 (d,J = 7.2 Hz, 1H), 6.98 (s, 1H), 6.53 (s, 1H), 5.87 (s, 1H), 5.44 (s, 2H), 5.36 (d,J = 31.7 Hz, 4H), 4.93 (d,J = 6.1 Hz, 1H), 3.93 (d,J = 9.1 Hz, 1H), 3.42 (d,J = 7.3 Hz, 10H), 3.30 (s, 3H),3.06 (s, 2H), 2.00 (d,J = 5.0 Hz, 2H), 1.93 - 1.80 (m, 2H), 1.44 (d,J = 6.6 Hz, 4H), 1.22 (d,J = 9.0 Hz, 3H), 1.15 (d,J = 7.3 Hz, 2H), 0.88 (s, 3H). 13C NMR (101 MHz, DMSO) δ 172.96, 172.59, 171.53, 170.50, 170.14, 159.64, 157.28, 153.73, 152.28, 150.50, 150.13, 146.79, 146.54, 146.39, 145.76, 145.68, 141.14, 134.89, 131.86, 131.61, 130.40, 129.35, 128.99, 127.42, 127.36, 125.18, 123.41, 120.90, 119.41, 116.70, 115.35, 114.37, 102.04, 97.09, 75.93, 75.44, 73.33, 72.86, 71.81, 71.11, 65.74, 50.03, 37.88, 37.40, 36.98, 35.56, 35.46, 30.74, 28.22, 26.25, 25.19, 22.57, 14.24, 8.25.

### Example 2 Stability Test

The PBS solution (1 mM, pH 7.4) of Compound (J1-1) prepared in Example 1 of the present invention was incubated at 37 °C. Corresponding samples (n=3) were taken on Day 1, Day 2, Day 3, Day 5, and Day 7, and the contents of (J1-1) and SN38 were determined by HPLC.

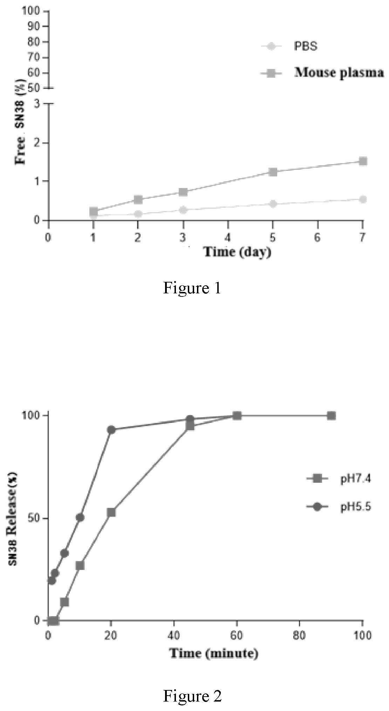

Mouse plasma stability: 40 µL of the PBS solution (1 mM, pH 7.4) of Compound (J1-1) prepared in Example 1 of the present invention was mixed with 360 µL of mouse plasma and incubated in a shaker at 37 °C. Corresponding samples (n=3) were taken on Day 1, Day 2, Day 3, Day 5, and Day 7, precipitated with cold acetonitrile, centrifuged at 4 °C for 30 min, and the supernatant was collected. The content of SN38 was determined by HPLC. The experimental results are shown in Figure 1. The results indicated that the release of SN38 from Compound (J1-1) prepared in Example 1 of the present invention in mouse plasma with PBS at pH 7.4 was less than 2% within seven days, suggesting that Compound (J1-1) prepared in Example 1 of the present invention exhibited excellent stability, demonstrating that the phenolic hydroxyl prodrug design involved in the present invention was sufficiently stable.

### Example 3 Enzyme Release Assay

Compound (J1-1) solution (100 µM, pH 7.4 or pH 5.5) prepared in Example 1 of the present invention was mixed with β-glucuronidase (300 U) and incubated in a shaker at 37 °C. Corresponding samples (n=3) were taken at 1 min, 2 min, 5 min, 10 min, 20 min, 45 min, 60 min, and 90 min, precipitated with cold acetonitrile, centrifuged at 4 °C for 15 min, and the supernatant was collected. The content of SN38 was determined by HPLC.

The experimental results are shown in Figure 2. The results indicated that 100% release of SN38 was achieved triggered by β-glucuronidase within 45 min at either pH 7.4 or pH 5.5, suggesting that Compound (J1-1) prepared in Example 1 of the present invention could rapidly release the drug and convert the prodrug into antitumor-active SN38. The complete release of phenolic hydroxyl-protected SN38 was unexpected.

### Example 4 In Vitro drug Cytotoxicity Assay

The in vitro cytotoxicity of Compound (J1-1) prepared in Example 1 of the present invention against HCT116, A549, MCF-7, HepG2, and 4T1 cells, as well as that in the presence of β-glucuronidase, was determined by MTT assay. A total of 200 µL of culture medium containing 4000 cells was seeded into each well of a 96-well plate. After incubation for adherence for 24 h in an incubator (37 °C, 5% CO₂), the culture medium was removed, and 200 µL of drug solutions at various concentrations with or without β-glucuronidase supplementation was added, followed by incubation for 72 h. The medium was removed, 100 µL of MTT solution (0.5 mg/mL) was added to each well, and the plate was incubated for 3 h. Subsequently, 100 µL of triple solution was added to each well, and the plate was incubated for 12 h. The absorbance (OD) at 570 nm was measured using a microplate reader. Cell viability was calculated according to the following formula: $Cell viability % = \frac{{OD}_{sample} - {OD}_{blank}}{{OD}_{control} - {OD}_{blank}} \times 100 %$

*ODₛₐₘₚₗₑ*is the absorbance of the drug-treated group, *OD_{blank}* is the absorbance of complete medium alone, *OD_{control}* is the absorbance of the cell-seeded group without drug treatment.

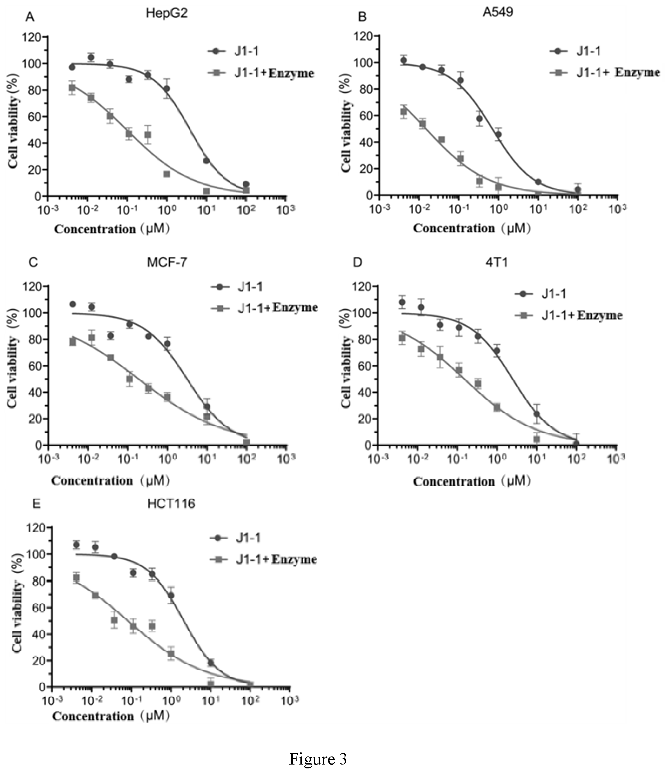

The experimental results are shown in Figure 3. The results demonstrated that Compound (J1-1) prepared in Example 1 of the present invention exhibited low cytotoxicity at the cellular level, and could release the active drug upon β-glucuronidase triggering, thereby achieving the purpose of killing tumor cells.

### Example 5 Antitumor Effect in HCT116 Xenograft Nude Mice

Female BALB/c nude mice (4 weeks old) were selected and acclimated for three days in a barrier facility. HCT116 cells (5×10⁶) were subcutaneously inoculated into the right flank of nude mice. When tumors grew to 80-100 mm³, tumor-bearing nude mice were randomly divided into four groups with six mice per group. Normal saline (blank group), 30 mg/kg of (J1-1), and 60 mg/kg of Compound (J1-1) prepared in Example 1 of the present invention (supplementary treatment group) were administered intravenously via the tail vein, respectively. Administration was performed twice a week for a total of five times. Body weight and tumor volume of nude mice were observed and recorded.

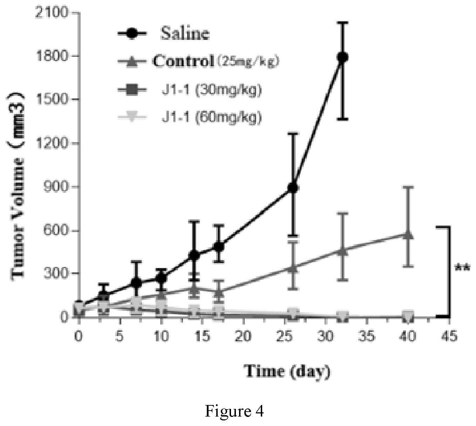

The experimental results are shown in Figure 4. The results demonstrated that Compound (J1-1) prepared in Example 1 of the present invention exhibited excellent therapeutic efficacy in the HCT116 cell xenograft tumor model. After five administrations, tumor volume was significantly reduced or even eliminated, indicating that Compound (J1-1) prepared in Example 1 of the present invention possesses the ability to inhibit tumor growth.

### Example 6 Synthesis of Compound (L1-1)

**The key intermediate A1 was synthesized according to Route 1.**

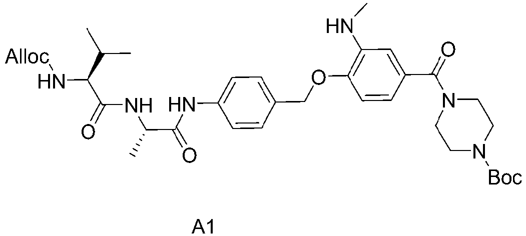

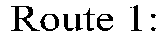

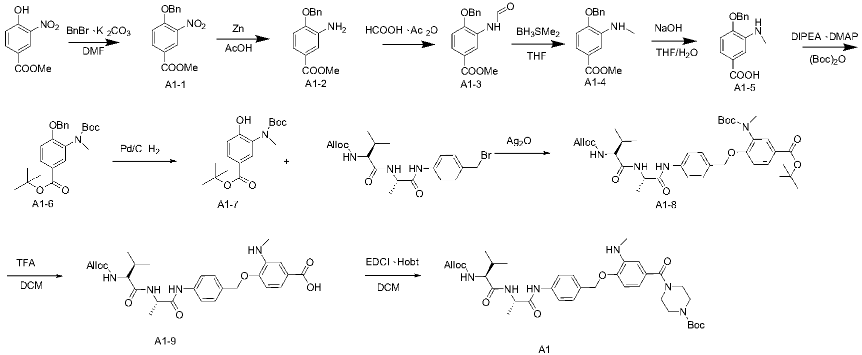

To a solution of methyl 3-nitro-4-hydroxybenzoate (20 g, 100 mmol) in N,N-dimethylformamide (100 mL) were added potassium carbonate (27.6 g, 200 mmol) and benzyl bromide (17.8 mL, 150 mmol) successively. After stirring at 60 °C for 6 h, the mixture was poured into a mixed solvent of 400 mL water and 150 mL ethyl acetate. The organic phase was washed with water (50 mL × 2) and saturated brine (50 mL) sequentially, dried over anhydrous sodium sulfate, and concentrated under reduced pressure. The residue was washed with petroleum ether to afford the desired product A1-1 (27.5 g, yield 96%). MS: 288 (M+H)+. 1H NMR (400 MHz, CDCl3) δ 8.52 (d, J = 2.0 Hz, 1H), 8.18 (dd, J = 8.8, 2.0 Hz, 1H), 7.58-7.30 (m, 5H), 7.18 (d, J = 8.8 Hz, 1H), 5.31 (s, 2H), 3.94 (s, 3H).

To a solution of A1-1 (27.5 g, 96 mmol) in a mixed solvent of acetic acid and tetrahydrofuran (80/80 mL) was added activated zinc powder (31.2 g, 480 mmol). After stirring at 70 °C for 2 h, the mixture was filtered, and the filtrate was slowly poured into saturated aqueous sodium bicarbonate solution to precipitate a solid, which was filtered and the filter cake was dried to give the desired product A1-2 (21.9 g, yield 89%). MS: 258 (M+H)+. 1H NMR (400 MHz, CDCl3) δ 7.49-7.33 (m, 7H), 6.87 (d, J = 8.3 Hz, 1H), 5.14 (s, 2H), 3.86 (s, 3H).

Formic acid (40 mL) and acetic anhydride (100 mL) were stirred at 70 °C for 2 h and then cooled to room temperature. A solution of A1-2 (21.9 g, 85 mmol) in tetrahydrofuran (150 mL) was slowly added to the above mixture, and the resulting mixture was stirred at room temperature for 2 h. Most of the solvent was removed by distillation under reduced pressure, and the remaining solution was slowly poured into saturated aqueous sodium bicarbonate solution to precipitate a white solid, which was filtered and the filter cake was dried to afford the desired product A1-3 (21 g, yield 87%). MS: 286 (M+H)+. 1H NMR (400 MHz, CDCl3) δ 9.89 (S, J = 7.8 Hz, 2H), δ7.54 (d, J = 7.8 Hz, 2H), 7.42 (d, J = 8.3 Hz, 1H), δ 7.44-7.24 (m, 5H), 7.08 (d, J = 8.4 Hz, 1H), 5.08 (s, 2H), 3.88 (s, 3H).

Under nitrogen protection at 0 °C, a 2 M solution of borane dimethyl sulfide in tetrahydrofuran (74 mL, 148 mmol) was slowly added dropwise to a solution of A1-3 (21 g, 74 mmol) in anhydrous tetrahydrofuran (150 mL). After stirring at 0 °C for 4 h, the reaction was quenched with cold methanol. Most of the solvent was removed by distillation under reduced pressure, and the remaining solution was slowly poured into saturated aqueous sodium bicarbonate solution to precipitate a white solid, which was filtered and the filter cake was dried to give the desired product A1-4 (16.4 g, yield 82%). MS: 272 (M+H)+. 1H NMR (400 MHz, CDCl3) δ 7.49-7.33 (m, 7H), 6.87 (d, J = 8.3 Hz, 1H), 5.14 (s, 2H), 3.86 (s, 3H), 2.91 (s, 3H).

To a solution of A1-4 (16.4 g, 60.7 mmol) in tetrahydrofuran/methanol/water (30/30/10 mL) was added sodium hydroxide (4.8 g, 120 mmol). The mixture was stirred at room temperature for 4 h, then adjusted to pH 3-4 with 1 M hydrochloric acid to precipitate a white solid, which was filtered and the filter cake was dried to afford the desired product A1-5 (13.8 g, yield 89%). MS: 258 (M+H)+. MS: 372 (M+H)+. 1H NMR (400 MHz, CDCl3) δ 7.51-7.33 (m, 7H), 6.88 (d, J = 8.3 Hz, 1H), 5.13 (s, 2H), 2.90 (s, 3H).

To a solution of A1-5 (13.8 g, 53.4 mmol) in tert-butanol (50 mL) were added di-tert-butyl dicarbonate (58 g, 270 mmol), DMAP (1.2 g, 10 mmol) and DIPEA (4.7 mL, 270 mmol). After stirring at 60 °C for 48 h, the mixture was poured into saturated aqueous ammonium chloride solution, extracted with ethyl acetate, dried over anhydrous magnesium sulfate, filtered, and the filtrate was collected and concentrated. The residue was purified by column chromatography to give the desired product A1-6 (16.3 g, yield 74%). MS: 414 (M+H)+. MS: 372 (M+H)+. 1H NMR (400 MHz, CDCl3) δ 7.51-7.33 (m, 7H), 6.88 (d, J = 8.3 Hz, 1H), 5.13 (s, 2H), 2.89 (s, 3H), 1.45 (s, 9H), 1.20 (s, 9H).

To a solution of A1-6 (16.3 g, 39 mmol) in methanol (100 mL) was added palladium on carbon (1.6 g). The mixture was stirred under hydrogen atmosphere at room temperature for 4 h, filtered, and the filtrate was concentrated to afford the desired product A1-7 (12.6 g, yield 89%). MS: 324 (M+H)+.

A1-8 was synthesized according to General Procedure 1.

General Procedure 1: To a dry two-necked flask were added A1-7 (1.0 equiv), the benzyl bromide intermediate of different trigger fragments (1.0 equiv) and silver oxide (1.5 equiv). Anhydrous tetrahydrofuran or anhydrous acetonitrile was added under nitrogen atmosphere and in the dark, and the mixture was stirred at room temperature for 12-24 h. After filtration, the filtrate was concentrated, and the crude product was purified by column chromatography.

A1-8: yield 78%, MS: 683 (M+H)+. 1H NMR (400 MHz, CDCl3) δ 8.99 (s, 1H), 7.84-7.65 (m, 2H), 7.62-7.41 (m, 3H), 7.19 (dd, J = 25.8, 11.3 Hz, 2H), 6.85 (d, J = 8.1 Hz, 1H), 5.95-5.71 (m, 2H), 5.25-5.15 (m, 2H), 5.10 (d, J = 10.4 Hz, 1H), 5.01 (s, 2H), 4.67 (p, J = 6.9 Hz, 1H), 4.55-4.43 (m, 2H), 4.13 (t, J = 7.3 Hz, 1H), 3.05 (s, 3H), 2.09-1.91 (m, 1H), 1.47 (d, J = 22.7 Hz, 9H), 1.36 (d, J = 6.9 Hz, 4H), 1.20 (d, J = 13.9 Hz, 9H), 0.86 (t, J = 5.7 Hz, 6H).

To a solution of A1-8 (1.3 g, 1.9 mmol) in dichloromethane (10 mL) was added trifluoroacetic acid (2 mL). The mixture was stirred at room temperature for 4 h, then dichloromethane and trifluoroacetic acid were removed by distillation under reduced pressure to give the desired crude product A1-9 (0.92 g, yield 92%). MS: 527 (M+H)+.

To a solution of A1-9 (0.92 g, 1.75 mmol) in dichloromethane (10 mL) were added EDCl·HCl (668 mg, 3.5 mmol), HOBt (270 mg, 2 mmol), N-Boc-piperazine (651 mg, 3.5 mmol) and triethylamine (478 µL, 3.5 mmol). After stirring at room temperature for 6 h, the mixture was poured into saturated aqueous ammonium chloride solution and extracted with dichloromethane. The organic phase was collected, dried, filtered, concentrated, and purified by column chromatography to afford the desired product A1 (1.08 g, yield 89%). MS: 695 (M+H)+. 1H NMR (400 MHz, CDCl3) δ 8.69 (s, 1H), 7.56 (d, J = 8.1 Hz, 2H), 7.33 (d, J = 8.3 Hz, 2H), 6.77 (dd, J = 13.0, 7.8 Hz, 2H), 6.69-6.56 (m, 2H), 5.99-5.82 (m, 1H), 5.34 (dd, J = 18.5, 7.7 Hz, 2H), 5.22 (d, J = 10.2 Hz, 1H), 5.01 (s, 2H), 4.65 (p, J = 7.0 Hz, 1H), 4.56 (t, J = 9.7 Hz, 2H), 4.35 (s, 1H), 4.04 (t, J = 6.6 Hz, 1H), 3.76-3.31 (m, 9H), 2.83 (s, 3H), 2.16 (dq, J = 13.3, 6.7 Hz, 1H), 1.71 (s, 6H), 1.46 (s, 9H), 0.94 (dt, J = 25.8, 12.9 Hz, 6H).

**Coupling of Key Intermediate with Drug DXD was carried out according to Route 2.**

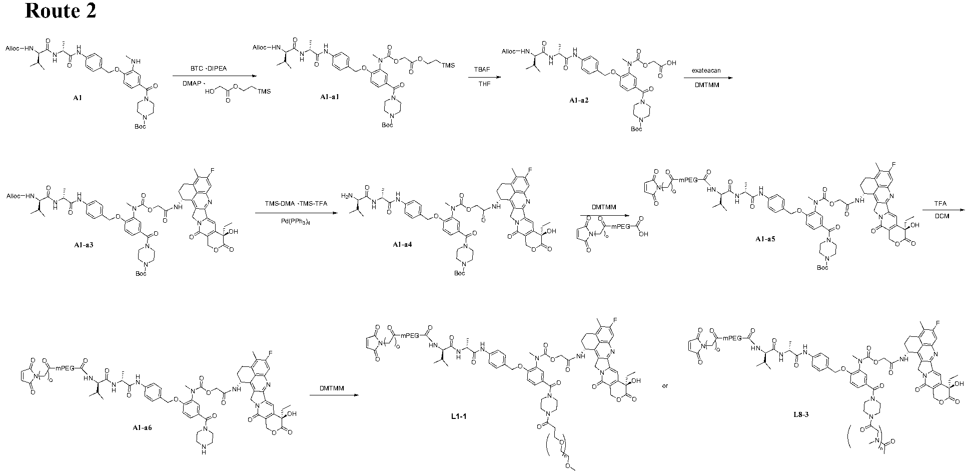

### Synthesis of Intermediate A1-a1

To a dry two-necked flask was added A1 (300 mg, 0.432 mmol), which was dissolved in 10 mL of anhydrous dichloromethane. Anhydrous DIPEA (300 µL, 1.73 mmol) was added under nitrogen atmosphere, followed by a solution of triphosgene (64 mg, 0.216 mmol) in dichloromethane (2 mL) at 0 °C. The mixture was stirred at room temperature for 1 h, then 2-(trimethylsilyl)ethyl 2-hydroxyacetate (176 mg, 1.29 mmol) and DMAP (157 mg, 1.29 mmol) were added. The mixture was stirred at 50 °C for 16 h, poured into saturated aqueous ammonium chloride solution, and extracted with dichloromethane. The organic phase was collected, dried, filtered, concentrated, and purified by column chromatography to afford the desired product A1-a1 (279 mg, yield 72%). MS: 897 (M+H)+. 1H NMR (400 MHz, CDCl3) δ 8.92 (s, 1H), 7.52 (t, J = 12.0 Hz, 2H), 7.42-7.19 (m, 5H), 6.96 (d, J = 8.4 Hz, 1H), 5.87 (ddt, J = 16.1, 10.6, 5.4 Hz, 1H), 5.68 (d, J = 7.9 Hz, 1H), 5.34-5.22 (m, 2H), 5.17 (d, J = 10.4 Hz, 1H), 5.06 (s, 2H), 4.73-4.48 (m, 4H), 4.31-4.06 (m, 3H), 3.70-3.35 (m, 7H), 3.17 (s, 2H), 2.28-2.04 (m, 2H), 1.44 (s, 9H), 1.38 (d, J = 6.8 Hz, 3H), 0.95-0.83 (m, 6H), 0.04--0.02 (m, 9H).

### Synthesis of Intermediate Al-a2

To a solution of A1-a1 (279 mg, 0.311 mmol) in tetrahydrofuran (15 mL) was added tetrabutylammonium fluoride (162 mg, 0.622 mmol). After stirring at room temperature for 8 h, the mixture was poured into saturated aqueous ammonium chloride solution and extracted with ethyl acetate. The organic phase was dried over anhydrous magnesium sulfate, filtered, and the filtrate was collected and concentrated to afford the desired product A1-a2 (220 mg, yield 89%). The crude product was used directly in the next step without purification. MS: 797 (M+H)+.

### Synthesis of Intermediate A1-a3

A1-a2 (277 mg, 0.277 mmol), exatecan mesylate (153 mg, 0.277 mmol), and DIPEA (97 µL, 0.554 mmol) were dissolved in 15 mL of DMF. A solution of DMTMM (150 mg, 0.554 mmol) in DMF (2 mL) was added dropwise under ice bath. After stirring for 4 h under ice bath, the mixture was poured into saturated aqueous ammonium chloride solution and extracted with ethyl acetate. The organic phase was dried over anhydrous magnesium sulfate, filtered, and the filtrate was collected and concentrated, and purified by column chromatography to afford the desired product A1-a3 (238 mg, yield 71%). MS: 1213 (M+H)+. 1H NMR (400 MHz, CDCl3 ) δ 8.24 - 7.94 (m, 2H), 7.64 - 7.47 (m, 3H), 7.43-7.41 (m, 3H), 7.31 (s, 1H), 7.19 (d, J = 12.0 Hz, 1H), 7.00 (d, J = 12.4 Hz, 2H), 5.97-5.93 (m, 1H), 5.53-5.49 (m, 4H), 5.40 (s, 1H), 5.35-5.31 (m, 1H), 5.30 - 5.21 (m, 2H), 5.15 (q, J = 9.7 Hz, 1H), 5.03 (s, 2H), 4.95 (s, 2H), 4.49 (dt, J = 9.7, 1.5 Hz, 2H), 4.23 (s, 1H), 3.59 -3.54(m, 4H), 3.43 - 3.28 (m, 5H), 3.24 (s, 3H), 3.01 - 2.52 (m, 4H), 2.41 - 1.95 (m, 6H), 1.44 (d, J = 6.8 Hz, 2H), 1.42-1.39 (m, 12H), 1.02 (d, J = 10 .2Hz, 6H), 0.96 (t, J = 10.8 Hz, 3H).

### Synthesis of Intermediate A1-a4

To a solution of A1-a3 (238 mg, 0.197 mmol) in DMF (10 mL) were added tetrakis(triphenylphosphine)palladium (23 mg) and piperidine (46 µL, 0.394 mmol). The mixture was stirred under nitrogen at room temperature for 12 h, filtered, and the filtrate was collected and concentrated and purified by column chromatography to afford the desired product A1-a4 (160 mg, yield 72%). MS: 1130 (M+H)+. 1H NMR (400 MHz, CDCl3 ) δ 8.23 - 7.96(m, 2H), 7.62 - 7.47 (m, 3H), 7.43-7.39 (m, 3H), 7.32 (s, 1H), 7.20 (d, J = 11.6 Hz, 1H), 7.01 (d, J = 9.9 Hz, 2H), 5.52-5.49 (m, 2H), 5.40 (s, 1H), 5.29 - 5.21 (m, 2H), 5.15 -5.09(m, 1H), 5.05(s, 2H), 4.95 (s, 2H), 4.48 - 4.42 (m, 2H), 4.23-4.19 (m, 2H), 3.56 -3.51(m, 3H), 3.41 - 3.28 (m, 5H), 3.24 (s, 3H), 3.010- 2.53 (m, 3H), 2.41 - 1.95 (m, 7H), 1.42 (d, J = 6.7 Hz, 2H), 1.40-1.397(m, 12H), 1.04 (d, J = 10 .1Hz, 6H), 0.96 (t, J = 10.6 Hz, 3H).

### Synthesis of Intermediate A1-a5

To a solution of A1-a4 (160 mg, 0.142 mmol) in DMF (5 mL) were added 3-(2-(2-(6-(2,5-dioxo-2,5-dihydro-1H-pyrrol-1-yl)hexanamido)ethoxy)ethoxy)propanoic acid (50 mg, 0.142 mmol) and DIPEA (50 µL, 0.284 mmol). A solution of DMTMM (78 mg, 0.284 mmol) in DMF (2 mL) was added dropwise at 0 °C. The mixture was stirred for 12 h, then DMF was removed under reduced pressure, and the crude product was purified by C18 flash chromatography to afford the desired product A1-a5 (142 mg, yield 68%). MS: 1468 (M+H)+. 1H NMR (400 MHz, CDCl3 ) δ 8.28 (s, 1H), 8.17 (d, J = 2.9 Hz, 1H), 7.77 (dd, J = 15.0, 3.1 Hz, 1H), 7.62 - 7.51 (m, 3H), 7.44 (dt, J = 4.1, 2.6 Hz, 3H), 7.20 (d, J = 15.0 Hz, 1H), 7.10 (d, J = 4.8 Hz, 3H), 7.03 (d, J = 14.1 Hz, 2H), 5.58 (d, J = 8.1 Hz, 3H), 5.45 - 5.20 (m, 4H), 5.15 - 4.90 (m, 5H), 4.00 (t, J = 10.0 Hz, 2H), 3.75 - 3.53 (m, 8H), 3.49 (d, J = 28.9 Hz, 7H), 3.37 - 3.21 (m, 9H), 2.99 - 2.61 (m, 4H), 2.46 (t, J = 8.4 Hz, 2H), 2.38 - 2.07 (m, 7H), 1.97 (q, J = 13.6 Hz, 1H), 1.68 - 1.52 (m, 2H), 1.48 - 1.39 (m, 12H), 1.38 - 1.11 (m, 4H), 1.02 - 0.83 (m, 9H).

### Synthesis of Intermediate A1-a6

To a solution of A1-a5 (142 mg, 0.096 mmol) in dichloromethane (10 mL) was added trifluoroacetic acid (2 mL). The mixture was stirred at room temperature for 4 h, then dichloromethane and trifluoroacetic acid were removed under reduced pressure to afford the desired crude product A1-a6 (121 mg, yield 92%). The crude product was used directly in the next step without purification. MS: 1368 (M+H)+.

### Synthesis of L1-1

To a solution of A1-a6 (121 mg, 0.088 mmol) in DMF (5 mL) were added 2,5,8,11,14,17,20,23-octaoxadocosan-25-oic acid (35 mg, 0.088 mmol) and DIPEA (30 µL, 0.176 mmol). A solution of DMTMM (49 mg, 0.176 mmol) in DMF (2 mL) was added dropwise at 0 °C. The mixture was stirred for 12 h, then DMF was removed under reduced pressure, and the crude product was purified by HPLC to afford the desired product L1-1 (62 mg, yield 68%). MS: 1748 (M+H)+. 1H NMR (400 MHz, DMSO-d6 ) δ 8.29 (s, 1H), 8.16 (d, J = 3.0 Hz, 1H), 7.76 (dd, J = 15.0, 3.1 Hz, 1H), 7.61 - 7.51 (m, 3H), 7.43 (dt, J = 4.0, 2.6 Hz, 3H), 7.20 -7.16 (m, 1H), 7.11 - 7.06 (m, 3H), 7.03-7.68 (m, 2H), 5.58 (d, J = 8.0 Hz, 3H), 5.43 - 5.20 (m, 5H), 5.14 - 4.90 (m, 4H), 4.00 - 3.89 (m, 2H), 3.75 - 3.53 (m, 12H), 3.49-3.40 (m, 56H), 3.37 - 3.21 (m, 16H), 2.98 - 2.63 (m, 6H), 2.46 - 2.39 (m, 6H), 2.38 - 2.07 (m, 7H), 1.97(q, J = 13.6 Hz, 1H), 1.69 - 1.54 (m, H), 1.47 - 1.36 (m, 16H), 1.34 - 1.20 (m, 4H), 1.04 - 0.82 (m, 9H).

### Synthesis of L8-3

Intermediate A1-a4 was subjected to condensation with maleimide-PEG acid (refer to the synthesis of A1-a5 in the present invention), deprotection of Boc (refer to the synthesis of A1-a6 in the present invention), and condensation with polylysine chain (refer to the synthesis of L1-1 in the present invention) to afford the desired product L8-3. 1H NMR (400 MHz, DMSO-d6 ) δ 8.28 (s, 1H), 8.17 (d, J = 1.4 Hz, 1H), 7.93 (s, 1H), 7.77 (dd, J = 6, 1.1 Hz, 1H), 7.56 (d, J = 6 Hz, 2H), 7.51 - 7.37 (m, 4H), 7.20 (d, J = 6 Hz, 1H), 7.13 (s, 2H), 7.02 (s, 1H), 6.40 (s, 1H), 5.56 (s, 2H), 5.53 (d, J = 2.0 Hz, 1H), 5.42 (t, J = 6.2 Hz, 1H), 5.36 (s, 1H), 5.14 (s, 1H), 5.04 (s, 2H), 4.99 - 4.92 (m, 3H), 4.88 (s, 1H), 4.75 (t, J = 6.4 Hz, 2H), 3.91 (s, 16H), 3.74 - 3.56 (m, 12H), 3.52 (s, 12H), 3.39 (s, 1H), 3.31 - 3.19 (m, 5H), 2.96 (s, 24H), 2.85 (dt, J = 7.8, 3.8 Hz, 1H), 2.80 - 2.69 (m, 3H), 2.63 - 2.58 (m, 4H), 2.50 - 2.43 (m, 1H), 2.37 - 2.25 (m, 4H), 2.11 (s, 3H), 1.91 (q, J = 5.4 Hz, 1H), 1.44 (d, J = 4.8 Hz, 3H), 1.00 - 0.85 (m, 9H).

**Coupling of the key intermediate and all phenolic hydroxyl drugs was carried out according to in Route 3.**

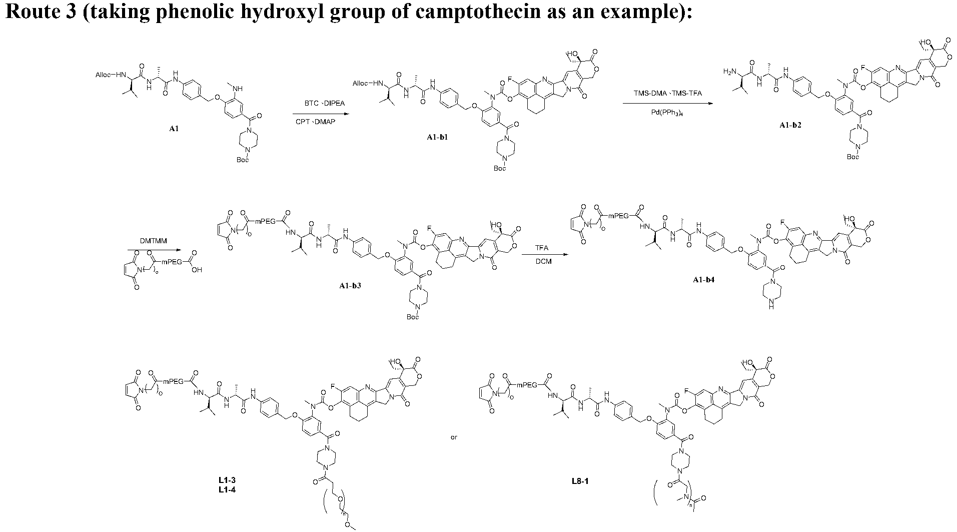

### Synthesis of A1-b1

To a dry two-necked flask was added A1 (300 mg, 0.432 mmol), which was dissolved in 10 mL of anhydrous dichloromethane. Anhydrous DIPEA (300 µL, 1.73 mmol) was added under nitrogen atmosphere, followed by a solution of triphosgene (64 mg, 0.216 mmol) in dichloromethane (2 mL) at 0 °C. The mixture was stirred at room temperature for 1 h, then camptothecin phenolic hydroxyl compound (218 mg, 0.52 mmol) and DMAP (157 mg, 1.29 mmol) were added. The mixture was stirred at 50 °C for 16 h, poured into saturated aqueous ammonium chloride solution, and extracted with dichloromethane. The organic phase was collected, dried, filtered, concentrated, and purified by column chromatography to afford the desired product A1-b1 (279 mg, yield 72%). MS: 1143 (M+H)+. 1H NMR (400 MHz, CDCl3) δ 8.22 (s, 1H), 8.17 (d, J = 1.1 Hz, 1H), 7.77-7.67 (m, 1H), 7.56 (d, J = 6 Hz, 2H), 7.44 (d, J = 6 Hz, 2H), 7.38-7.32 (m, 2H), 7.20-7.15 (m, 1H), 7.05 (s, 1H), 5.98-5.92 (m, 1H), 5.78-5.60 (m, 2H), 5.52 (d, J = 8.8 Hz, 1H), 5.27-5.19 (m, 3H), 5.04 (s, 2H), 4.96 (t, J = 3.4 Hz, 2H), 4.50 (d, J = 4.9 Hz, 2H), 4.26 (s, 1H), 3.75 (t, J = 4.6 Hz, 2H), 3.59 (t, J = 4.1 Hz, 4H), 3.45 (s, 1H), 3.34-3.29 (m, 4H), 3.25 (s, 3H), 2.97 (t, J = 4.7 Hz, 2H), 2.78-2.62 (m, 1H), 2.28-2.19 (m, 3H), 1.50-1.34 (m, 12H), 0.96 (d, J = 5.1 Hz, 6H), 0.89 (t, J = 5.4 Hz, 3H).

### Synthesis of Al-b2

To a solution of A1-b1 (0.2 mmol) in DCM (10 mL) were added TMS-DMA (1.2 mmol) and TMS-TFA (1.2 mmol). The mixture was stirred under nitrogen at room temperature for 1 h, then tetrakis(triphenylphosphine)palladium (0.04 mmol) was added, and the mixture was stirred under nitrogen at room temperature for 12 h. The mixture was poured into saturated aqueous ammonium chloride solution and extracted with dichloromethane. The organic phase was collected, dried, filtered, concentrated, and purified by column chromatography to afford the desired product A1-b2 (yield 69%). MS: 1058 (M+H)+. 1H NMR (400 MHz, CDCl3) δ 8.17 (d, J = 2.3 Hz, 1H), 7.77 (dd, J = 12.0, 2.5 Hz, 1H), 7.60-7.51 (m, 2H), 7.47-7.41 (m, 2H), 7.37 (d, J = 12.7 Hz, 1H), 7.20 (d, J = 15.0 Hz, 1H), 7.05 (s, 1H), 6.14 (s, 1H), 5.76 (d, J = 16.5 Hz, 2H), 5.12-5.00 (m, 3H), 4.98 (s, 2H), 3.82-3.69 (m, 3H), 3.60 (dd, J = 12.2, 4.1 Hz, 4H), 3.42 (s, 1H), 3.33 (dd, J = 12.2, 4.1 Hz, 4H), 3.25 (s, 3H), 2.97 (t, J = 9.4 Hz, 2H), 2.32-1.96 (m, 6H), 1.74 (s, 1H), 1.49-1.36 (m, 12H), 0.96 (d, J = 10.2 Hz, 6H), 0.89 (t, J = 10.8 Hz, 3H).

### Synthesis of L1-3

Intermediate A1-b2 was subjected to condensation with maleimide-PEG acid (refer to the synthesis of A1-a5 in the present invention), deprotection of Boc (refer to the synthesis of A1-a6 in the present invention), and condensation with dodecaethylene glycol propionic acid (refer to the synthesis of L1-1 in the present invention) to afford the desired product L1-3. 1H NMR (400 MHz, DMSO-d6) δ 9.83 (s, 1H), 8.16 (d, J = 2.3 Hz, 1H), 7.79-7.70 (m, 2H), 7.61-7.50 (m, 2H), 7.48-7.40 (m, 2H), 7.19 (d, J = 12.0 Hz, 1H), 7.14 (s, 2H), 6.92 (s, 1H), 5.68 (s, 1H), 5.61 (s, 1H), 5.54 (s, 1H), 5.21 (s, 1H), 5.16 (q, J = 9.9 Hz, 1H), 5.03 (s, 2H), 4.95 (s, 2H), 4.74 (t, J = 12.8 Hz, 2H), 4.61 (d, J = 17.3 Hz, 1H), 3.74 (t, J = 9.2 Hz, 2H), 3.69-3.58 (m, 13H), 3.57-3.52 (m, 4H), 3.51 (s, 52H), 3.40 (s, 3H), 3.27 (t, J = 6.2 Hz, 2H), 3.25 (s, 3H), 3.11 (s, 1H), 2.97 (t, J = 9.2 Hz, 2H), 2.79-2.60 (m, 5H), 2.53 (t, J = 6.8 Hz, 2H), 2.31-2.17 (m, 3H), 1.93 (q, J = 10.8 Hz, 1H), 1.44 (d, J = 9.9 Hz, 3H), 0.96 (d, J = 10.1 Hz, 6H), 0.89 (t, J = 10.8 Hz, 3H).

### Synthesis of L1-4

Intermediate A1-b2 was subjected to condensation with maleimidocaproic acid (refer to the synthesis of A1-a5 in the present invention), deprotection of Boc (refer to the synthesis of A1-a6 in the present invention), and condensation with dodecaethylene glycol propionic acid (refer to the synthesis of L1-1 in the present invention) to afford the desired product L1-4. 1H NMR (400 MHz, DMSO-d6) δ 8.64 (s, 1H), 8.17 (d, J = 2.3 Hz, 1H), 7.96 (s, 1H), 7.77 (dd, J = 12.0, 2.3 Hz, 1H), 7.61-7.51 (m, 2H), 7.48-7.40 (m, 2H), 7.30 (d, J = 12.7 Hz, 1H), 7.20 (d, J = 11.8 Hz, 1H), 7.12 (s, 2H), 7.00 (s, 1H), 5.74 (d, J = 12.6 Hz, 2H), 5.39 (s, 1H), 5.20 (d, J = 14.6 Hz, 1H), 5.04 (s, 2H), 4.97 (s, 2H), 4.95-4.86 (m, 1H), 4.00 (t, J = 11.2 Hz, 2H), 3.75 (t, J = 9.3 Hz, 2H), 3.67-3.49 (m, 54H), 3.40 (s, 3H), 3.33 (s, 1H), 3.25 (s, 3H), 2.97 (t, J = 9.4 Hz, 2H), 2.81-2.62 (m, 1H), 2.54 (t, J = 12.4 Hz, 2H), 2.31-2.15 (m, 3H), 2.13-1.93 (m, 3H), 1.72-1.55 (m, 2H), 1.44 (d, J = 9.7 Hz, 3H), 1.40-1.11 (m, 4H), 0.96 (d, J = 10.2 Hz, 6H), 0.89 (t, J = 10.8 Hz, 3H).

### Synthesis of L8-1

Intermediate A1-b2 was subjected to condensation with maleimide-PEG acid (refer to the synthesis of A1-a5 in the present invention), deprotection of Boc (refer to the synthesis of A1-a6 in the present invention), and condensation with deca-polylysine (refer to the synthesis of L1-1 in the present invention) to afford the desired product L8-1. 1H NMR (400 MHz, DMSO-d6) δ 8.17 (d, J = 1.4 Hz, 1H), 7.77 (dd, J = 7.5, 1.4 Hz, 1H), 7.56 (d, J = 7.5 Hz, 2H), 7.44 (d, J = 7.5 Hz, 2H), 7.33 (d, J = 8.1 Hz, 1H), 7.20 (d, J = 7.5 Hz, 1H), 7.14 (s, 1H), 7.00 (s, 1H), 6.78 (s, 1H), 6.74 (s, 2H), 5.63-5.55 (m, 1H), 5.53 (s, 1H), 5.39 (s, 2H), 5.04 (s, 2H), 4.96 (t, J = 6.8 Hz, 2H), 4.75 (t, J = 7.9 Hz, 2H), 4.59 (d, J = 2.5 Hz, 1H), 3.91 (s, 20H), 3.75 (t, J = 5.8 Hz, 2H), 3.71-3.55 (m, 12H), 3.51 (d, J = 8.4 Hz, 13H), 3.28 (q, J = 4.5 Hz, 2H), 3.25 (s, 3H), 3.00-2.90 (m, 32H), 2.81-2.69 (m, 1H), 2.66 (t, J = 7.9 Hz, 2H), 2.35-2.15 (m, 5H), 2.11 (s, 3H), 1.99 (q, J = 6.9 Hz, 1H), 1.44 (d, J = 6.0 Hz, 3H), 0.96 (d, J = 6.4 Hz, 6H), 0.89 (t, J = 6.8 Hz, 3H).

### Synthesis of L1-2

Using A1 and various camptothecin phenolic hydroxyl compounds as starting materials, the desired product L1-2 was obtained according to Route 3 by referring to the synthesis of L1-3 in the present invention. MS: 1999 (M+H)+. 1H NMR (400 MHz, DMSO-d6) δ 8.20 (s, 1H), 8.16 (d, J = 2.3 Hz, 1H), 7.76 (dd, J = 12.0, 2.5 Hz, 1H), 7.59-7.52 (m, 2H), 7.46-7.39 (m, 3H), 7.19 (d, J = 12.0 Hz, 1H), 7.13 (s, 2H), 7.01 (d, J = 4.5 Hz, 2H), 6.38 (s, 1H), 6.27 (s, 1H), 5.56 (d, J = 5.4 Hz, 3H), 5.41 (t, J = 3.8 Hz, 1H), 5.39 (s, 1H), 5.37-5.23 (m, 3H), 5.04 (s, 2H), 4.93 (s, 2H), 4.75 (t, J = 12.8 Hz, 2H), 3.71-3.56 (m, 15H), 3.56-3.53 (m, 4H), 3.52 (s, 52H), 3.46 (s, 1H), 3.40 (s, 3H), 3.30 (t, J = 3.5 Hz, 1H), 3.27 (t, J = 3.3 Hz, 1H), 3.25 (s, 3H), 2.95-2.67 (m, 5H), 2.66-2.52 (m, 4H), 2.51-2.37 (m, 2H), 2.36-2.29 (m, 4H), 2.32-2.14 (m, 2H), 1.96 (q, J = 10.8 Hz, 1H), 1.44 (d, J = 9.8 Hz, 3H), 0.96 (d, J = 10.2 Hz, 6H), 0.89 (t, J = 10.8 Hz, 3H).

### Synthesis of L8-2

Using A1 and various camptothecin phenolic hydroxyl compounds as starting materials, the desired product L8-2 was obtained according to Route 3 by referring to the synthesis of L8-1 in the present invention. 1H NMR (400 MHz, DMSO-d6) δ 8.31 (s, 1H), 8.17 (d, J = 2.3 Hz, 1H), 7.77 (dd, J =12.0, 2.3 Hz, 1H), 7.64-7.38 (m, 6H), 7.24-7.18 (m, 2H), 7.13 (d, J = 2.5 Hz, 3H), 7.01 (s, 1H), 5.74 (d, J = 6.2 Hz, 2H), 5.31 (d, J = 3.0 Hz, 1H), 5.25 (s, 1H), 5.08-4.98 (m, 3H), 4.97 (s, 2H), 4.75 (t, J = 12.8 Hz, 2H), 3.91 (s, 20H), 3.81-3.55 (m, 14H), 3.52 (s, 12H), 3.47 (s, 1H), 3.34-3.18 (m, 5H), 3.01-2.85 (m, 32H), 2.79-2.59 (m, 3H), 2.53 (t, J = 12.5 Hz, 2H), 2.31-2.16 (m, 3H), 2.11 (s, 3H), 1.98 (q, J = 10.8 Hz, 1H), 1.44 (d, J = 9.8 Hz, 3H), 0.96 (d, J = 10.2 Hz, 6H), 0.89 (t, J = 10.8 Hz, 3H).

### Example 7 Synthesis of Compounds L3-1 and L4-1

### Intermediates C1 and D1 were synthesized according to Route 3.

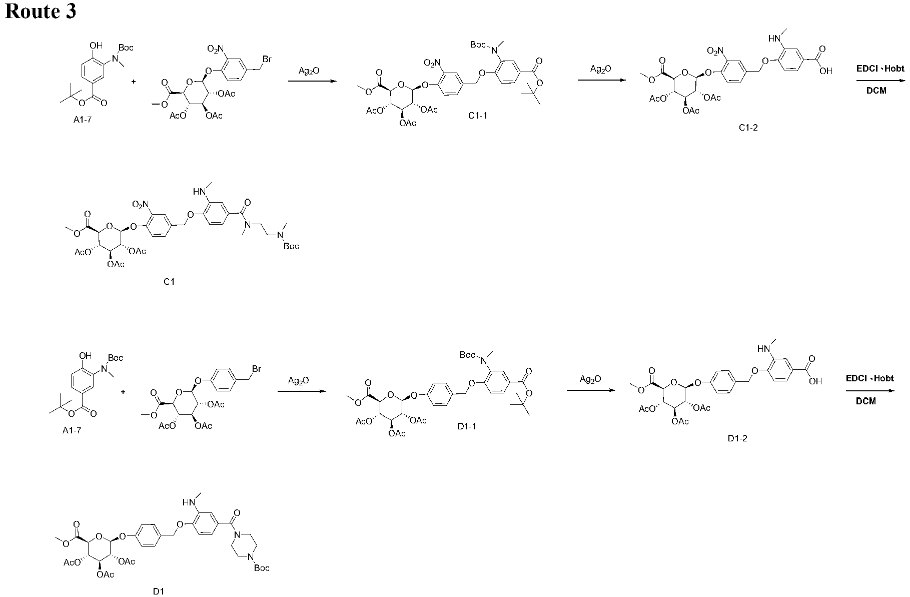

C1-1 and D1-1 were synthesized according to General Procedure 1.

C1-1: yield 81%, MS: 791 (M+H)+. 1H NMR (400 MHz, CDCl3) δ 8.21 (dd, J = 4.0, 2.4 Hz, 1H), 7.79 (dd, J = 12.0, 2.3 Hz, 1H), 7.38 (dd, J = 11.9, 2.4 Hz, 1H), 7.17-7.00 (m, 1H), 6.55 (d, J = 12.7 Hz, 1H), 5.53 (dd, J = 13.4, 12.3 Hz, 1H), 5.16 (s, 1H), 5.01-4.74 (m, 1H), 4.61 (dd, J = 15.4, 12.2 Hz, 1H), 4.30 (d, J = 15.4 Hz, 1H), 3.70 (s, 1H), 3.25 (s, 2H), 2.04 (s, 3H), 2.02 (s, 3H), 1.99 (s, 3H), 1.42 (s, 12H), 1.40 (s, 6H).

D1-1: yield 79%, MS: 746 (M+H)+. 1H NMR (400 MHz, CDCl3) δ 8.21 (d, J = 1.1 Hz, 1H), 7.79 (dd, J = 6, 1.1 Hz, 1H), 7.13 (d, J = 6 Hz, 1H), 6.99 (d, J = 6 Hz, 1H), 6.89 (d, J = 6 Hz, 1H), 5.91 (dd, J = 7.9, 6.6 Hz, 1H), 5.61-5.50 (m, 1H), 5.46 (d, J = 6.2 Hz, 1H), 5.28-4.98 (m, 1H), 3.85-3.55 (m, 1H), 3.25 (s, 1H), 2.06 (s, 3H), 2.03 (s, 3H), 2.01 (s, 3H), 1.43 (s, 12H), 1.41 (s, 6H).C1-2 and D1-2 were synthesized by referring to the synthesis of A1-9.C1-2: yield 91%, MS: 691 (M+H)+.D1-2: yield 93%, MS: 646 (M+H)+.C1-2 and D1-2 were used directly in the next step without purification.

C1 and D1 were synthesized by referring to the synthesis of A1. C1: yield 84%, MS: 805 (M+H)+. 1H NMR (400 MHz, CDCl3) δ 8.22 (d, J = 2.4 Hz, 1H), 7.37 (td, J = 12.0, 2.3 Hz, 2H), 7.20 (dd, J = 11.4, 7.2 Hz, 2H), 7.00 (d, J = 12.0 Hz, 1H), 6.67 (d, J = 12.8 Hz, 1H), 5.69 (dd, J = 13.4, 12.8 Hz, 1H), 5.16 (s, 2H), 4.92-4.65 (m, 3H), 4.29 (d, J = 16 Hz, 1H), 3.70 (s, 3H), 3.67-3.27 (m, 4H), 3.23 (s, 6H), 2.68 (s, 3H), 2.04 (s, 3H), 2.02 (s, 3H), 1.99 (s, 3H), 1.40 (s, 9H).

D1: yield 82%, MS: 760 (M+H)+. 1H NMR (400 MHz, CDCl3) δ 7.35 (dd, J = 6, 1.1 Hz, 1H), 7.19 (d, J = 1.1 Hz, 1H), 7.00 (dd, J = 6, 3.7 Hz, 3H), 6.89 (d, J = 6 Hz, 2H), 6.60 (d, J = 6.3 Hz, 1H), 5.78 (t, J = 6.5 Hz, 1H), 5.16 (s, 2H), 4.78-4.67 (m, 2H), 4.58 (s, 1H), 4.50 (d, J = 7.7 Hz, 1H), 3.70 (s, 3H), 3.59 (t, J = 3.8 Hz, 4H), 3.34 (t, J = 3.8 Hz, 4H), 2.68 (s, 3H), 2.02 (s, 6H), 2.00 (s, 3H), 1.41 (s, 9H).

### Synthesis of L3-1 referred to Route 4.

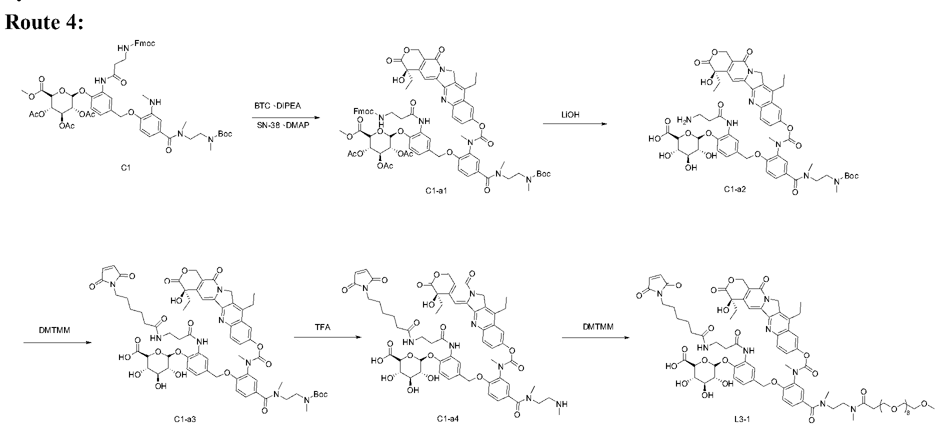

### Synthesis of Intermediate C1-a1

Intermediate C1-a1 was synthesized by referring to the synthesis of A1-b1.C1-a1: yield 74%, MS: 1486 (M+H)+. 1H NMR (400 MHz, CDCl3) δ 8.25 (s, 1H), 8.17 (d, J = 2.5 Hz, 1H), 7.91 (d, J = 2.6 Hz, 1H), 7.88 (d, J = 2.5 Hz, 1H), 7.84 (d, J = 2.4 Hz, 1H), 7.77 (dd, J = 12.0, 2.4 Hz, 1H), 7.65 (d, J = 11.9 Hz, 1H), 7.47-7.10 (m, 10H), 6.87 (s, 1H), 6.83 (dd, J = 12.0, 2.3 Hz, 1H), 6.18 (d, J = 5.3 Hz, 1H), 5.47 (dd, J = 3.2, 0.9 Hz, 1H), 5.31-5.23 (m, 2H), 5.19-5.11 (m, 3H), 4.99-4.92 (m, 4H), 4.72 (d, J = 14.0 Hz, 2H), 4.49 (d, J = 3.9 Hz, 1H), 3.70 (s, 3H), 3.64-3.43 (m, 8H), 3.37-3.27 (m, 1H), 3.24 (d, J = 8.1 Hz, 9H), 3.00 (t, J = 8.8 Hz, 1H), 2.71 (t, J = 12.8 Hz, 2H), 2.24 (q, J = 10.8 Hz, 1H), 2.02 (s, 9H), 1.92 (q, J = 10.8 Hz, 1H), 1.42 (s, 9H), 1.18 (t, J = 10.5 Hz, 3H), 0.89 (t, J = 10.8 Hz, 3H).

### Synthesis of Intermediate C1-a2

To a solution of C1-a1 (1 equiv) in THF/MeOH/H₂O (3:3:1) was added 1 M aqueous LiOH solution (5 equiv). After stirring at room temperature for 4 h, the mixture was purified by C18 flash chromatography to afford C1-a2 (yield 69%). MS: 1124 (M+H)+. 1H NMR (400 MHz, CDCl3) δ 8.27-8.12 (m, 2H), 7.87-7.72 (m, 2H), 7.65 (d, J = 11.8 Hz, 1H), 7.46-7.29 (m, 2H), 7.19 (dd, J = 12.0, 7.6 Hz, 2H), 7.00 (s, 1H), 6.83 (dd, J = 12.0, 2.3 Hz, 1H), 5.80 (s, 1H), 5.60 (s, 1H), 5.22-5.11 (m, 3H), 4.97 (s, 2H), 4.49 (dd, J = 5.4, 0.9 Hz, 1H), 4.18-3.98 (m, 2H), 3.66-3.42 (m, 6H), 3.39-3.30 (m, 2H), 3.24 (d, J = 8.1 Hz, 9H), 3.11 (td, J = 12.5, 0.9 Hz, 2H), 2.81-2.63 (m, 2H), 2.24 (q, J = 10.8 Hz, 1H), 1.98 (q, J = 10.8 Hz, 1H), 1.42 (s, 9H), 1.18 (t, J = 10.5 Hz, 3H), 0.89 (t, J = 10.8 Hz, 3H).

### Synthesis of L3-1

Intermediate C1-a2 was subjected to condensation with maleimidocaproic acid (refer to the synthesis of A1-a5), Boc deprotection (refer to the synthesis of A1-a6), and condensation with octaethylene glycol propionic acid (refer to the synthesis of L1-1) to afford L3-1. Overall yield: 12%, MS: 1611 (M+H)+. 1H NMR (400 MHz, DMSO-d6) δ 8.12 (d, J = 2.3 Hz, 1H), 7.79 (d, J = 2.4 Hz, 1H), 7.72 (dd, J = 11.9, 2.4 Hz, 1H), 7.60 (d, J = 11.8 Hz, 1H), 7.38-7.25 (m, 2H), 7.23 (s, 1H), 7.14 (dd, J = 11.9, 7.5 Hz, 2H), 7.06 (s, 2H), 6.95 (s, 1H), 6.79 (dd, J = 12.0, 2.3 Hz, 1H), 6.27 (d, J = 2.3 Hz, 1H), 5.80 (s, 1H), 5.33-5.26 (m, 2H), 5.13 (s, 2H), 4.93 (s, 2H), 4.40 (s, 2H), 4.34 (s, 1H), 4.29 (dd, J = 5.2, 0.6 Hz, 1H), 4.17 (dd, J = 4.7, 2.3 Hz, 1H), 4.10 (t, J = 4.9 Hz, 1H), 3.97 (t, J = 7.9 Hz, 2H), 3.85 (d, J = 0.5 Hz, 1H), 3.77 (d, J = 12.8 Hz, 1H), 3.71-3.50 (m, 4H), 3.50 (s, 32H), 3.49-3.41 (m, 4H), 3.37 (s, 1H), 3.23 (s, 3H), 3.21 (s, 3H), 2.92 (s, 3H), 2.69 (t, J = 12.7 Hz, 2H), 2.38 (s, 1H), 2.24-2.03 (m, 4H), 1.70-1.52 (m, 2H), 1.44 (s, 1H), 1.41-1.23 (m, 4H), 1.21-1.03 (m, 6H), 0.88 (t, J = 10.8 Hz, 3H).

### Synthesis of L4-1 referred to Route 5.

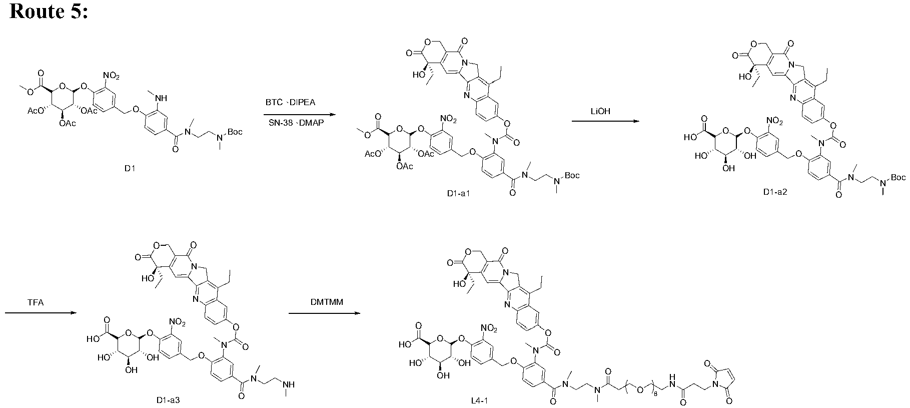

### Synthesis of D1-a1

D1-a1 was synthesized by referring to the synthesis of A1-b1. D1-a1: yield 72%, MS: 1486 (M+H)+. 1H NMR (400 MHz, CDCl3 ) δ 8.22 (d, J = 1.2 Hz, 1H), 8.17 (d, J = 1.1 Hz, 1H), 7.77 (dd, J = 6.0, 1.1 Hz, 1H), 7.65 (d, J = 6.0 Hz, 1H), 7.43 - 7.30 (m, 3H), 7.21 (dd, J = 7.9, 6.0 Hz, 2H), 7.03 (s, 1H), 6.05 (s, 1H), 5.67 (s, 1H), 5.42 (d, J = 6.2 Hz, 1H), 5.19 (dd, J = 6.9, 4.7 Hz, 1H), 5.16 (s, 2H), 5.03 (td, J = 8.0, 3.1 Hz, 1H), 4.99 (d, J = 1.1 Hz, 2H), 4.70 (d, J = 7.9 Hz, 1H), 3.97 (dd, J = 6.1, 2.5 Hz, 1H), 3.70 (s, 3H), 3.59 - 3.55 (m, 2H), 3.52 - 3.44 (m, 4H), 3.41 - 3.35 (m, 1H), 3.24 (d, J = 4.2 Hz, 3H), 3.23 (s, 6H), 2.21 (q, J = 5.4 Hz, 1H), 2.15 - 2.06 (m, 1H), 2.04 (s, 6H), 2.01 (s, 3H), 1.40 (s, 9H), 1.18 (t, J = 5.3 Hz, 3H), 0.89 (t, J = 5.4 Hz, 3H).

### Synthesis of L4-1

D1-a1 was subjected to hydrolysis (refer to the synthesis of C1-a2), deprotection of Boc (refer to the synthesis of A1-a6), and condensation with C3-maleimide-octaethylene glycol propionic acid (refer to the synthesis of L1-1) to afford the desired product L4-1. Overall yield: 11%, MS: 1557 (M+H)+. 1H NMR (400 MHz, CDCl3 ) δ 8.22 (d, J = 2.3 Hz, 1H), 8.17 (d, J = 2.4 Hz, 1H), 7.77 (dd, J = 12.0, 2.3 Hz, 1H), 7.65 (d, J = 11.8 Hz, 1H), 7.37 (ddd, J = 11.4, 6.4, 2.3 Hz, 3H), 7.26 (s, 2H), 7.21 (dd, J = 12.0, 7.5 Hz, 2H), 6.86 (s, 1H), 5.92 (d, J = 7.4 Hz, 1H), 5.81 (s, 1H), 5.77 (s, 1H), 5.65 (s, 1H), 5.16 (s, 2H), 4.96 (s, 2H), 4.78 (s, 1H), 4.65 (dd, J = 9.6, 1.2 Hz, 1H), 4.60 (s, 2H), 4.44 (dd, J = 7.36, 4.6 Hz, 1H), 4.21 (dd, J = 9.6, 4.6 Hz, 1H), 3.70 - 3.53 (m, 10H), 3.52 (s, 30H), 3.51 - 3.45 (m, 4H), 3.31 - 3.26 (m, 3H), 3.25 (s, 3H), 3.23 (s, 3H), 3.11 (s, 1H), 2.94 (s, 3H), 2.80 (s, 1H), 2.69 (t, J = 12.4 Hz, 2H), 2.40 (q, J = 10.8 Hz, 1H), 2.20 - 2.08 (m, 2H), 1.18 (t, J = 10.5 Hz, 3H), 0.89 (t, J = 10.8Hz, 3H).

### Example: Synthesis of L5-L7 referred to Route 6.

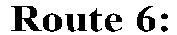

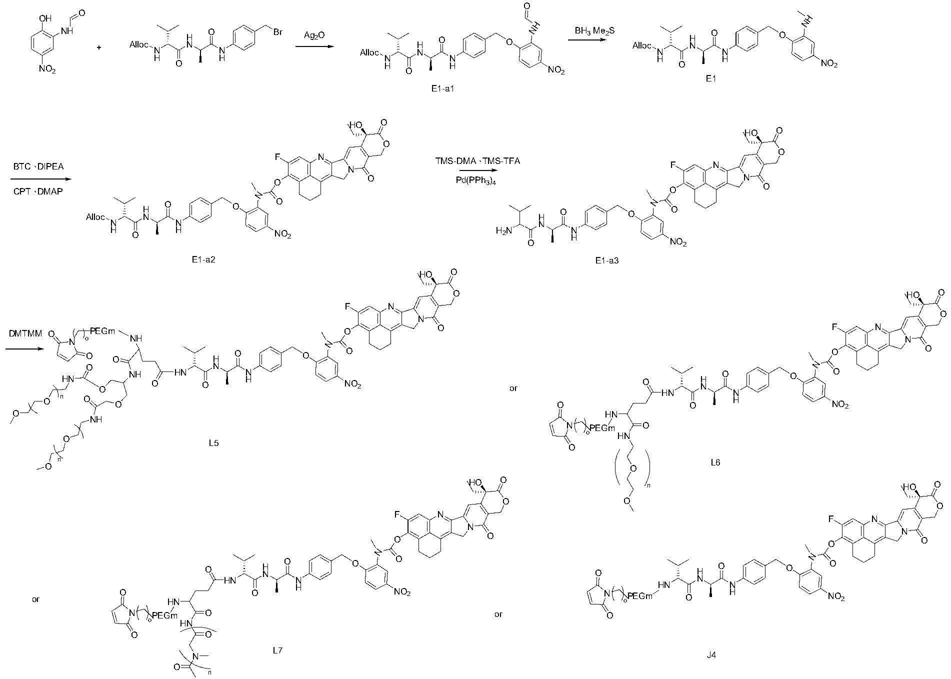

Camptothecin derivatives with different substrates can be converted to the key intermediate E1-a3 according to Route 6 by following General Procedure 1 and known synthetic conditions (reduction, condensation, Alloc deprotection, etc.). Intermediate E1-a3 is then coupled with various side chains (referring to the synthesis of L1-1 in the present invention) to afford L5, L6, L7, and J4.

### Synthesis of the side chain of L5

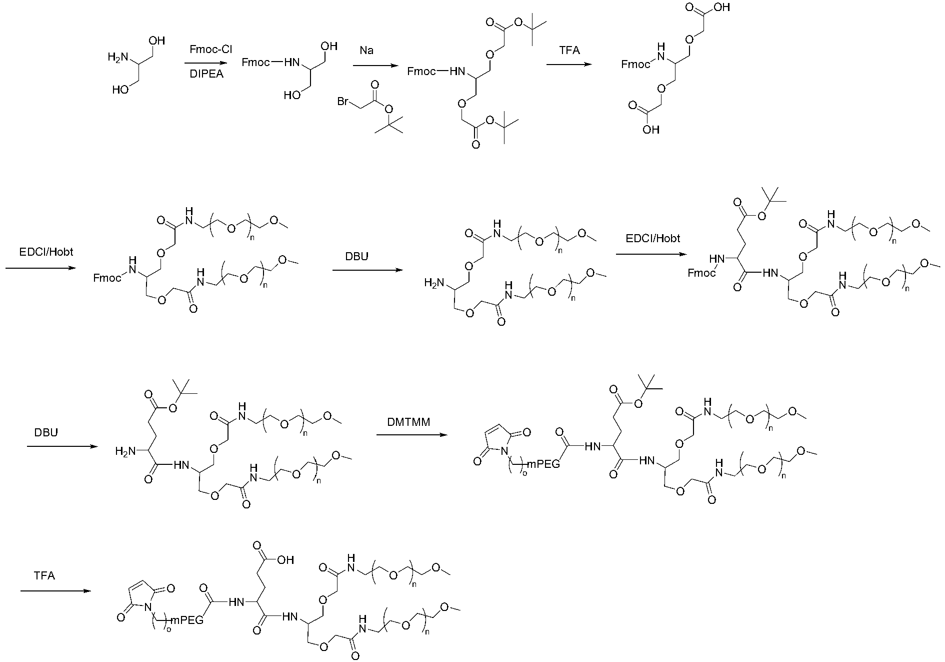

Overall yield of L5-1: 8%, MS:1103(M/2+H)⁺_{∘} 1H NMR (400 MHz, DMSO-d6 ) δ 8.59 (d, J = 3.1 Hz, 1H), 8.33 (s, 1H), 7.86(s, 2H), 7.80 -7.73 (m, 2H), 7.69 (s, 1H), 7.61 - 7.52 (m, 2H), 7.43 -7.39 (m, 3H), 7.20 (d, J = 15.0 Hz, 1H), 7.05 (d, J = 17.8 Hz, 2H), δ 6.37 (s, 1H), 5.76 (s, 1H), 5.56 (s, 1H), 5.41 - 5.34 (m, 2H), 5.21 (s, 2H), 5.16 - 5.05 (m, 1H), 5.04 (s, 2H), 4.94 (s, 2H), 4.50 (tt, *J* = 12.3, 5.3 Hz, 1H), 4.35 (s, 4H), 4.14 (t, *J* = 13.8 Hz, 1H), 3.98 (t, *J* = 11.1 Hz, 2H), 3.72-3.67 (m, 9H), 3.55 -3.52 (m, 6H), 3.52 (s, 52H), 3.47 - 3.43 (m, 10H), 3.25 - 3.19 (m, 8H), 3.12 - 3.08 (m, 4H), 2.95 - 2.67 (m, 2H), 2.66 - 2.47 (m, 1H), 2.34 (s, 3H), 2.30 - 1.90 (m, 10H), 1.70 - 1.47 (m, 5H), 1.44 - 1.17 (m, 8H), 1.02 - 0.80 (m, 9H).

Overall yield of L5-2: 11%, MS:1068(M/2+H)⁺_{∘} 1H NMR (400 MHz, DMSO-d6 ) δ 8.57 -8.54 (m, 1H), 8.32 (s, 1H), 7.88 (s, 2H), 7.80 -7.72 (m, 2H), 7.68 (s, 1H), 7.61 - 7.54 (m, 2H), 7.42 -7.39 (m, 3H), 7.20 -7.18 (m, 1H), 7.05 - 7.00 (m, 2H), δ 6.37 (s, 1H), 5.78 (s, 1H), 5.54 (s, 1H), 5.41 - 5.34 (m, 3H), 5.21 (s, 1H), 5.16 - 5.02 (m, 1H), 5.04 - 4.94 (m, 2H), 4.50- 4.35 (m, 4H), 4.14 - 3.97 (m, 3H), 3.72-3.67 (m, 8H), 3.55 -3.52 (m, 5H), 3.52 (s, 54H), 3.47 - 3.40 (m, 9H), 3.26 - 3.19 (m, 7H), 3.13 - 3.08 (m, 5H), 2.96 - 2.67 (m, 4H), 2.65 - 2.47 (m, 2H), 2.30 - 1.89(m, 10H), 1.69 - 1.47 (m, 4H), 1.40 - 1.19 (m, 6H), 1.02 - 0.80 (m, 9H).

Overall yield of L5-3: 14%, MS:1058(M/2+H)⁺_{∘} 1H NMR (400 MHz, DMSO-d6 ) δ 8.56 -8.50 (m, 1H), 8.33 (s, 1H), 7.86 (s, 2H), 7.80 -7.72 (m, 2H), 7.68 (s, 2H), 7.60 - 7.56 (m, 2H), 7.41 -7.39 (m, 3H), 7.22 -7.18 (m, 1H), 7.06 - 7.00 (m, 2H), 5.78 - 5.70 (m, 2H), 5.56 (s, 2H), 5.40 - 5.32 (m, 2H), 5.21 - 5.02 (m, 2H), 5.04 - 4.96 (m, 2H), 4.51- 4.37 (m, 3H), 4.14 - 3.99 (m, 3H), 3.74 -3.66 (m, 10H), 3.55 -3.52 (m, 8H), 3.52 (s, 50H), 3.50 - 3.40 (m, 8H), 3.25 - 3.19 (m, 6H), 3.15 - 3.08 (m, 4H), 2.95 - 2.67 (m, 4H), 2.65 - 2.47 (m, 4H), 2.30 - 1.90 (m, 8H), 1.64 - 1.45 (m, 3H), 1.40 - 1.18 (m, 6H), 1.02 - 0.80 (m, 9H).

### Synthesis of the side chain of L6

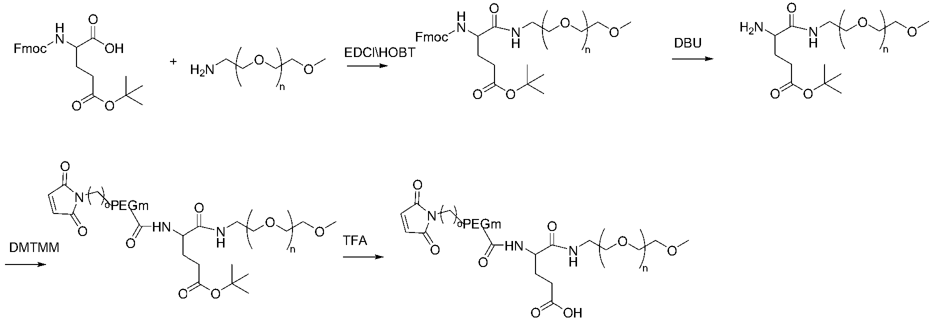

Overall yield of L6-1: 7%, MS:1785(M+H)⁺_{∘} 1H NMR (400 MHz, DMSO-*d*6 ) δ 8.59 (d, J = 1.4 Hz, 1H), 8.18 (s, 1H), 7.83 (dd, J = 7.5, 1.4 Hz, 1H), 7.56 (d, J = 7.5 Hz, 2H), 7.44 (d, J = 7.5 Hz, 2H), 7.34 (d, J = 8.1 Hz, 1H), 7.20 (d, J = 7.5 Hz, 1H), 7.14 (s, 2H), 6.99 (s, 1H), 6.53 (d, J = 9.5 Hz, 2H), 6.33 (s, 1H), 5.73 (d, J = 25.5 Hz, 2H), 5.44 (s, 1H), 5.38 (q, J = 6.2 Hz, 1H), 5.32 (s, 1H), 5.21 (t, J = 6.4 Hz, 1H), 5.18 (d, J = 1.4 Hz, 2H), 5.04 (s, 2H), 4.99 (d, J = 11.0 Hz, 1H), 4.75 (t, J = 7.9 Hz, 2H), 3.75 (t, J = 6.0 Hz, 2H), 3.70 - 3.65 (m, 4H), 3.62 (t, J = 7.8 Hz, 2H), 3.55 (t, J = 3.0 Hz, 2H), 3.52 (s, 38H), 3.44 - 3.37 (m, 6H), 3.28 (t, J = 4.0 Hz, 2H), 3.25 (s, 3H), 2.97 (t, J = 5.8 Hz, 2H), 2.79 - 2.62 (m, 5H), 2.57 (q, J = 6.9 Hz, 1H), 2.33 - 2.12 (m, 4H), 2.05 (t, J = 5.5 Hz, 2H), 1.93 (q, J = 6.8 Hz, 1H), 1.44 (d, J = 6.1 Hz, 3H), 0.96 (d, J = 6.4 Hz, 6H), 0.89 (t, J = 6.8 Hz, 3H).

### Synthesis of the side chain of L7

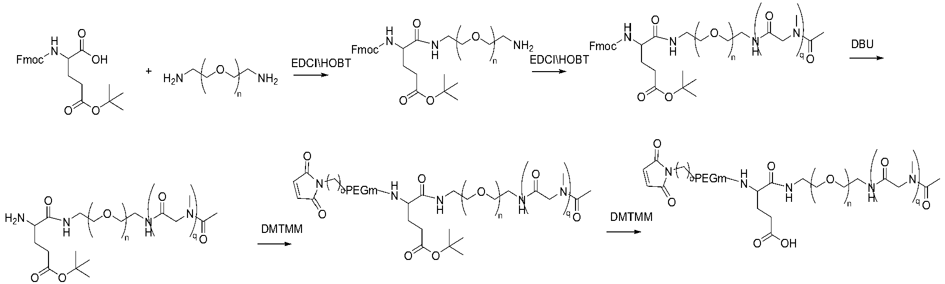

Overall yield of L7-1: 10%, 1H NMR (400 MHz, DMSO-d6 ) δ 8.90 (s, 1H), 8.59 (d, J = 2.3 Hz, 1H), 8.38 (s, 1H), 7.83 (dd, J = 12.0, 2.5 Hz, 1H), 7.66 - 7.11 (m, 8H), 7.00 (d, J = 3.9 Hz, 3H), 6.35 (s, 1H), 5.81 - 5.72 (m, 2H), 5.66 (q, J = 6.7 Hz, 1H), 5.49 (s, 1H), 5.33 (d, J = 17.6 Hz, 1H), 5.03 (d, J = 10.8 Hz, 4H), 4.75 (t, J = 8.3 Hz, 2H), 4.22 (t, J = 12.0 Hz, 1H), 3.91 (s, 20H), 3.81 - 3.58 (m, 10H), 3.52 (s, 16H), 3.41 (td, J = 11.4, 1.1 Hz, 2H), 3.34 - 3.16 (m, 8H), 3.01 - 2.90 (m, 32H), 2.82 - 2.57 (m, 5H), 2.37 - 2.00 (m, 11H), 1.44 (d, J = 9.8 Hz, 3H), 0.96 (d, J = 10.2 Hz, 6H), 0.89 (t, J = 10.8 Hz, 3H).

Overall yield of L7-2: 9%, 1H NMR (400 MHz, DMSO-d6 ) δ 8.58 (d, J = 2.3 Hz, 1H), 8.23 (s, 1H), 8.08 (s, 1H), 7.82 (dd, J = 11.9, 3.4 Hz, 1H), 7.62 - 7.37 (m, 5H), 7.32 - 7.10 (m, 4H), 7.05 (s, 1H), 6.58 (s, 1H), 5.81 (s, 1H), 5.73 (s, 1H), 5.49 (s, 1H), 5.38 (d, J = 9.2 Hz, 2H), 5.17 (d, J = 17.6 Hz, 1H), 5.06 - 4.90 (m, 6H), 4.75 (t, J = 8.5 Hz, 2H), 4.59 - 4.46 (m, 1H), 3.91 (s, 20H), 3.68 - 3.56 (m, 10H), 3.52 (s, 16H), 3.41 (dd, J = 13.6, 6.9 Hz, 3H), 3.33 - 3.17 (m, 7H), 3.08 - 2.89 (m, 33H), 2.82 - 2.52 (m, 5H), 2.32 - 1.94 (m, 11H), 1.44 (d, J = 9.8 Hz, 3H), 0.96 (d, J = 10.2 Hz, 6H), 0.89 (t, J = 10.8 Hz, 3H).

Overall yield of L7-3: 6%. 1H NMR (400 MHz, DMSO-d6 ) δ 8.59 (d, J = 2.3 Hz, 1H), 8.07 (s, 1H), 7.83 (dd, J = 12.0, 2.3 Hz, 1H), 7.59 - 7.27 (m, 9H), 7.20 (d, J = 12.0 Hz, 1H), 7.00 (s, 1H), 6.92 (s, 1H), 6.45 (s, 1H), 6.34 (s, 1H), 5.63 (t, J = 11.0 Hz, 1H), 5.54 (d, J = 13.9 Hz, 2H), 5.27 (d, J = 6.3 Hz, 3H), 5.05 (d, J = 4.2 Hz, 4H), 5.01 - 4.93 (m, 1H), 4.75 (t, J = 8.3 Hz, 2H), 4.52 - 4.41 (m, 1H), 3.91 (s, 2H), 3.72 - 3.57 (m, 8H), 3.52 (s, 16H), 3.50 - 3.36 (m, 3H), 3.33 - 3.23 (m, 7H), 2.96 (s, 3H), 2.93 - 2.49 (m, 8H), 2.34 (s, 3H), 2.30 - 2.12 (m, 2H), 2.10 (d, J = 5.1 Hz, 3H), 2.09 - 1.92 (m, 4H), 1.84 - 1.79 (m, 1H), 1.44 (d, J = 9.8 Hz, 3H), 0.96 (d, J = 10.3 Hz, 6H), 0.89 (t, J = 10.8 Hz, 3H).

### The side chain of J4 was purchased from various reagent companies.

Overall yield of J4-1: 13%. 1H NMR (400 MHz, DMSO-d6 ) δ 8.59 (d, J = 2.3 Hz, 1H), 7.83 - 7.78 (m, 1H), 7.65 - 7.60 (m, 1H), 7.58 - 7.53 (m, 2H), 7.41 - 7.36(m, 4H), 7.23 - 7.17 (m, 2H), 7.12 (s, 2H), 7.00 (s, 1H), 6.25 (s, 1H), 5.60 (d, J = 6.9 Hz, 2H), 5.42 (s, 1H), 5.22 - 5.01 (m, 4H), 4.98 (s, 2H), 4.75 (t, J = 11.0 Hz, 2H), 3.65 -3.60 (m, 4H), 3.54 - 3.49 (m, 29H), 3.48 (s, 1H), 3.41 (s, 1H), 3.28 (t, J = 8.0 Hz, 2H), 3.25 (s, 3H), 2.84 - 2.69 (m, 1H), 2.66 (t, J = 11.0 Hz, 2H), 2.37 (t, J = 13.2 Hz, 2H), 2.18 -2.01 (m, 2H), 1.44 (d, J = 10.5 Hz, 3H), 1.18 (t, J = 10.7 Hz, 3H), 0.96 (d, J = 10.2 Hz, 6H), 0.89 (t, J = 10.8 Hz, 3H).

Overall yield of J4-2: 13%. 1H NMR (400 MHz, DMSO-d6 ) δ 9.43 (s, 1H), 8.59 (d, J = 1.4 Hz, 1H), 7.83 (dd, J = 7.5, 1.4 Hz, 1H), 7.56 (d, J = 7.5 Hz, 2H), 7.44 (d, J = 7.5 Hz, 2H), 7.29 (d, J = 8.1 Hz, 1H), 7.20 (d, J = 7.5 Hz, 1H), 7.11 (s, 2H), 6.99 (s, 1H), 6.81 (s, 1H), 5.64 (d, J = 10.6 Hz, 2H), 5.46 (s, 1H), 5.30 - 5.13 (m, 2H), 5.04 (s, 2H), 5.00 - 4.90 (m, 2H), 4.75 (t, J = 7.7 Hz, 2H), 3.84 - 3.56 (m, 6H), 3.52 (s, 28H), 3.44 (s, 1H), 3.34 - 3.20 (m, 5H), 2.97 (t, J = 5.8 Hz, 2H), 2.80 - 2.53 (m, 3H), 2.35 (t, J = 4.3 Hz, 2H), 2.31 - 2.02 (m, 4H), 1.44 (d, J = 6.1 Hz, 3H), 0.96 (d, J = 6.4 Hz, 6H), 0.89 (t, J = 6.8 Hz, 3H).

J1-2 was synthesized from various camptothecin derivatives by referring to J1-1 and Route A. Overall yield is 11%. MS:1558(M+H)⁺_{∘} 1H NMR (500 MHz, DMSO-d6 ) δ 8.56 (d, J = 2.9 Hz, 1H), 8.33 (s, 1H), 7.86 - 7.71 (m, 2H), 7.40 (s, 2H), 7.16 (dd, J = 14.9, 9.4 Hz, 2H), 7.01 (d, J = 15.9 Hz, 1H), 6.92 - 6.75 (m, 2H), 6.19 (s, 1H), 5.84 (s, 1H), 5.57 (dd, J = 18.0, 10.4 Hz, 3H), 5.14 (s, 2H), 4.96 (s, 2H), 4.73 (t, J = 10.2 Hz, 2H), 4.64 - 4.48 (m, 2H), 4.22 - 4.03 (m, 2H), 3.86 - 3.55 (m, 8H), 3.51 (s, 28H), 3.48 (s, 2H), 3.38 (s, 1H), 3.27 (t, J = 7.6 Hz, 2H), 3.24 (s, 3H), 2.96 (t, J = 11.8 Hz, 2H), 2.67 (dt, J = 19.4, 13.0 Hz, 4H), 2.50 (t, J = 8.4 Hz, 2H), 2.35 (s, 1H), 2.30 - 2.10 (m, 4H), 2.07 (s, 1H), 0.89 (t, J = 10.8 Hz, 3H).

J1-3 was synthesized from various camptothecin derivatives by referring to J1-1 and Route A. Overall yield is 10%. MS:1541(M+H)⁺_{∘} 1H NMR (500 MHz, DMSO-*d*6 ) δ 9.11 (s, 1H), 8.59 (d, *J* = 3.1 Hz, 1H), 8.29 (s, 1H), 7.92 - 7.74 (m, 2H), 7.50 (d, J = 15.0 Hz, 1H), 7.28 (d, J = 14.1 Hz, 3H), 7.19 (dd, J = 15.0, 9.5 Hz, 2H), 7.00 (s, 1H), 6.83 (dd, J = 15.0, 2.9 Hz, 1H), 5.92 (s, 1H), 5.75 (s, 1H), 5.63 (s, 1H), 5.25 - 5.07 (m, 3H), 4.98 (s, 2H), 4.75 (t, J = 10.2 Hz, 2H), 4.45 - 4.39(m, 2H), 3.96 (s, 1H), 3.91 - 3.57 (m, 9H), 3.52 (s, 28H), 3.31- 3.27(m, 4H), 3.25 (s, 3H), 2.97 (t, J = 11.7 Hz, 2H), 2.81 - 2.52 (m, 6H), 2.34 - 2.02 (m, 5H), 1.75 (s, 1H), 0.89 (t, J = 10.8 Hz, 3H).

### Synthesis of J2-1

### The side chain of Lysine:

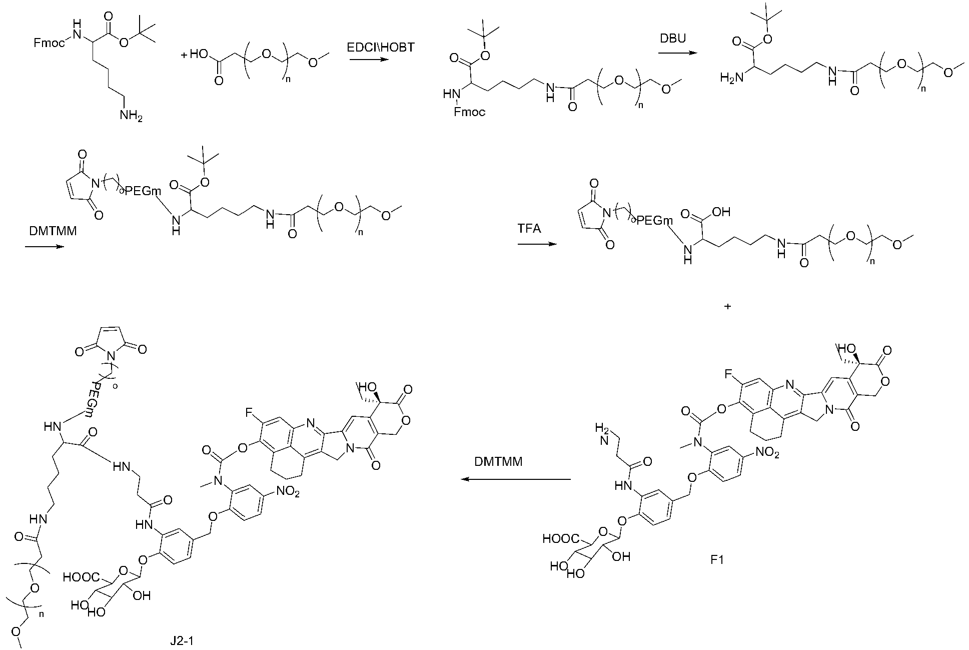

Overall yield of J2-1: 8%. 1H NMR (400 MHz, DMSO-d6 ) δ 8.59 (d, J = 1.4 Hz, 1H), 8.12 (s, 1H), 7.83 (dd, J = 8.8, 1.4 Hz, 2H), 7.19 (dd, J = 9.9, 7.5 Hz, 2H), 7.11 (s, 2H), 6.98 - 6.89 (m, 2H), 6.86 - 6.76 (m, 2H), 6.45 (s, 1H), 5.83 (s, 1H), 5.64 (s, 1H), 5.59 (s, 1H), 5.16 (s, 2H), 5.05 (d, J = 7.7 Hz, 1H), 4.96 (t, J = 6.8 Hz, 2H), 4.52 (t, J = 6.9 Hz, 1H), 4.35 (dd, J = 9.7, 8.3 Hz, 1H), 4.22 (t, J = 8.0 Hz, 1H), 4.00 (t, J = 5.1 Hz, 2H), 3.82 (t, J = 8.3 Hz, 1H), 3.75 (t, J = 5.8 Hz, 2H), 3.65 - 3.58 (m, 4H), 3.55 (ddd, J = 6.2, 5.0, 2.2 Hz, 3H), 3.52 - 3.50 (m, 44H), 3.41 (d, J = 5.5 Hz, 4H), 3.25 (s, 3H), 2.99 (dt, J = 11.8, 5.4 Hz, 4H), 2.76 - 2.64 (m, 3H), 2.57 (t, J = 4.2 Hz, 2H), 2.35 (t, J = 8.0 Hz, 2H), 2.27 - 2.07 (m, 4H), 1.98 - 1.79 (m, 4H), 1.63 - 1.59 (m, 5H), 1.35 - 1.21 (m, 6H), 0.89 (t, J = 6.8 Hz, 3H).

Overall yield of J2-2 :7%. 1H NMR (400 MHz, DMSO-d6 ) δ 8.59 (d, J = 2.9 Hz, 1H), 7.88 - 7.75 (m, 2H), 7.50 (d, J = 15.0 Hz, 1H), 7.32 - 7.16 (m, 3H), 7.13 (s, 2H), 7.03 (s, 1H), 6.83 (dd, J = 15.0, 2.9 Hz, 1H), 6.17 (d, J = 18.5 Hz, 2H), 6.07 (d, J = 15.6 Hz, 1H), 5.73 (d, J = 29.1 Hz, 2H), 5.51 (s, 1H), 5.16 (s, 2H), 5.05 (t, J = 13.0 Hz, 1H), 4.97 (s, 2H), 4.26 (ddd, J = 17.2, 15.8, 4.5 Hz, 2H), 4.05 - 3.71 (m, 7H), 3.66 - 3.58 (m, 4H), 3.57 - 3.47 (m, 49H), 3.40 (s, 3H), 3.25 (s, 3H), 3.08 - 2.90 (m, 4H), 2.77 - 2.45 (m, 6H), 2.31 - 2.15 (m, 3H), 2.09 - 1.80 (m, 4H), 1.71 - 1.55 (m, 3H), 1.53 - 1.17 (m, 8H), 0.89 (t, J = 13.6 Hz, 3H).

### Synthesis of J3-1:

The key intermediate F1 was coupled with the side chain of L7 to afford the target product J3-1. Overall yield is 9%. 1H NMR (400 MHz, DMSO-d6 ) δ 8.76 (s, 1H), 8.59 (d, J = 2.9 Hz, 1H), 8.04 (s, 1H), 7.91 - 7.75 (m, 2H), 7.19 (dd, J = 15.0, 9.5 Hz, 2H), 7.08 (s, 2H), 6.91 (s, 1H), 6.82 (dt, J = 14.3, 7.1 Hz, 1H), 6.77 (s, 1H), 6.20 (s, 1H), 5.83 (s, 1H), 5.70 (s, 1H), 5.62 (s, 1H), 5.43 (d, J = 15.6 Hz, 2H), 5.16 (s, 2H), 4.97 (s, 2H), 4.75 (t, J = 10.9 Hz, 2H), 4.33 - 4.10 (m, 2H), 3.94 - 3.89 (m, 20H), 3.99 - 3.79 (m, 5H), 3.89 - 3.56 (m, 14H), 3.51 (d, J = 5.7 Hz, 8H), 3.49 - 3.35 (m, 3H), 3.32 - 3.23 (m, 7H), 3.21 - 3.11 (m, 1H), 3.01 - 2.93 (m, 32H), 2.74 - 2.58 (m, 9H), 2.30 - 2.17 (m, 3H), 2.13 (d, J = 20.0 Hz, 6H), 2.09 - 1.73 (m, 5H), 1.57 (s, 2H), 0.89 (t, J = 10.8 Hz, 3H).

### Example 8 Antibody Preparation

The light and heavy chains of the antibody were separately constructed into the expression cassette of the plasmid vector, and the plasmids were extracted. Suspension-adapted CHO cells were revived in OPM-CD medium (Manufacturer: OPM, Cat. No. P83059) and cultured to a density of 2×10⁶ cells/mL with viability above 95% in a volume of 1000 mL. The light-chain plasmid (0.5 mg) and heavy-chain plasmid (0.5 mg) were combined and dissolved in 10 mL of medium. 3 mg of PEI (Manufacturer: Polyscience, Cat. No. 24765-1) dissolved in medium was diluted into 10 mL of medium. The plasmid and PEI solutions were mixed, incubated at room temperature for 10 minutes, then added dropwise to 1000 mL of cell culture. After cultivation at 37 °C for 5 days, the supernatant was harvested by centrifugation at 12000 g for 15 minutes.

The supernatant was purified by affinity chromatography and eluted with 50 mM acetic acid. The neutralized fractions were buffer-exchanged into PBS pH 7.4 using a 30 kDa ultrafiltration tube (Manufacturer: Merck, Cat. No. UFC9030). The antibody concentration was determined by measuring the absorbance at 280 nm; the concentration was calculated as the absorbance divided by the theoretical extinction coefficient.

### Example 9 Preparation of Antibody-Drug Conjugates

For conjugation, 30 mg of antibody was diluted to 3 mg/mL with 10 mM sodium phosphate buffer pH 7.4. A stock solution of tris(2-carboxyethyl)phosphine hydrochloride (TCEP, 10 mM in 10 mM sodium phosphate buffer pH 7.4; Manufacturer: Sigma, Cat. No. C4706-2G, CAS 51805-45-9) was added to achieve a final molar ratio of TCEP to antibody of X:1. The antibody was reduced at 37 °C for 45 minutes.

A linker compound stock solution (5 mM in dimethyl sulfoxide, DMSO) was added to a final molar ratio of linker to antibody of Y:1. The mixture was incubated at 2-8 °C for 2 hours for conjugation. The reaction was quenched with 20 mM cysteine, and the mixture was buffer-exchanged using a 30 kDa ultrafiltration tube (Manufacturer: Merck, Cat. No. UFC9030) at 3000 g and 2-8 °C until residual small molecules were less than 1/1000 of the reaction concentration. The product was sterile-filtered, quantified, aliquoted, and frozen.

To obtain products with different drug-to-antibody ratios (DAR), the molar ratio of antibody:reducing agent:linker was adjusted within 1:X:Y while other conditions remained unchanged. The final DAR was determined by instrumental analysis. Typically, a molar ratio of X = 3, Y = 6 yielded conjugates with DAR ≈ 4, and X = 5, Y = 15 yielded conjugates with DAR ≈ 8.

### SEC-HPLC Analysis (Purity)

Antibodies and conjugates were analyzed by size-exclusion high-performance liquid chromatography (SEC-HPLC) using a TSKgel G3000SWXL column (7.8 mm × 30 cm, 5 µm, Cat. No. 08541). Approximately 0.3 mg of sample (300 µL) was centrifuged at 10000 g for 5 minutes, and the supernatant was injected. The mobile phase was 50 mM sodium phosphate + 0.1 M sodium chloride pH 6.8. The column was attached to an Agilent 1260 HPLC system; flow rate = 0.8 mL/min. The column was equilibrated with mobile phase for >30 min until the 280 nm UV baseline was stable. A 100 µg sample was injected and eluted for 20 minutes. The percentages of high-molecular-weight species, monomer, and low-molecular-weight species were calculated from peak areas; the monomer percentage was taken as sample purity.

### HIC-HPLC Analysis (DAR)

The average number of linker payloads conjugated per antibody (DAR) was determined by hydrophobic interaction chromatography (HIC-HPLC) using a TSKgel Butyl-NPR column (4.6 mm × 10 cm, 2.5 µm, Cat. No. 042168). Approximately 0.3 mg of sample (150 µL) was mixed with 150 µL of mobile phase A, centrifuged at 10000 g for 5 minutes, and the supernatant was injected. Mobile phase A: 20 mM sodium phosphate + 1.5 M ammonium sulfate pH 7.0. Mobile phase B: 20 mM sodium phosphate + 20% (v/v) acetonitrile pH 7.0. The column was attached to an Agilent 1260 HPLC system; flow rate = 0.6 mL/min. After equilibration with mobile phase A for >30 min until the 280 nm UV baseline was stable, a 50 µg sample was injected. The column was washed with mobile phase A for 2 min, followed by an 18-minute linear gradient from 0% B to 100% B, then held at 100% B for 5 minutes.

The percentage of each DAR species was calculated from peak areas. The average DAR was obtained by summing (percentage × DAR) for all peaks and dividing by the total percentage. The DAR of the conjugates in this invention is not limited to 8 and can range from 2 to 8.

Conjugates were named according to target-compound-DAR, as shown in Table 1.

**Table 1 Antibody-Drug Conjugates**

| **Antibody to be conjugated** | **Linker compound / linker-payload** | **Conjugate name** | **ADC HIC-HPLC DAR** | **ADC** |
|---|---|---|---|---|
| 3101, B7-H3 | L5-3 | B7-H3 L5-3 DAR4 | 4 | 98.8 |
| | J1-2 | B7-H3 J1-2 DAR4 | 4 | 95.5 |
| | J1-3 | B7-H3 J1-3 DAR4 | 4.5 | 99.3 |
| | J2-1 | B7-H3 J2-1 DAR4 | 4.9 | 99.11 |
| | J2-2 | B7-H3 J2-2 DAR4 | 4.9 | 99.48 |
| | L1-2 | B7-H3 L1-2 DAR4 | 5.8 | 99.62 |
| | L1-4 | B7-H3 L1-4 DAR4 | 6.0 | 99.2 |
| | AZ0133 | B7-H3 AZ0133 DAR4 | 5 | 97.6 |
| | Deruxtecan | B7-H3 Deruxtecan DAR4 | 4.4 | 92.7 |
| Trastuzumab, HER2 | J1-2 | HER2 J1-2 DAR8 | 8.5 | 94.98 |
| | J1-3 | HER2 J1-3 DAR8 | 8.2 | 92.03 |
| | J2-1 | HER2 J2-1 DAR8 | 8.4 | 95.6 |
| | J2-2 | HER2 J2-2 DAR8 | 7.8 | 98.5 |
| | AZ0133 | HER2 AZ0133 DAR8 | 6 | 95.1 |
| | Deruxtecan | HER2 Deruxtecan DAR8 | 6.5 | 96.3 |
| 59H4, DLL3 | J2-1 | DLL3 J2-1 DAR8 | 8 | 89.9 |
| | Deruxtecan | DLL3 Deruxtecan DAR8 | 8 | 91.9 |
| Isotype | AZ0133 | Isotype AZ0133 | 5.3/ 8 | 96.2/ 70.3 |
| | Deruxtecan | Isotype Deruxtecan | 5.6/ 8 | 95.56/ 97.03 |

### Example 10 In Vivo Activity of Antibody-Drug Conjugates

Human tumor cell lines were revived and cultured to the logarithmic growth phase with viability >90%. Immunodeficient mice (NCG or equivalent) were inoculated with 1 × 10⁴ to 1×10⁶ cells. When tumor volume reached approximately 120 mm², mice were randomized into groups of 4-5 animals and administered a single intravenous dose on the day of grouping. Tumor size was measured twice weekly. Antitumor activity was evaluated by comparing tumor volumes among treatment groups and the vehicle group. In the graphs, the x-axis represents time after treatment or days after inoculation, and the y-axis represents tumor volume.

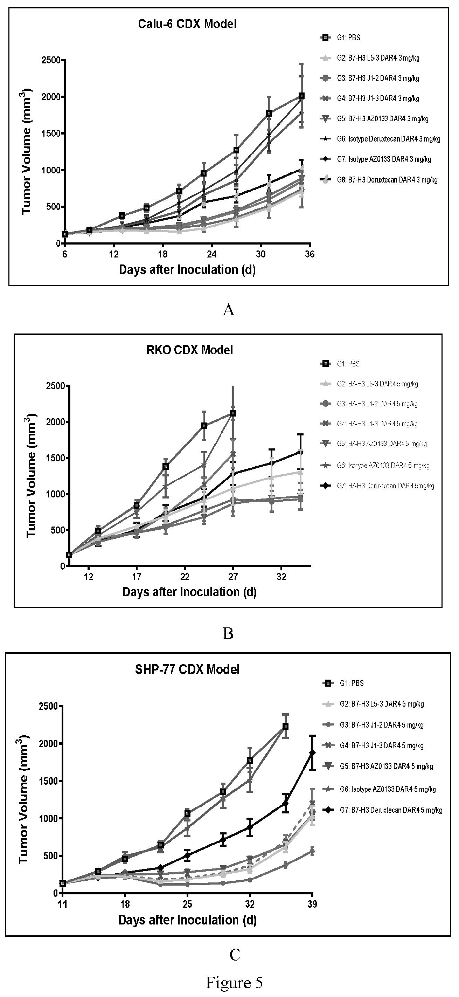

Several linker-payload conjugates were prepared with the anti-B7-H3 antibody (3101) and the tumor cell growth inhibitory activity of the resulting conjugates was evaluated in an immunodeficient mouse tumor xenograft model using the human lung cancer cell line Calu-6. As shown in Figure 5A, tumor volumes ranked in descending order: G1 > G6 > G7 > G8 > G5 > G4 > G3 > G2. Conjugates of the candidate compounds exhibited comparable activity with no significant differences and were superior to the positive control.

The tumor cell growth inhibitory activity of the aforementioned conjugates were further evaluated in an immunodeficient mouse tumor xenograft model using the human colorectal cancer cell line RKO (Figure 5B). Tumor volumes ranked: G1 or G6 > G4 > G7 > G2 > G3 > G5. Conjugate G3 showed favorable antitumor activity.

The tumor cell growth inhibitory activity of the aforementioned conjugates were further evaluated in an immunodeficient mouse tumor xenograft model using the human lung cancer cell line SHP-77 (Figure 5C), tumor volumes ranked: G1 or G6 > G7 > G4 or G5 or G2 > G3. Conjugate G3 again demonstrated superior activity.

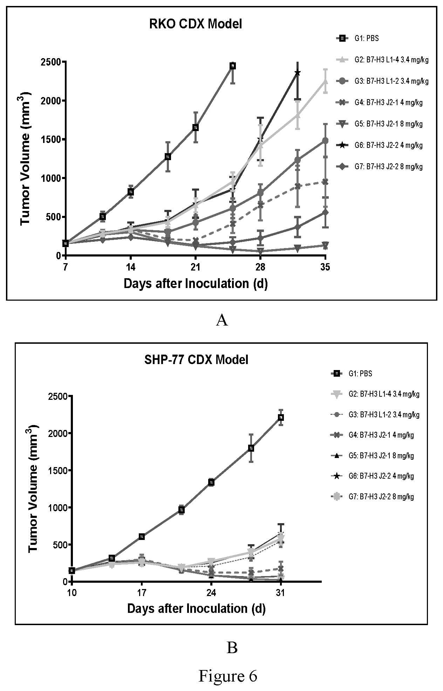

Linker compounds were further optimized, conjugated, and the tumor cell growth inhibitory activity of the resulting conjugates was re-evaluated in an immunodeficient mouse tumor xenograft model using the human colorectal cancer cell line RKO and the human lung cancer cell line SHP-77. As shown in Figure 6A (RKO), tumor volumes ranked: G1 > G6 > G2 > G3 > G4 > G7 > G5. Conjugate G5 exhibited the best in vivo activity. As shown in Figure 6B (SHP-77), tumor volumes ranked: G1 > G6 > G2 > G3 > G4 > G7 > G5. Conjugate G5 exhibited the best in vivo activity.

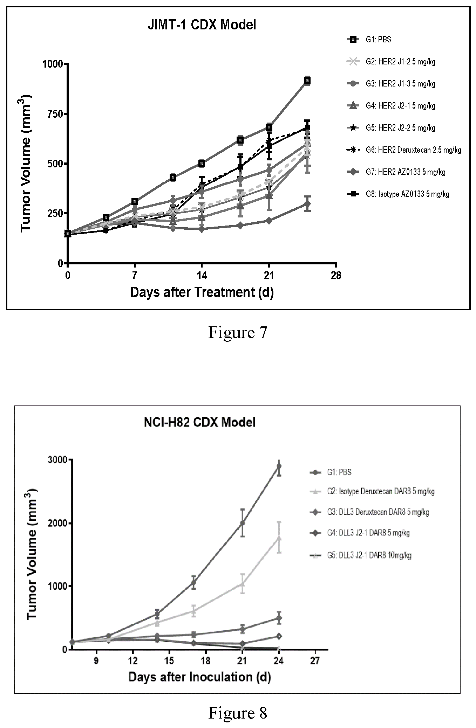

Based on the above results, conjugate G5 showed slightly superior tumor growth inhibition compared with other groups. The linker compound was further conjugated to trastuzumab (anti-HER2 antibody) and the tumor cell growth inhibitory activity of the resulting conjugates was evaluated in an immunodeficient mouse tumor xenograft model using the human breast cancer cell line JIMT-1 (Figure 7). Tumor volumes ranked: G1 > G6 or G8 > G3 > G2 > G4 > G6 > G7. Conjugate G4 showed favorable activity during the intermediate phase, comparable to or slightly lower than the control conjugate.

Linker compound J2-1 was conjugated to the anti-DLL3 antibody 59H4 and the tumor cell growth inhibitory activity of the resulting conjugates was evaluated in an immunodeficient mouse tumor xenograft model using the human lung cancer cell line NCI-H82 (Figure 8). Tumor volumes ranked: G1 > G2 > G3 > G2 > G4 > G5. The conjugate of J2-1 exhibited improved activity compared with the control.

In summary, antibody-drug conjugates comprising linker compounds J1-2, J1-3, J2-1, and J2-2 exhibited in vivo antitumor activity comparable to or superior to control conjugates.

### Example 11 In Vitro Activity of Antibody-Drug Conjugates

In vitro activities of candidate linker-payloads and their conjugates were determined. Human tumor cell lines were revived and cultured to the logarithmic growth phase with viability >90%. Cells were seeded in 96-well plates at 500-5000 cells/well in 150 µL of medium and incubated at 37 °C in a 5% CO₂ incubator for approximately 20 h. 50 µL of medium containing ADCs at various concentrations was added to achieve 12 serial dilutions from 0 to 1000 nM (starting at 1000 nM, 5-fold dilutions, zero-drug control, replicates for each sample). After 4-7 days of co-incubation, 50 µL of CTG reagent (CellTiter-Glo, Promega, Cat. No. G7575) was added to each well. Following a 2-minute reaction, fluorescence intensity (MFI) was measured using a Tecan Spark reader according to the manufacturer's instructions.

Mean MFI values were plotted against log₁₀(concentration, nM), and EC/IC50 values were calculated by four-parameter logistic fitting.

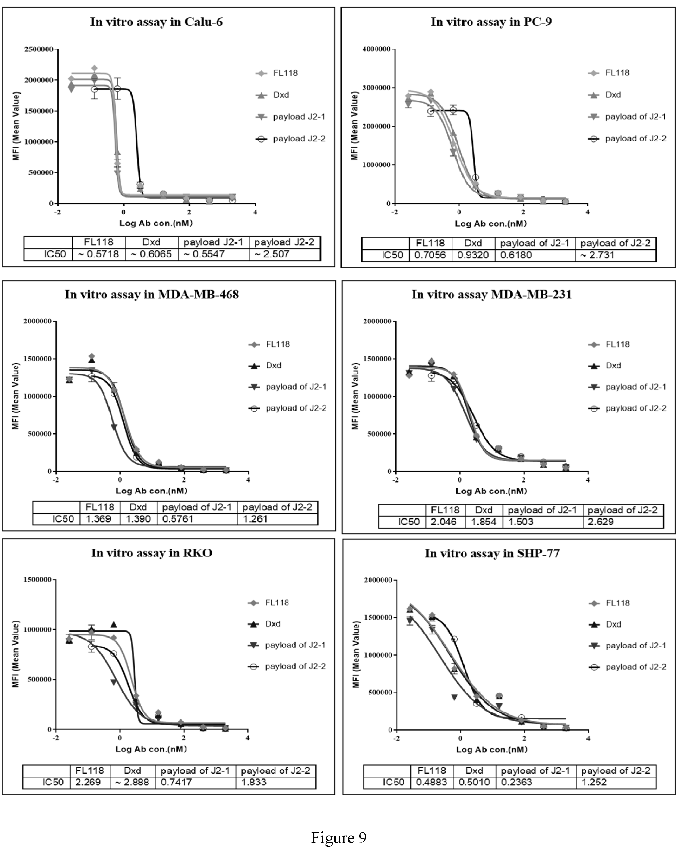

First, free payloads were tested in multiple tumor cell lines: Deruxtecan payload (Dxd), J2-1 payload, J2-2 payload, and FL118. As shown in Figure 9, cytotoxicity varied across cell lines.

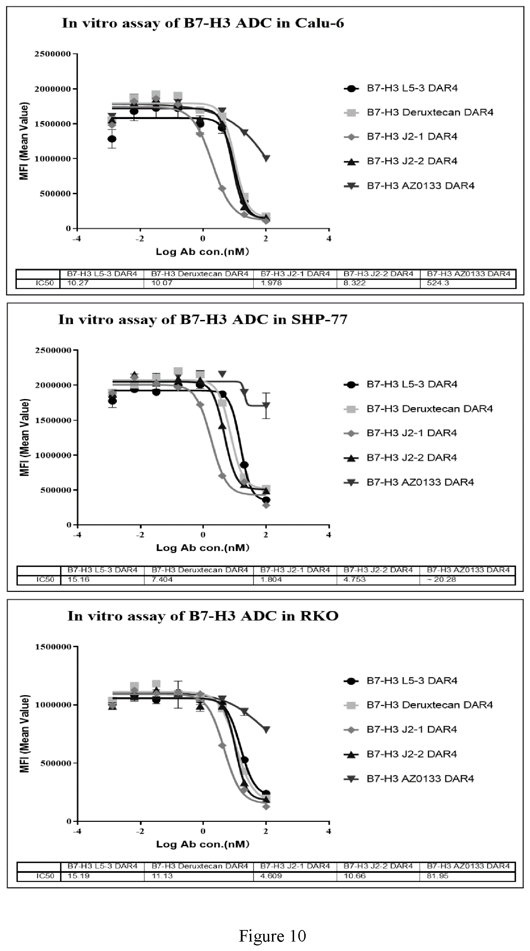

In vitro activities of ADCs were then determined (Figure 10). Ranking of in vitro potency among conjugates differed from that observed in vivo. Antitumor efficacy was primarily based on in vivo CDX models.

The scope of protection of the present invention is not limited to the above examples. Without departing from the spirit and scope of the inventive concept, variations and advantages conceivable by those skilled in the art are encompassed within the present invention, with the scope of protection defined by the appended claims.

## Claims

1. A linker compound useful in a prodrug delivery system, and pharmaceutically acceptable salts, solvates, polymorphs, or isomers thereof, wherein, the structure of the linker compound is as shown in formula (1): 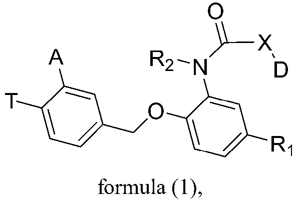
wherein, T represents an attachment site for linking R-TR;
in the R-TR, TR is selected from one or more of the following: a cathepsin substrate; an amino acid or short peptide chain of a cathepsin substrate; a β-glucuronidase substrate; a galactosidase substrate; a metalloproteinase substrate; an aromatic boronic acid or boronic ester oxidizable to a hydroxyl group; a nitro group, an azide group, or an azo group reducible to an amino group; a phosphate ester of a hydroxyl group, or a sulfate ester of a hydroxyl group; R is a substituent group selected from one or more of -H, PEG, maleimide-modified PEG, a sugar, folic acid, a polypeptide, an antibody, or a protecting group;
wherein, A represents an attachment site for linking LR;
the LR is selected from one or more of H, CH₃(CH₂)n, a nitro group, a methoxy group, a PEG chain, maleimide-modified PEG, a maleimide-modified fatty chain, a maleimide-modified polyamino acid chain, a fatty chain, a polyamino acid chain, a sugar chain, a fatty acid, a sugar, folic acid, a polypeptide, or an antibody;
wherein, D represents an attachment site for linking DR;
the DR contains an exposed -OH, -NH₂, -NHR, or -SH; the DR is a chemical drug;
wherein, X is O, N, or S;
wherein, R₂ is H or CH₃(CH₂)n; n = 0-20;
wherein, R₁ is an electron-withdrawing group.

2. The linker compound according to claim 1, wherein, the chemical drug is a camptothecin compound or a paclitaxel compound, or a derivative of these two types of compounds;
and/or, the electron-withdrawing group R1 includes halogen, cyano, nitro, carboxylic acid, sulfonic acid, carboxylate ester group, sulfonate ester group, amide, and/or a group linked via an amide bond.

3. The linker compound according to claim 2, wherein, the chemical drug comprises SN-38, DXd, Exatecan, Belotecan, Paclitaxel, Docetaxel, Podophyllotoxin, or CA4.

4. The linker compound according to claim 1, wherein, when the attachment site T is connected via an amide bond, the structure of the linker compound is as shown in formula (1-1) below: 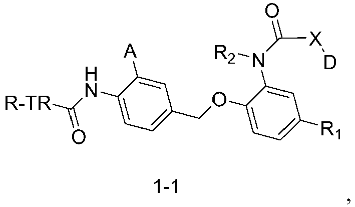
wherein, TR is selected from a single amino acid, a dipeptide, or a tetrapeptide among cathepsin substrates or metalloproteinase substrates;
wherein the single amino acid is selected from unprotected or protected single amino acids, PEG chains, polyamino acid chains, and maleimide-modified single amino acids, including -Lys-, -Cit-, -Arg-, -Leu-, -His-, -Asp-, -Glu-, -Ala-, -Val-, -Ile-, -Pro-, -Phe-, -Trp-, -Met-, -Ser-, -Cys- or -Asn-; the dipeptide is selected from unprotected or protected dipeptides, PEG chains, polyamino acid chains, and maleimide-modified dipeptides, including -Phe-Lys-, -Val-Lys-, -Ala-Lys-, -Val-Cit-, -Phe-Cit-, -Leu-Cit-, -Ile-Cit, -Trp-Cit-, -Phe-Gly-, -Ala-Ala- or -Phe-Arg-; the tetrapeptide is selected from unprotected or protected tetrapeptides, PEG chains, polyamino acid chains, and maleimide-modified tetrapeptides, including -Gly-Gly-Phe-Gly-;
and/or,
when the attachment site T is connected via an ether bond or an ester bond, the structure of the linker compound is as shown in formula (1-2) below: 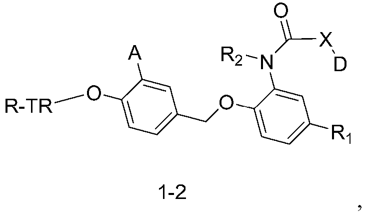
wherein, TR includes β-glucuronidase, β-galactosidase, phosphatase, sulfatase, and pyrophosphatase;
and/or, when the attachment site A is connected via an amide bond, the structure of the linker compound is as shown in formula (1-5) below: 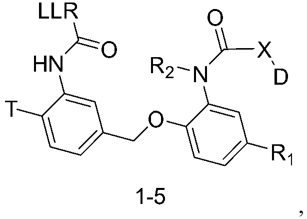
wherein, LLR is a modifying fragment, including one or more of PEG, a sugar, an alkyl chain, maleimide-modified PEG, a modified alkyl chain, or a fatty acid;
the group attached at the attachment site T includes β-glucuronidase, β-galactosidase, phosphatase, sulfatase, or pyrophosphatase;
and/or, when the group attached at the attachment site A is H, the structure of the linker compound is as shown in formula (1-6) below: 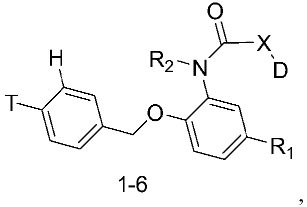
T is selected from a single amino acid, a dipeptide, or a tetrapeptide among cathepsin substrates or metalloproteinase substrates;
wherein the single amino acid is selected from unprotected or protected single amino acids, PEG chains, polyamino acid chains, and maleimide-modified single amino acids, including -Lys-, -Cit-, -Arg-, -Leu-, -His-, -Asp-, -Glu-, -Ala-, -Val-, -Ile-, -Pro-, -Phe-, -Trp-, -Met-, -Ser-, -Cys- or -Asn-; the dipeptide is selected from unprotected or protected dipeptides, PEG chains, polyamino acid chains, and maleimide-modified dipeptides, including -Phe-Lys-, -Val-Lys-, -Ala-Lys-, -Val-Cit-, -Phe-Cit-, -Leu-Cit-, -Ile-Cit, -Trp-Cit-, -Phe-Gly-, -Ala-Ala- or -Phe-Arg-; the tetrapeptide is selected from unprotected or protected tetrapeptides, PEG chains, polyamino acid chains, and maleimide-modified tetrapeptides, including -Gly-Gly-Phe-Gly-.

5. The linker compound according to claim 1, wherein, when R₁ is a group linked via an amide bond, the linker compound further comprises an attachment site L₁, and the structure of the linker compound is as shown in formula (L) below: 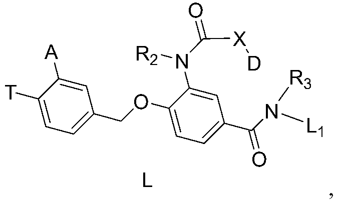
wherein, R₃ is selected from one or more of H, C1-C6 alkyl, or cycloalkyl; L₁ is selected from one or more of H, an amino protecting group, a fatty chain, a PEG chain, a polyamino acid chain, a sugar chain, and a maleimide-modified PEG chain, a maleimide-modified fatty chain, a maleimide-modified polyamino acid chain, or a maleimide-modified sugar chain;
R₃ and L₁ are capable of linking to form a 3-6 ring, including 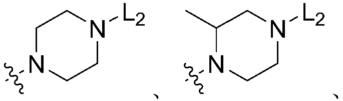 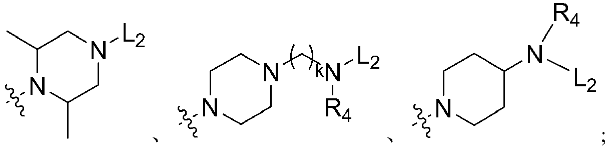 L₂ selected from one or more of H, an amino protecting group, a C1-C6 alkyl or cycloalkyl group, a fatty chain, a linear or branched PEG chain, a sugar chain, a polyamino acid chain, a maleimide-modified PEG chain, a maleimide-modified fatty chain, a maleimide-modified polyamino acid chain, or a maleimide-modified sugar chain;
R₄ is selected from one or more of H, C1-C6 alkyl, or cycloalkyl;
and/or,
when the R₁ is a nitro group, the structure of the linker compound is as shown in formula (J) below:

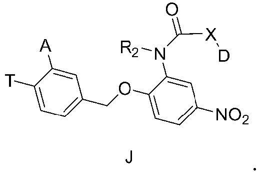

6. The linker compound according to claim 5, wherein, the linker compound (L) comprises (L-5) and (L-6), respectively as shown in the following formulas: 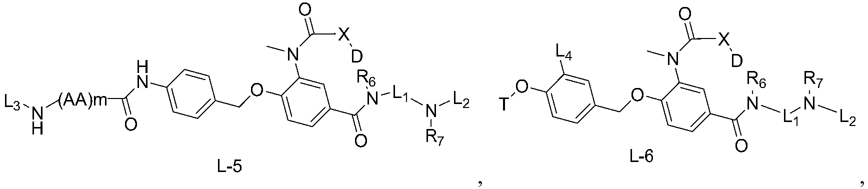 wherein,
in formula (L-5), AA represents an amino acid, m represents the number of amino acids, and m is selected from 1-6; L3 is selected from one or more of H, an amino protecting group, a fatty chain, a PEG chain, a polyamino acid chain, a sugar chain, and a maleimide-modified PEG chain, a maleimide-modified fatty chain, a maleimide-modified polyamino acid chain, or a maleimide-modified sugar chain; R₆ and R₇ are selected, either together or independently, from H or C1-C6 alkyl or cycloalkyl; L₁ is selected from C1-C6 alkyl or cycloalkyl, or R₆ and L₁ may form a cycloalkyl group; L₂ is selected from one or more of H, an amino protecting group, a fatty chain, a PEG chain, a polyamino acid chain, a sugar chain, and a maleimide-modified PEG chain, a maleimide-modified fatty chain, a maleimide-modified polyamino acid chain, or a maleimide-modified sugar chain; L₂ and L₃ are selected, either together or independently, from one or more of H, an amino protecting group, a fatty chain, a PEG chain, a polyamino acid chain, a sugar chain, and a maleimide-modified PEG chain, a maleimide-modified fatty chain, a maleimide-modified polyamino acid chain, or a maleimide-modified sugar chain; L₂ and L₃ are not simultaneously selected from a maleimide-modified PEG chain, a fatty chain, a polyamino acid chain, or a sugar chain;
in formula (L-6), T is selected from one or more of β-glucuronide, β-galactoside, phosphate ester, pyrophosphate ester, or sulfate ester; L₄ is selected from hydrogen, C1-C6 alkyl or cycloalkyl, a nitro group, an amino group, a methoxy group, a carboxyl group, an ester group, a fatty chain, a linear or branched PEG chain, a sugar chain, or a polyamino acid chain 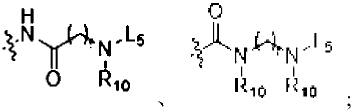
R₁₀ selected from hydrogen, C1-C3 alkyl, or cycloalkyl; L₅ is selected from hydrogen, an amino protecting group, C1-C6 alkyl or cycloalkyl, a fatty chain, a linear or branched PEG chain, a sugar chain, a polyamino acid chain, a maleimide-modified PEG chain, a maleimide-modified fatty chain, a maleimide-modified polyamino acid chain, or a maleimide-modified sugar chain; R₆ and R₇ are selected, either together or independently, from H or C1-C6 alkyl or cycloalkyl; L₁ is selected from C1-C6 alkyl or cycloalkyl, or R₆ and L₁ may form a cycloalkyl group; L₂ is selected from one or more of H, an amino protecting group, a fatty chain, a PEG chain, a polyamino acid chain, a sugar chain, and a maleimide-modified PEG chain, a maleimide-modified fatty chain, a maleimide-modified polyamino acid chain, or a maleimide-modified sugar chain.

7. A covalently conjugated prodrug or antibody-drug conjugate, wherein, it is formed by covalently linking maleimide of the linker compound shown in formula (L) and formula (J) according to claim 5 to albumin, an antibody, or an antigen-binding fragment thereof, and its structure is as shown in the following formulas (L1) to (L8) and formulas (J1) to (J4): 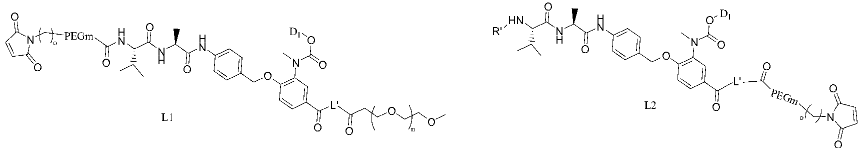 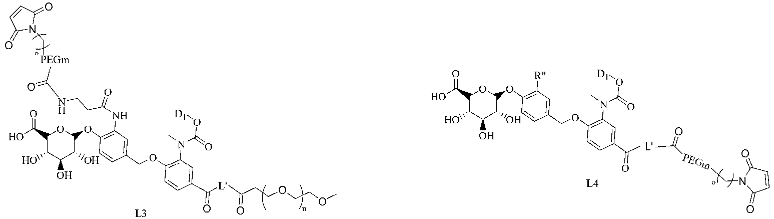 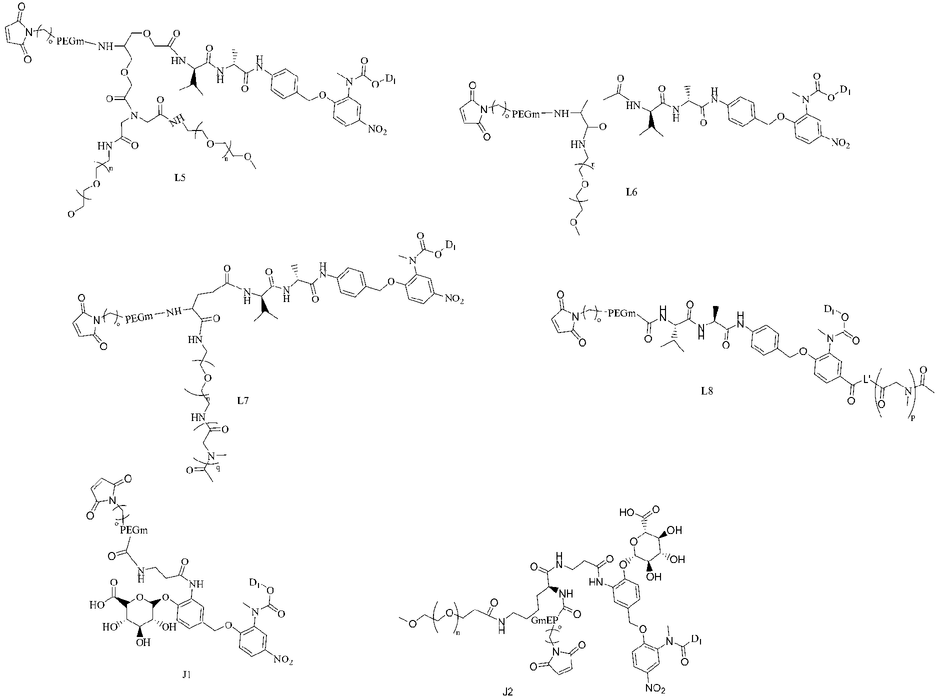 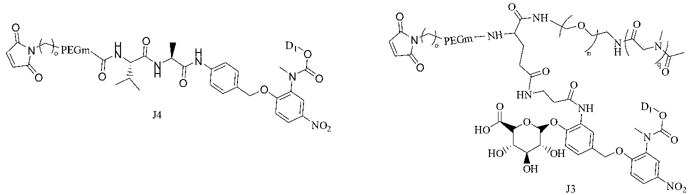
wherein, in the above formulas (L1) to (L8), formula (J1) and formula (J4), n, o, and m represent the number of repeating units, n is selected from 1-24; o is selected from 2-6; m is selected from 0-12;
in formulas (L1) to (L4) and (L8), L' is selected from one or more of 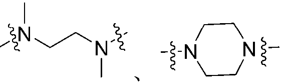 and 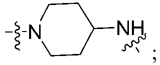
in formula (L2), R' is selected from hydrogen, acetyl, amino protecting groups including Boc, Cbz, Fmoc, Alloc, as well as PEG chains, polyamino acid chains, sugar chains, and fatty chains; in formula (L4), R" is selected from hydrogen, nitro, and methoxy;
D1 represents a hydroxyl-containing drug including camptothecins, and D1 includes:

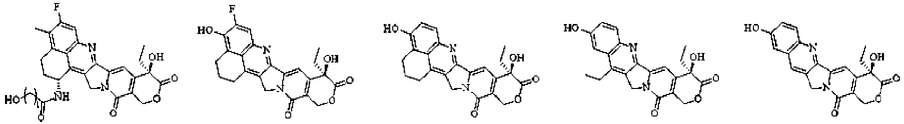

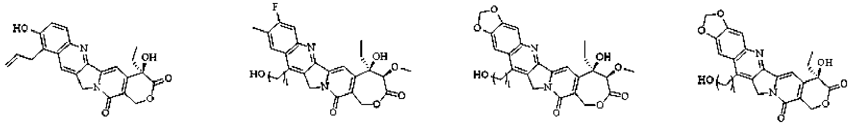

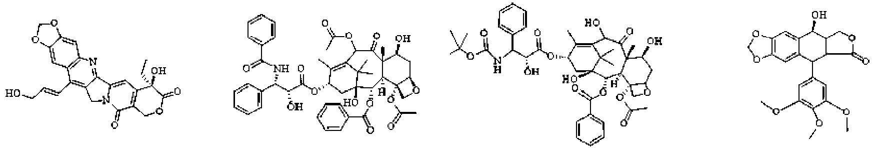

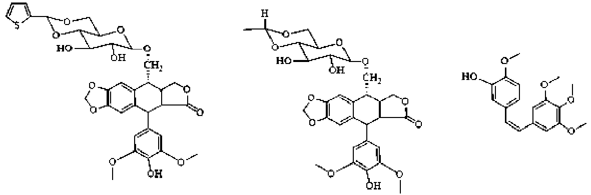

8. The covalently conjugated prodrug or antibody-drug conjugate according to claim 7, wherein, formulas (L1)-(L8) and formulas (J1)-(J4) include the following formulas (J1-1)-(J1-4), (J2-1)-(J2-2), (J4-1)-(J4-2), (L1-1)-(L1-4), (L4-1), (L5-1)-(L5-3), (L7-1)-(L7-4), (L8-1)-(L8-3):

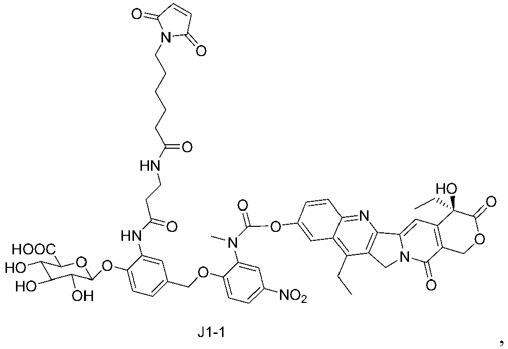

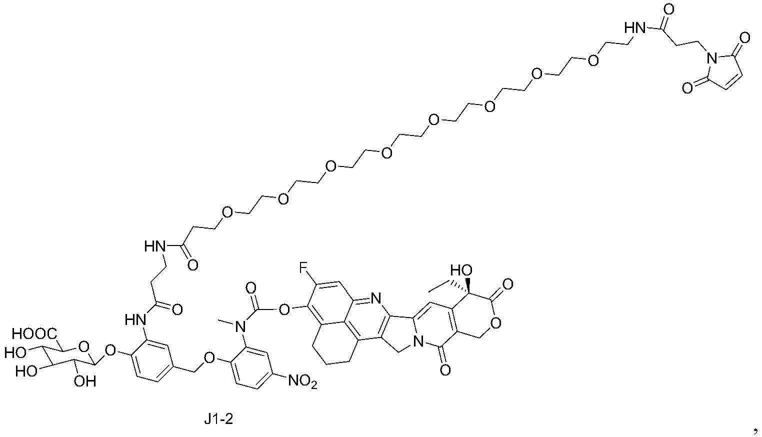

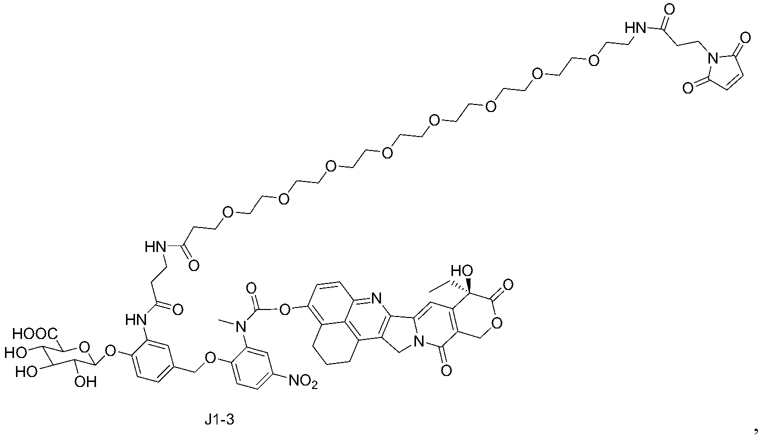

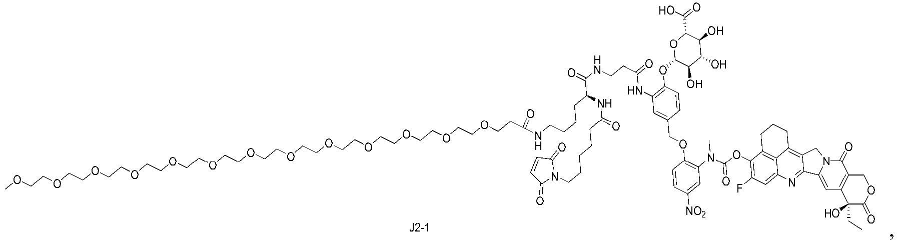

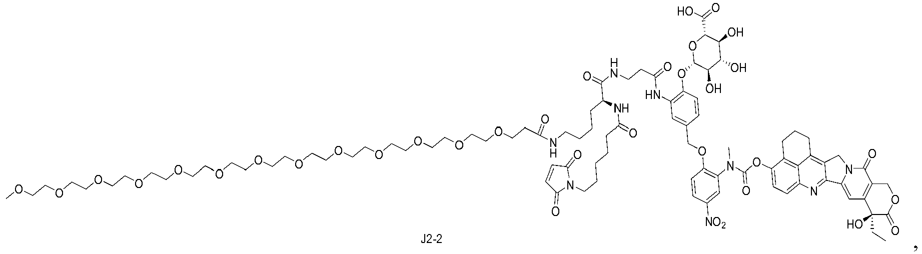

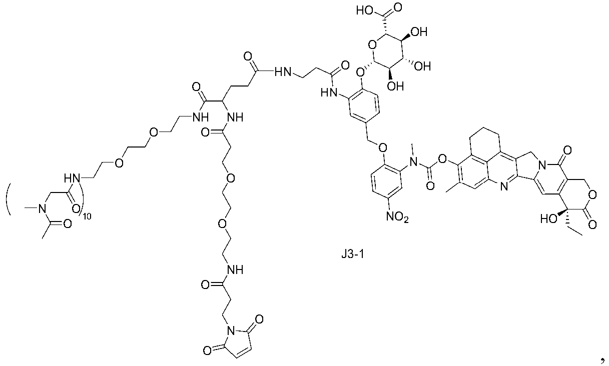

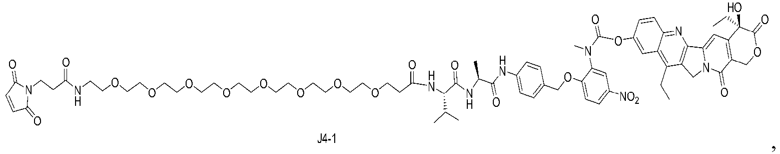

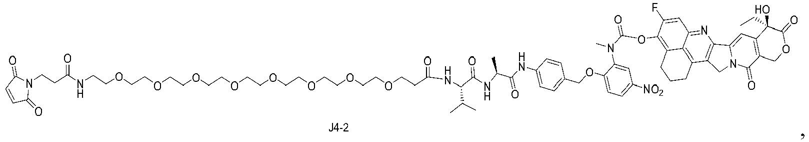

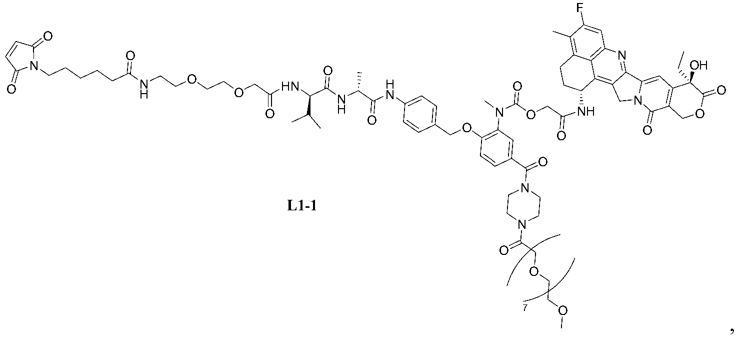

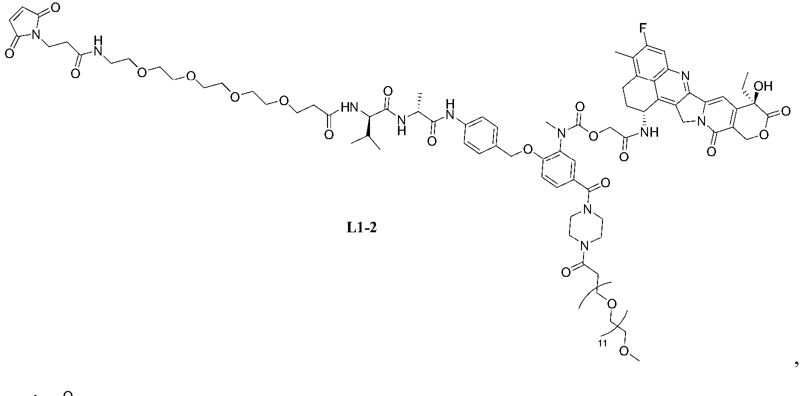

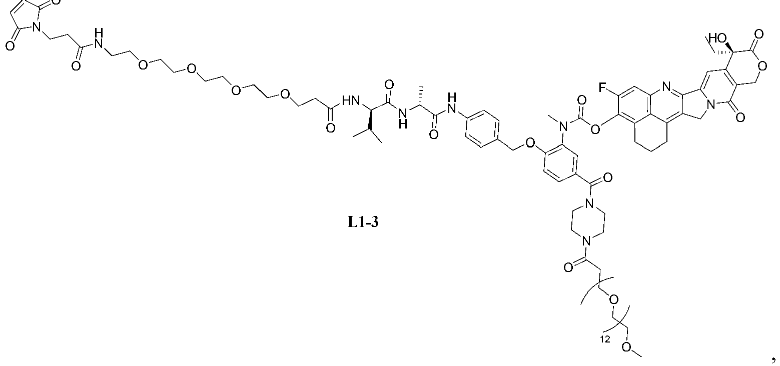

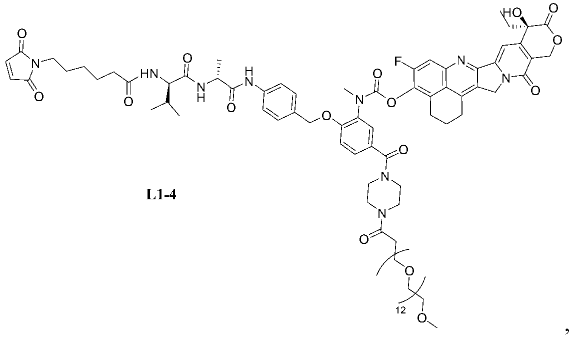

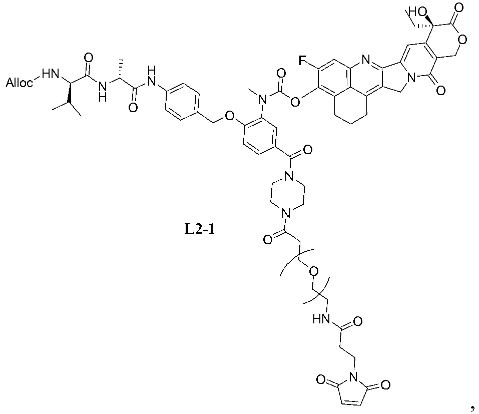

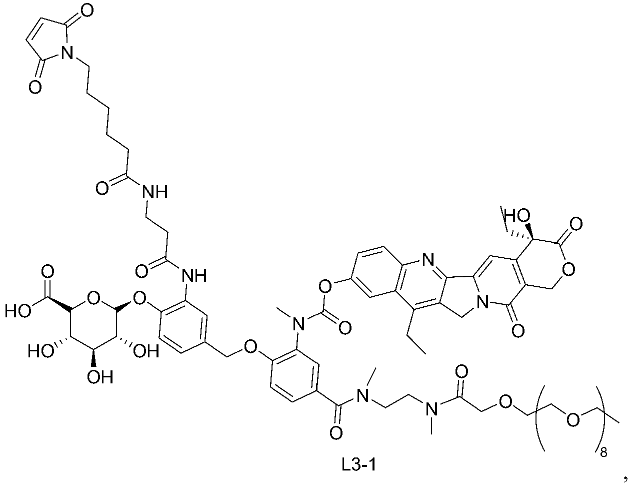

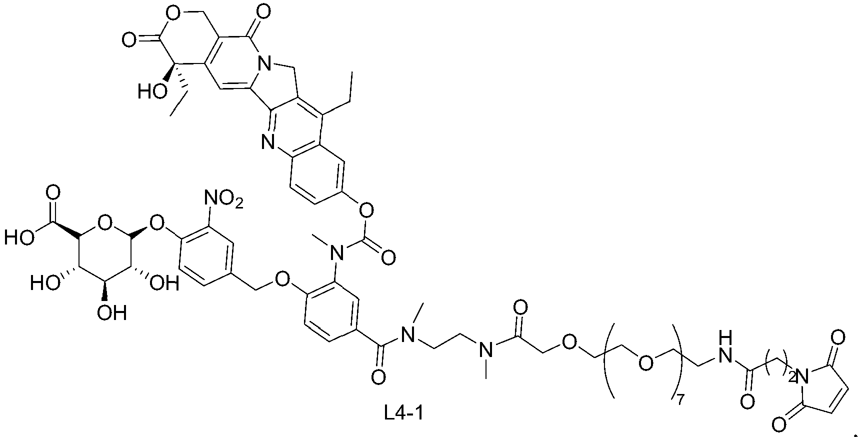

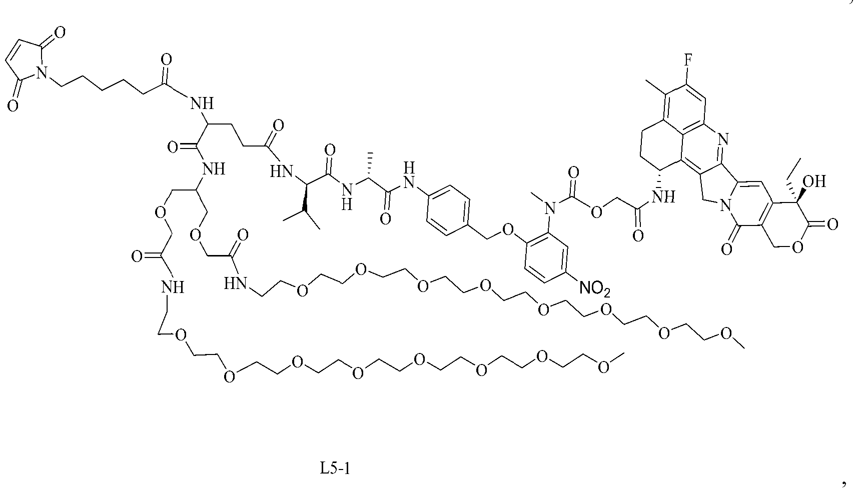

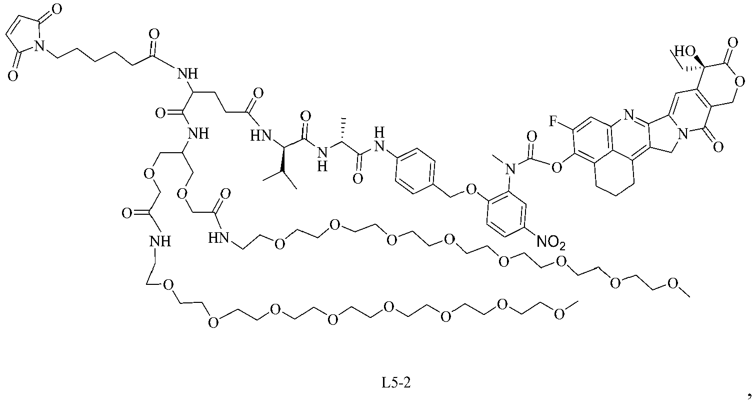

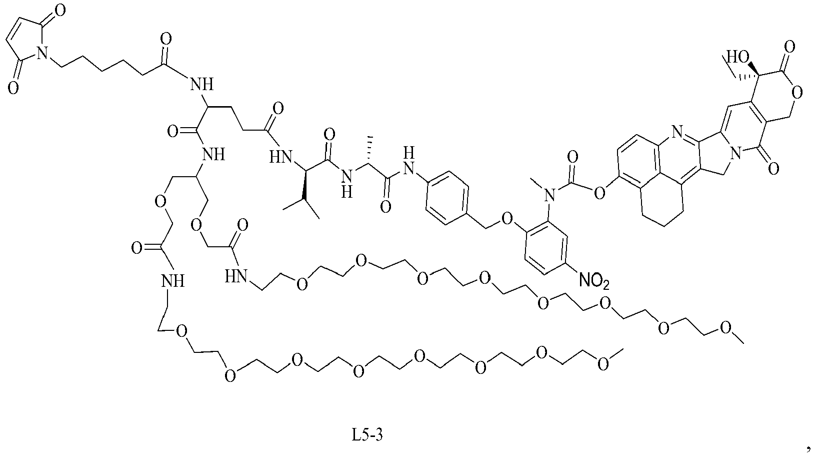

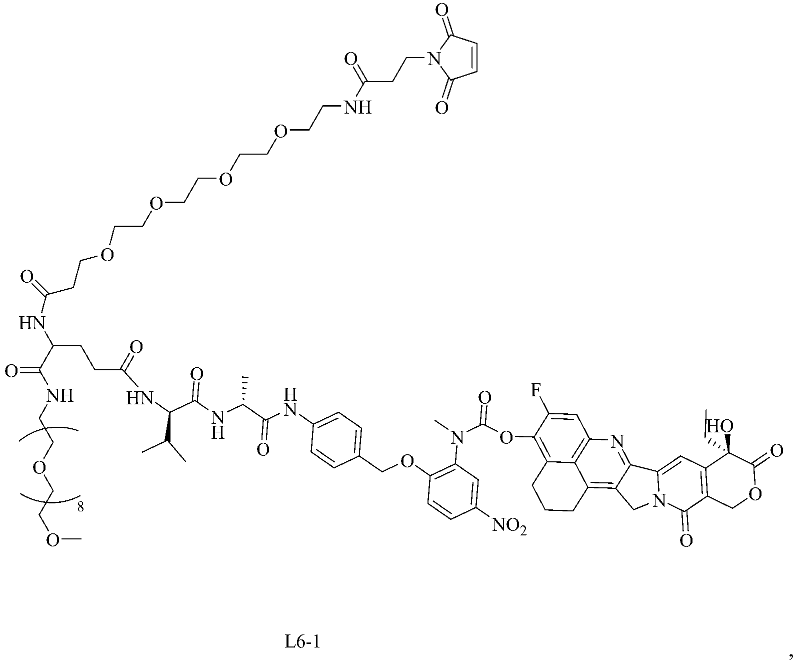

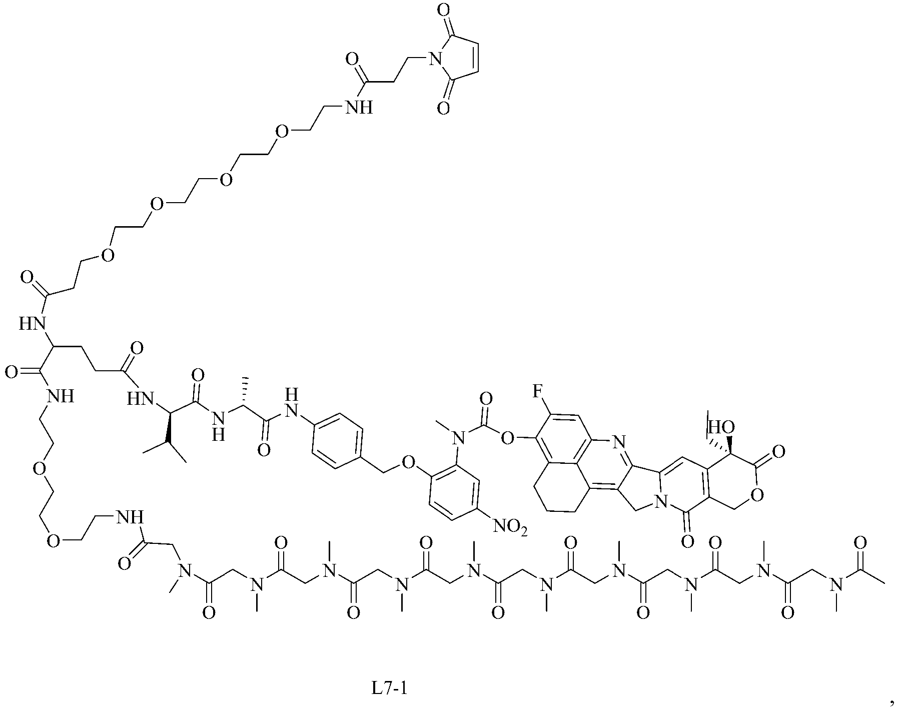

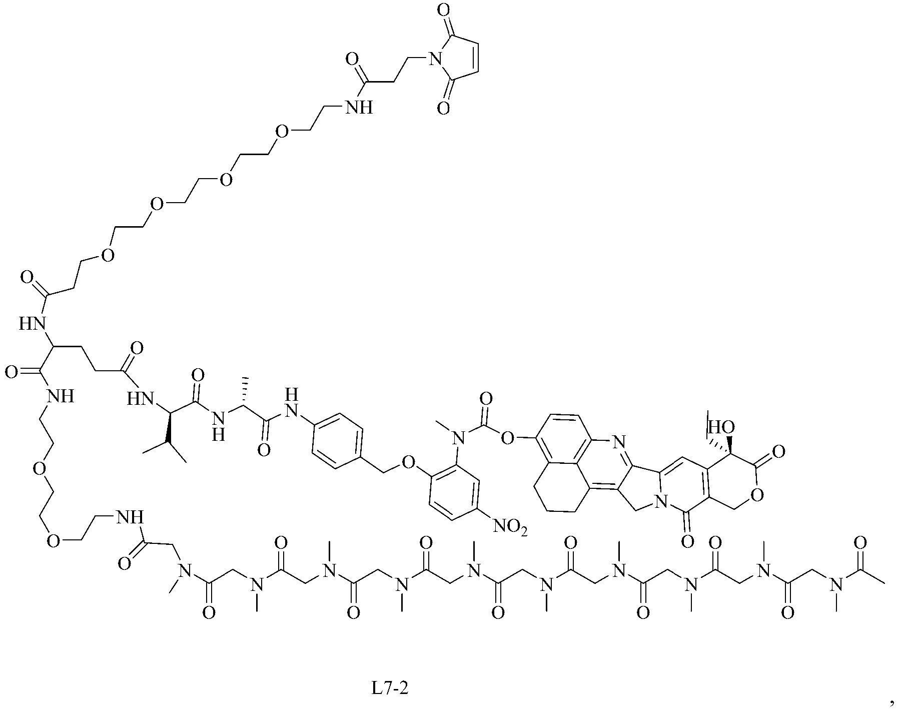

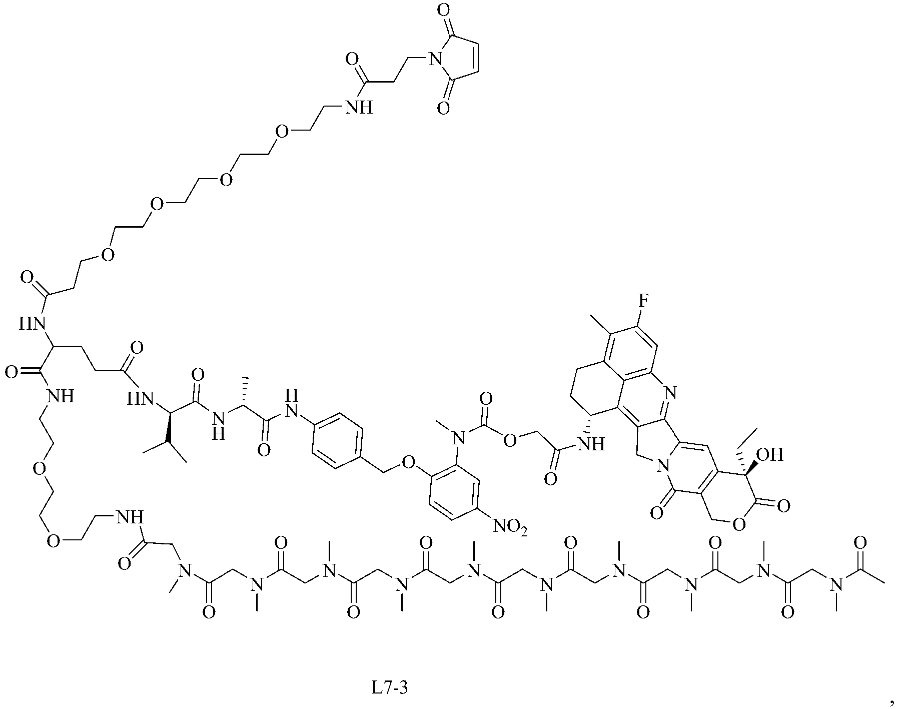

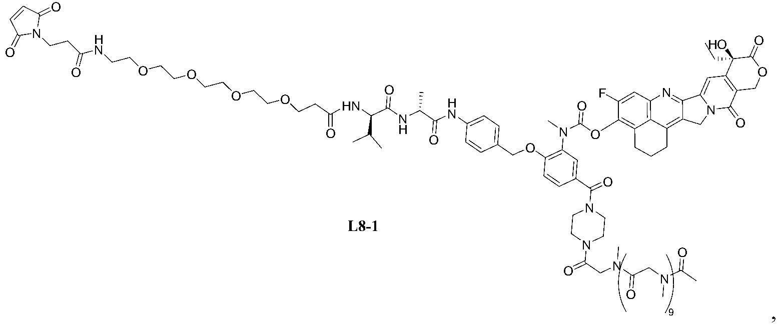

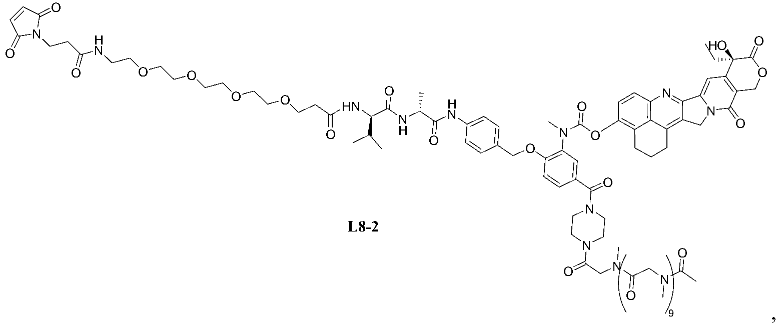

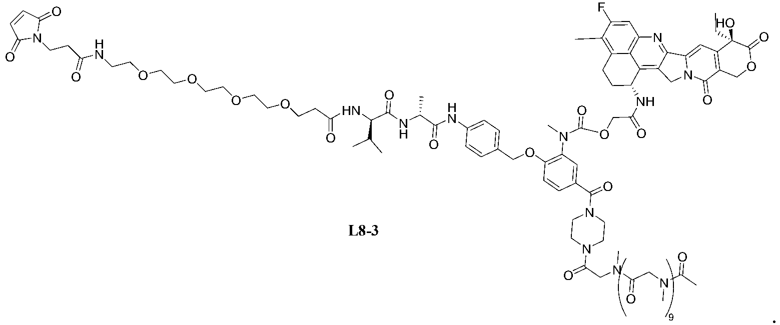

9. The covalently conjugated prodrug or antibody-drug conjugate according to claim 7 or 8, wherein, the antibody or antigen-binding fragment thereof is selected from a rabbit-derived antibody, a mouse-derived antibody, a chimeric antibody, a humanized antibody, a fully human antibody, or a protein fragment with functions similar to an antibody.

10. The covalently conjugated prodrug or antibody-drug conjugate according to claim 9, wherein, the antibody or antigen-binding fragment thereof is selected from: anti-CD3 antibody, anti-FOLR1 antibody, anti-ROR1 antibody, anti-TNFα antibody, anti-TF antibody, anti-EpCAM antibody, anti-EGFRvIII antibody, anti-DLL-3 antibody, anti-PSMA antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-Claudin 6 antibody, anti-Claudin 9 antibody, anti-Claudin 18.2 antibody, anti-Mesothelin antibody, anti-HER2 antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3 antibody, anti-MUC1/CD227 antibody, anti-AXL antibody, anti-CD166 antibody, anti-B7-H3(CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, anti-CD70 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD20 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody and anti-CD123 antibody;
and/or, the antibody or antigen-binding fragment thereof is selected from:
i) Trastuzumab antibody;
ii) ILB-3101 antibody;
iii) 59H4 antibody.

11. The covalently conjugated prodrug or antibody-drug conjugate according to any one of claims 7-10, wherein, the conjugation ratio of the antibody or antigen-binding fragment thereof to the linker compound is 3-8; preferably, the conjugation ratio is optimized to 6-8 or 4-6.

12. A pharmaceutical composition comprising the linker compound of the prodrug delivery system according to any one of claims 1-6, a pharmaceutically acceptable salt, solvate, polymorph, isomer, or derivative thereof, or the covalently conjugated prodrug or antibody-drug conjugate according to any one of claims 7-11, and a pharmaceutically acceptable excipient, diluent or carrier.

13. The use of the linker compound of the prodrug delivery system according to any one of claims 1-6, a pharmaceutically acceptable salt, solvate, polymorph, isomer, or derivative thereof, or the covalently conjugated prodrug or antibody-drug conjugate according to any one of claims 7-11, or the pharmaceutical composition according to claim 12 in the preparation of a medicament for treating tumors.

14. The use according to claim 13, wherein, the tumor is breast cancer, ovarian cancer, ovarian teratoma, cervical cancer, uterine cancer, prostate cancer, kidney cancer, urethral cancer, testicular cancer, bladder cancer, liver cancer, gastric cancer, endometrial cancer, salivary gland cancer, esophageal cancer, head and neck cancer, lung cancer, colon cancer, rectal cancer, colorectal cancer, bone cancer, skin cancer, thyroid cancer, pancreatic cancer, melanoma, neurological tumors, glioma, neuroblastoma, glioblastoma multiforme, squamous cell carcinoma, myeloma, sarcoma, lymphoma, and leukemia or hematologic malignancies.

15. A method for preparing the linker compound (L) according to claim 5, wherein, the preparation reaction process is as shown in the following scheme: 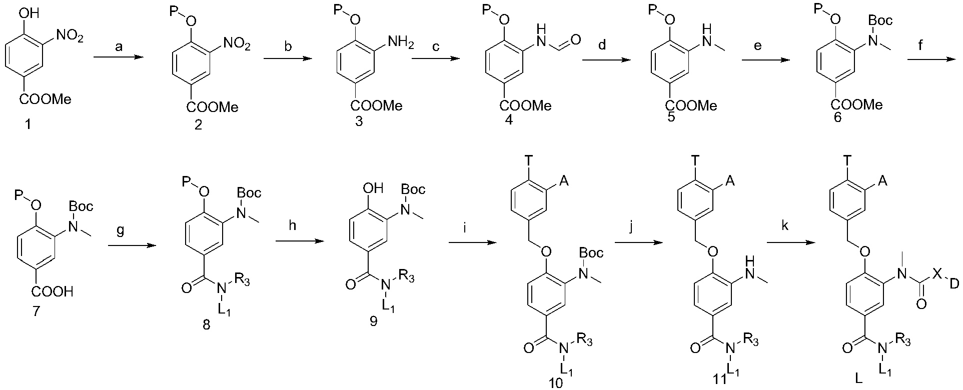 the reaction steps include:
a) substitution reaction: under alkaline conditions, compound 1 and a hydroxyl protecting group undergo a nucleophilic substitution reaction in a solvent to generate compound 2;
b) reduction reaction: compound 2 is reduced using a zinc powder/acid or iron powder/acid system to obtain compound 3, with the solvent being a conventional organic solvent/acid;
c) condensation reaction: under a nitrogen atmosphere, compound 3 undergoes a condensation reaction with formic acid and acetic anhydride to generate compound 4;
d) reduction reaction: under a nitrogen atmosphere, compound 4 is dissolved in a solvent and undergoes a reduction reaction with borane dimethyl sulfide complex to generate compound 5;
e) substitution reaction: under alkaline conditions, compound 5 undergoes a substitution reaction with BOC anhydride to obtain compound 6;
f) hydrolysis reaction: under alkaline conditions, compound 6 undergoes a hydrolysis reaction to obtain compound 7;
g) condensation reaction: compound 7 undergoes a condensation reaction with an amine in the presence of a condensing agent to generate compound 8;
h) decomposition reaction: compound 8 undergoes a deprotection reaction with Pd/C to generate compound 9, with the solvent being methanol or a mixed solvent of methanol and tetrahydrofuran and/or ethyl acetate;
i) etherification reaction: under nitrogen protection, different trigger fragment benzyl bromide substrates react in the presence of silver oxide or a base to generate compound 10, with the solvent being anhydrous tetrahydrofuran, anhydrous dichloromethane, or anhydrous acetonitrile;
j) deprotection reaction: compound 10 is dissolved in a solvent and subjected to acidic conditions to remove the N-Boc protection, yielding compound 11;
k) condensation reaction: under nitrogen protection, compound 11 is dissolved in an organic solvent, bis(trichloromethyl) carbonate and an anhydrous organic base are added; after reacting for 1 h, the active drug and DMAP are added, and the reaction is carried out at 20-60 °C overnight to obtain compound 12.

16. The preparation method according to claim 15, wherein, in step a), the hydroxyl protecting group includes benzyl bromide, p-methoxybenzyl bromide MOMCl; the base includes potassium carbonate, cesium carbonate, sodium carbonate; the solvent includes acetone, acetonitrile or N,N-dimethylformamide; the molar ratio of compound 1 to the base is 1:(1.5-2); the molar ratio of compound 1 to the protecting group is 1:(1.5-2);
in step b), the molar ratio of compound 2 to the zinc powder or iron powder is 1:5;
in step c), the molar ratio of compound 3 to formic acid is 1:(5-10), and the molar ratio of compound 3 to acetic anhydride is 1:(5-10);
in step d), the molar ratio of compound 4 to borane dimethyl sulfide complex is 1:3;
in step e), the base includes DIPEA, DMAP;
in step f), the base includes LiOH, NaOH or KOH; the molar ratio of compound 4 to borane dimethyl sulfide complex is 1:(2-5);
in step g), the condensing agent includes DCC, EEDQ, HATU, EDC/HOBt, PyBOP; the molar ratio of compound 7 to the amine is 1:(1-2), and the molar ratio of compound 7 to the condensing agent is 1:(2-5);
in step h), the molar ratio of compound 8 to Pd/C is 1:(0.1-0.2);
in step i), the molar ratio of compound 9 to different benzyl bromides or benzyl alcohols is 1:(1-1.2), and the molar ratio of compound 9 to silver oxide/phosphorus reagent/azo compound is 1:(1.2-2);
in step j), the acid includes hydrochloric acid, trifluoroacetic acid, and the solvent includes tetrahydrofuran or dichloromethane;
in step k), the anhydrous organic base includes triethylamine, DIPEA, pyridine; the drug contains exposed -OH, -NH2, -NHR, -SH; the molar ratio of compound 11 to bis(trichloromethyl) carbonate is 1:0.5, the molar ratio of compound 11 to the anhydrous organic base is 1:(3-5); the molar ratio of compound 11 to the active drug is 1:(1-1.2).

17. A method for preparing a covalently conjugated prodrug or antibody-drug conjugate (J1), wherein, the reaction process of the preparation method is as shown in the following scheme: 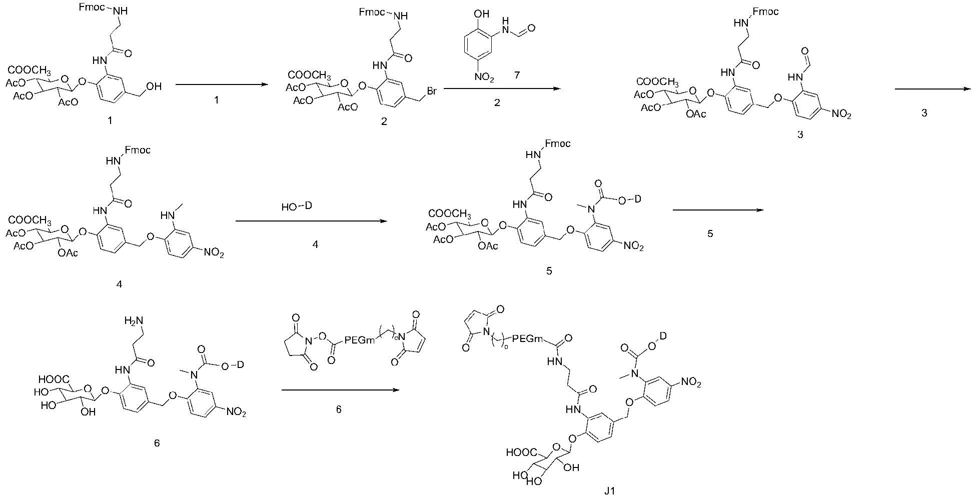 the reaction steps include:
1) bromination reaction: compound 1 undergoes a bromination reaction to obtain compound 2; the bromination reagent includes phosphorus tribromide, triphenylphosphine and carbon tetrabromide, and the solvent is anhydrous dichloromethane;
2) etherification reaction: compound 2 undergoes an etherification reaction to generate compound 3, and the organic solvent is anhydrous acetonitrile;
3) reduction reaction: under a nitrogen atmosphere, compound 3 and borane dimethyl sulfide complex are dissolved in a solvent to undergo a reduction reaction to generate compound 4, and the solvent is anhydrous tetrahydrofuran;
4) condensation reaction: under nitrogen protection, compound 4 is dissolved in an organic solvent, bis(trichloromethyl) carbonate and triethylamine are added, after reacting for 1 h, a hydroxyl-containing drug is added, and the reaction is carried out at room temperature overnight to obtain compound 5;
5) deprotection: under nitrogen protection, compound 5 is dissolved in an organic solvent, and a 0.2M aqueous alkali solution is added under ice bath conditions for deprotection to obtain compound 6;
6) condensation reaction: compound 6 and compound 9 are dissolved in an organic solvent and stirred at room temperature for 6-8 h to react to generate the prodrug having the structure of general formula (J1).,

18. The preparation method according to claim 17, wherein, in step 1), the molar ratio of compound 1 to the bromination reagent is 1:3;
in step 3), the molar ratio of compound 3 to borane dimethyl sulfide complex is 1:3;
in step 4), the molar ratio of compound 4 to bis(trichloromethyl) carbonate is 1:0.5, the molar ratio of compound 4 to triethylamine is 1:4; the molar ratio of compound 4 to the hydroxyl-containing drug is 1:1;
in step 5), the base includes one or more of lithium hydroxide, sodium hydroxide, potassium hydroxide; the organic solvent includes one or more of methanol, acetonitrile, tetrahydrofuran and water; the molar ratio of compound 5 to the base is 1:8;
in step 6), the molar ratio of compound 6 to compound 9 is 1:1; the organic solvent is one or a mixture of methanol, acetonitrile, tetrahydrofuran, N,N-dimethylformamide and water.
